# EUROPEAN PATENT SPECIFICATION

(11) **EP 4 051 262 B1**
(45) Date of publication and mention of the grant of the patent: **28.10.2026**
(21) Application number: 20811859.6
(22) Date of filing: 29.10.2020
(51) Int. Cl.: A61K 31/435, A61K 31/395, A61P 7/00, A61P 7/06, A61P 13/12

(54) **VADADUSTAT FOR USE IN THE TREATMENT OF ANAEMIA ASSOCIATED WITH OR SECONDARY TO CHRONIC KIDNEY DISEASE**
VADADUSTAT ZUR BEHANDLUNG VON ANÄMIE IM ZUSAMMENHANG MIT ODER ALS FOLGE EINER CHRONISCHEN NIERENERKRANKUNG
VADADUSTAT POUR SON UTILISATION DANS LE TRAITEMENT DE L'ANÉMIE ASSOCIÉE OU SECONDAIRE À UNE MALADIE RÉNALE CHRONIQUE

(30) Priority: 31.10.2019 US 201962928994 P; 06.11.2019 US 201962931458 P; 08.11.2019 US 201962933077 P; 02.09.2020 US 202063073612 P
(43) Date of publication of application: 07.09.2022
(73) Proprietor: Akebia Therapeutics, Inc., Cambridge, MA 02142 (US); Tanabe Pharma Corporation, Osaka-shi, Osaka 541-8505 (JP)
(72) Inventor: DEGOMA, Emil, Cambridge, MA 02142 (US); MARUYAMA, Nobuko, Osaka-shi, 541-8505 (JP); KANEKO, Genki, Osaka-shi, 541-8505 (JP)
(74) Representative: Jones Day
(86) International application number: PCT/US2020/058007
(87) International publication number: WO 2021/087144

(56) References cited:
- ANON.: "Akebia Announces Positive Top-Line Results from its Phase 2 Study of Vadadustat in Dialysis Patients with Anemia Related to Chronic Kidney Disease", AKEBIA PRESS RELEASE, 8 September 2015 (2015-09-08), XP093193715, Retrieved from the Internet <URL:https://ir.akebia.com/static-files/54c1a177-07fa-4aa2-a670-5deeb3a78ce4> [retrieved on 20240808]
- ANON.: "Akebia Therapeutics, Inc. - Annual Report for the Fiscal Year ended December 31 2014", 4 March 2015 (2015-03-04), XP093193723, Retrieved from the Internet <URL:https://www.sec.gov/Archives/edgar/data/1517022/000156459015001260/akba-10k_20141231.htm> [retrieved on 20240808]
- ECKARDT K-U ET AL: "Safety and Efficacy of Vadadustat for Anemia in Patients Undergoing Dialysis", NEW ENGLAND JOURNAL OF MEDICINE, vol. 384, no. 17, 29 April 2021 (2021-04-29), United States, pages 1601 - 1612, XP093324474, ISSN: 0028-4793, DOI: 10.1056/NEJMoa2025956
- PABLO E. PERGOLA ET AL: "Vadadustat, a novel oral HIF stabilizer, provides effective anemia treatment in nondialysis-dependent chronic kidney disease", KIDNEY INTERNATIONAL, vol. 90, no. 5, 1 November 2016 (2016-11-01), GB, pages 1115 - 1122, XP055619130, ISSN: 0085-2538, DOI: 10.1016/j.kint.2016.07.019

## Description

### BACKGROUND OF THE INVENTION

Treatment of anemia associated with chronic kidney disease (CKD) using erythropoiesis-stimulating agents (ESAs), such as epoetin alfa, epoetin beta, darbepoetin, or peginesatide, often results in prolonged, supraphysiologic erythropoietin (EPO) levels, which are implicated in increased unwanted cardiovascular side effects, including hypertension and thromboembolic events. Kidney Int. 2016, 90(5), 1115 discloses vadadustat for oral use in the treatment of anaemia in non-dialysis-dependent chronic kidney disease patients. The patients were treated for 20 weeks and underwent a titrated dosing schedule, including specific oral doses of 150, 450 and 600 mg Therefore, a need exists for treatment of anemia associated with chronic kidney disease (CKD) without prolonged, supraphysiologic erythropoietin (EPO) levels.

### SUMMARY

This invention provides a compound for use in a method of treating anemia in a patient having anemia associated with or secondary to chronic kidney disease, wherein the compound is {[5-(3-chlorophenyl)-3-hydroxypyridine-2-carbonyl]amino}acetic acid having the structure of Compound **1,** 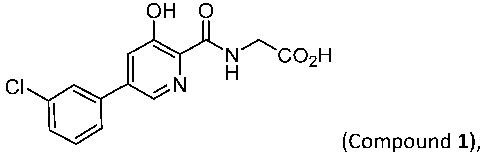 or a pharmaceutically acceptable salt thereof, wherein the patient has been previously treated with an erythropoiesis stimulating agent (ESA) within six weeks or eight weeks prior to or during a screening period before administering a dose of Compound 1, and wherein the method comprises orally administering the compound to the patient for 53-260 weeks.

In another aspect, the invention provides a compound for use in a method of increasing hemoglobin levels to 10.0 g/dL - 13.0 g/dL in a patient having anemia associated with or secondary to chronic kidney disease, wherein the compound is {[5-(3-chlorophenyl)-3-hydroxypyridine-2-carbonyl]amino}acetic acid having the structure of Compound 1, 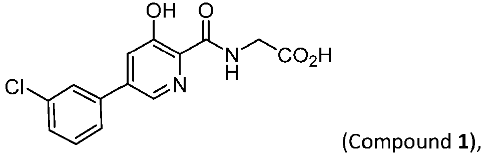 or a pharmaceutically acceptable salt thereof, wherein the patient has been previously treated with an erythropoiesis stimulating agent (ESA) within six weeks or eight weeks prior to or during a screening period before administering a dose of Compound **1,** and wherein the method comprises orally administering the compound to the patient for 53-260 weeks.

In yet another aspect, the invention provides a compound for use in a method of maintaining or controlling hemoglobin levels at 10.0 - 13.0 g/dL in a patient having anemia associated with or secondary to chronic kidney disease, wherein the compound is {[5-(3-chlorophenyl)-3-hydroxypyridine-2-carbonyl]amino}acetic acid having the structure of Compound **1,** 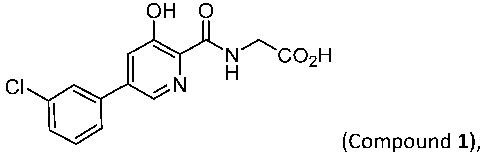
or a pharmaceutically acceptable salt thereof, wherein the patient has been previously treated with an erythropoiesis stimulating agent (ESA) within six weeks or eight weeks prior to or during a screening period before administering a dose of Compound **1,** and
wherein the method comprises orally administering the compound to the patient for 53-260 weeks.

In one embodiment, the patient has non-dialysis dependent chronic kidney disease (NDD-CKD). In another embodiment of the invention, the patient has dialysis-dependent chronic kidney disease (DD-CKD).

In one embodiment of the invention, the erythropoiesis stimulating agent is epoetin, darbepoetin alfa (DA), or methoxy polyethylene glycol-epoetin beta (epoetin beta pegol).

In yet another embodiment, the method comprises orally administering once daily to the patient 150, 300 mg, 450 mg, or 600 mg of Compound **1.**

In another aspect, the invention provides a pharmaceutical composition for use in a method of treating anemia in a patient having anemia associated with or secondary to chronic kidney disease, the composition comprising {[5-(3-chlorophenyl)-3-hydroxypyridine-2-carbonyl]amino}acetic acid having the structure of Compound 1, 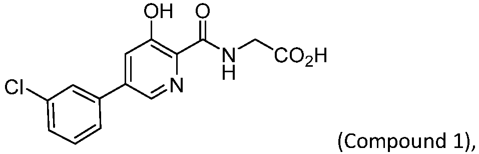
or a pharmaceutically acceptable salt thereof, and one or more excipients,
wherein the patient has been previously treated with an erythropoiesis stimulating agent (ESA) within six weeks or eight weeks prior to or during a screening period before the administration of Compound 1, and
   wherein the method comprises orally administering the pharmaceutical composition to the patient for 53-260 weeks.

In one embodiment of the pharmaceutical composition, the patient has non-dialysis dependent chronic kidney disease (NDD-CKD).

In one embodiment of the pharmaceutical composition, the patient has dialysis-dependent chronic kidney disease (DD-CKD).

In one embodiment of the pharmaceutical composition, the erythropoiesis stimulating agent is epoetin, darbepoetin alfa (DA), or methoxy polyethylene glycol-epoetin beta (epoetin beta pegol).

In one embodiment of the pharmaceutical composition, the method comprises orally administering once daily to the patient 150, 300, 450, or 600 mg of Compound 1.

### BRIEF DESCRIPTION OF THE DRAWINGS

**FIG. 1** is a schematic representation of the study design for the Phase III randomized, open-label, active-controlled NDD-CKD Conversion/Correction Study (J01).
**FIG. 2A** is a mean Hb over time for the entire 52 weeks of the NDD-CKD Conversion/Correction Study for the overall population of patients. **FIG. 2B** is a mean hemoglobin over time (52 weeks) in the ESA non-user group. **FIG. 2C** is a Mean hemoglobin over time (52 weeks) in the ESA user group. **FIG. 2D** is a mean hemoglobin over time (52 weeks) in the ESA user subgroups categorized by Hb during screening: ≥11.0 g/dl. **FIG. 2E** is a mean hemoglobin over time (52 weeks) in the ESA user subgroups categorized by Hb during screening: Hb <11.0 g/dl.

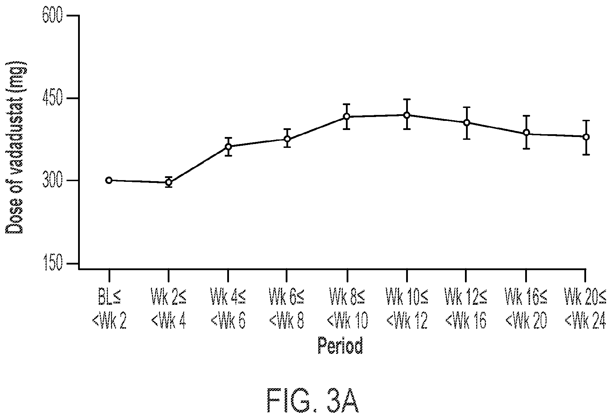

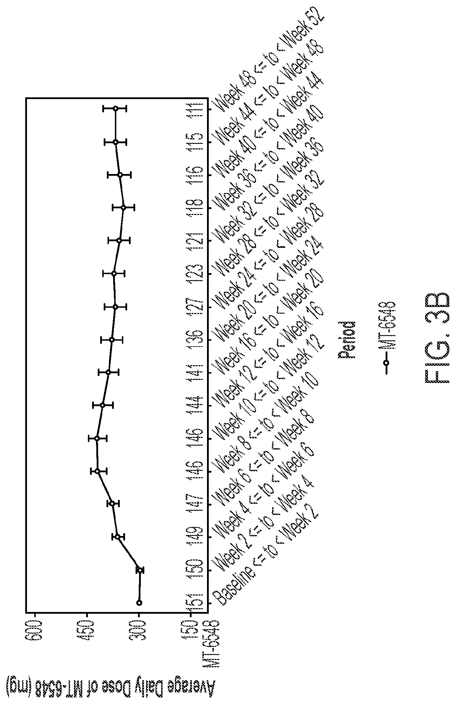

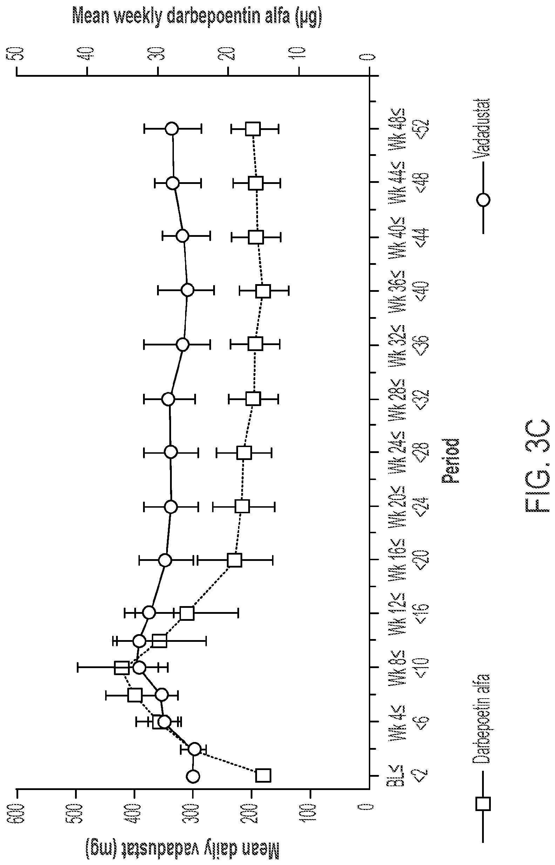

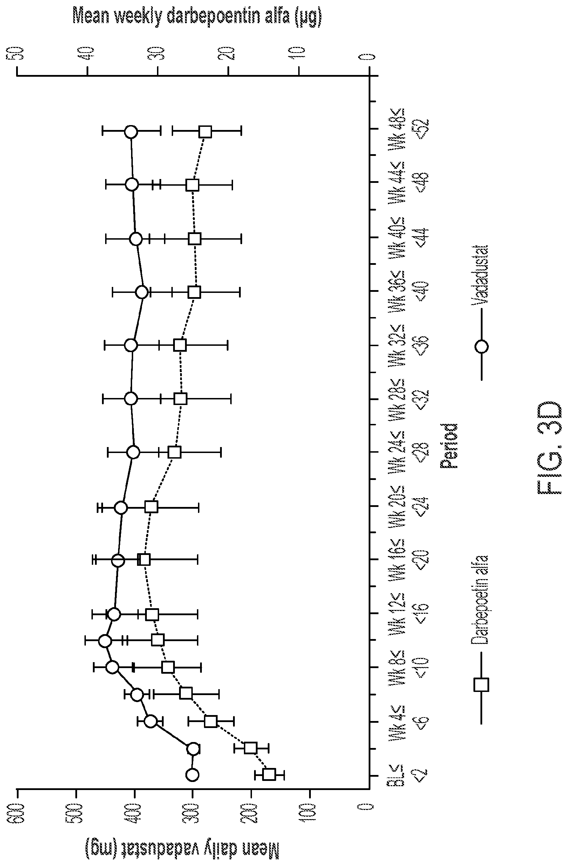

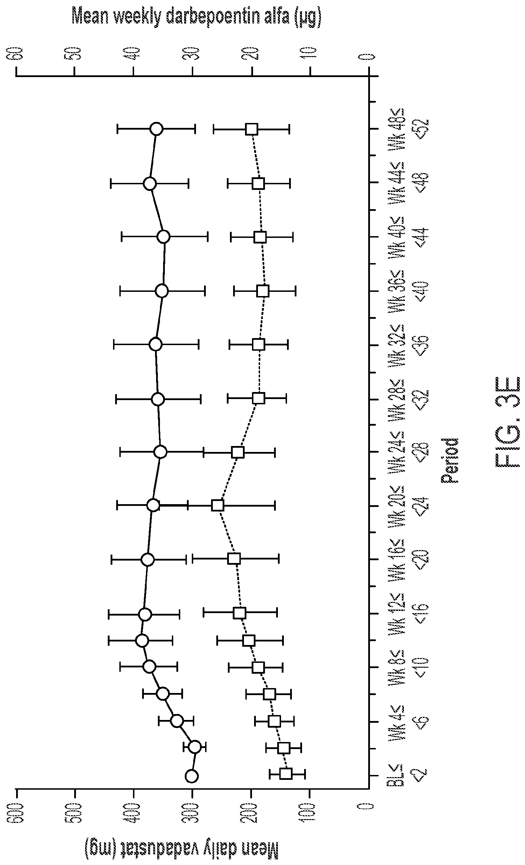

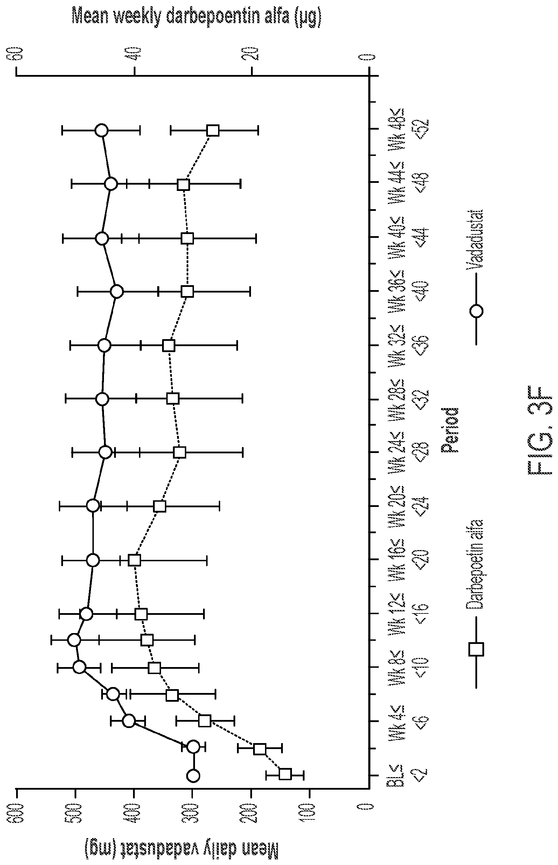

**FIG. 3A** shows the average dose of Compound **1** for the overall population up to week 24 of the study, and **FIG. 3B** shows the average dose of Compound **1** for the overall population up to week 52 of the study. **FIG. 3C** shows the average dose of Compound **1** (daily) or darbepoetin alfa (weekly) in the ESA non-user group. **FIG. 3D** shows the average dose of Compound **1** (daily) or darbepoetin alfa (weekly) in the ESA user group. **FIG. 3E** shows the average dose of Compound **1** (daily) or darbepoetin alfa (weekly) in the ESA user subgroup categorized by Hb during screening: ≥11.0 g/dl. **FIG. 3F** shows the average dose of Compound **1** (daily) or darbepoetin alfa (weekly) in the ESA user subgroup categorized by Hb during screening: Hb <11.0 g/dl.
**FIG. 4** compares various iron related parameters for the overall population receiving therapy with Compound **1** and those receiving therapy with darbepoetin alfa (DA) over time, including differences in serum ferritin (ng/mL), TSAT %, TIBC (µg/dL), hepcidin (ng/mL), serum iron (µg/dL), and the monthly dose of iron by any route (mg).
**FIG. 5** shows mean red blood cell-related parameters over time: MCH (mean corpuscular hemoglobin), MCHC (mean corpuscular hemoglobin concentration), MCV (mean corpuscular volume); and RDW (red cell distribution width) for Compound **1** and DA patients.

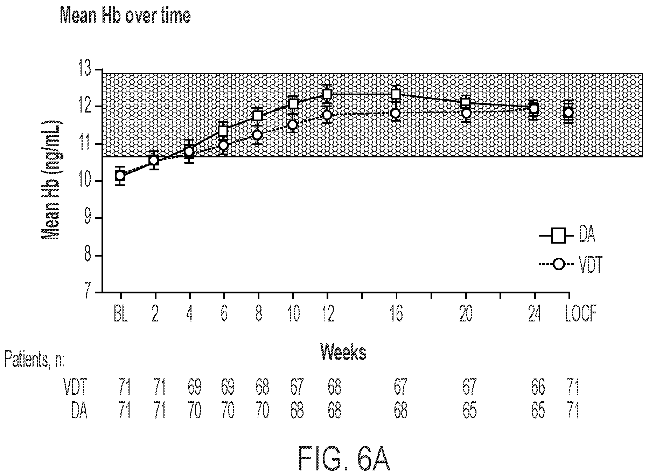

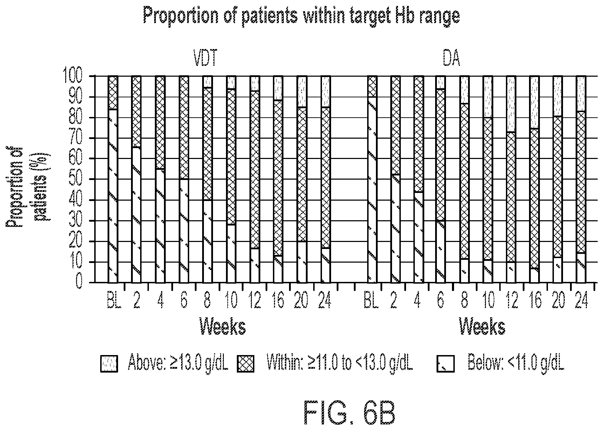

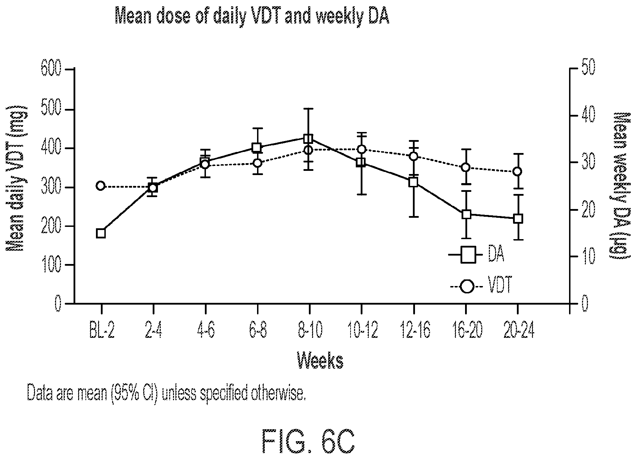

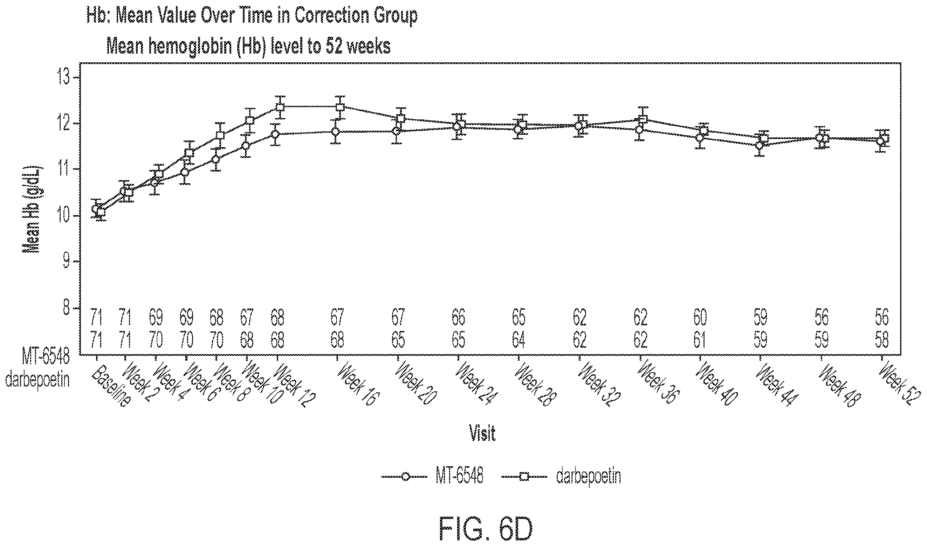

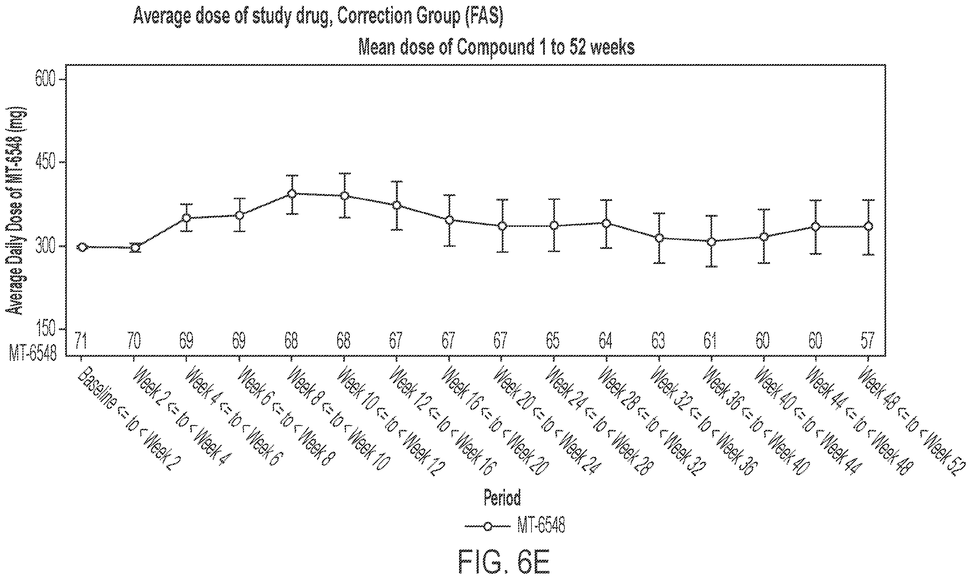

**FIGS. 6A-6E** provide graphical representations of the mean dosages and mean Hb levels of Compound 1 compared to darbepoetin alpha from baseline to week 24 for the correction population of the NDD-CKD Conversion/Correction Study. **FIG. 6A** shows the Hb level from baseline to week 24 for the correction population of NDD-CKD Conversion/Correction Study. **FIG. 6B** shows the proportion of patients within target Hb range from baseline to week 24 for the correction population of the NDD-CKD Conversion/Correction Study. **FIG. 6C** shows the mean daily dose of Compound 1 and weekly dose of darbepoetin alpha from baseline to week 24 for the correction population of the NDD-CKD Conversion/Correction Study. **FIG. 6D** shows the mean hemoglobin (Hb) level to week 52 for the correction population of the NDD-CKD Conversion/Correction Study. **FIG. 6E** shows the mean daily dose of Compound 1 from baseline to week 52 for the correction population of the NDD-CKD Conversion/Correction Study.

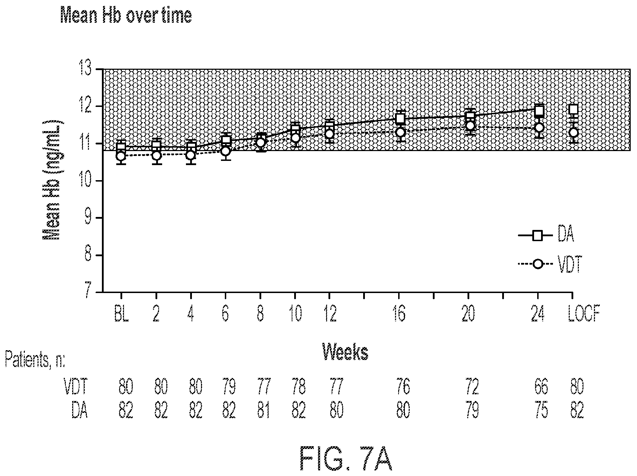

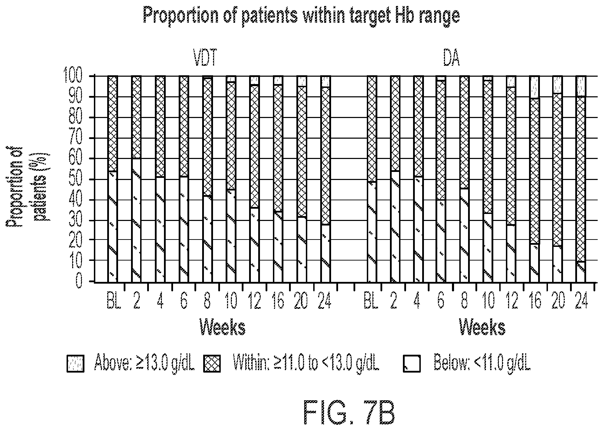

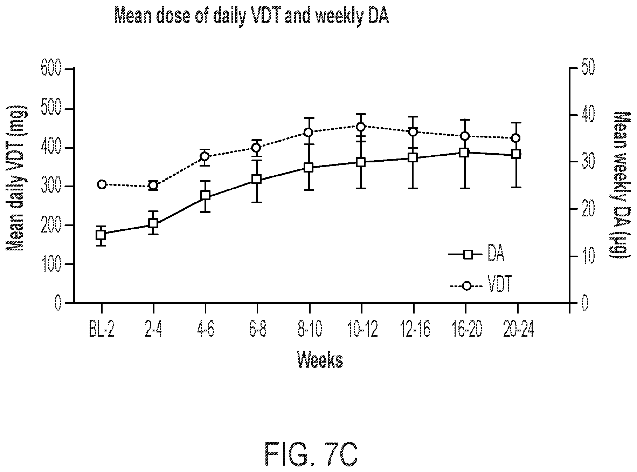

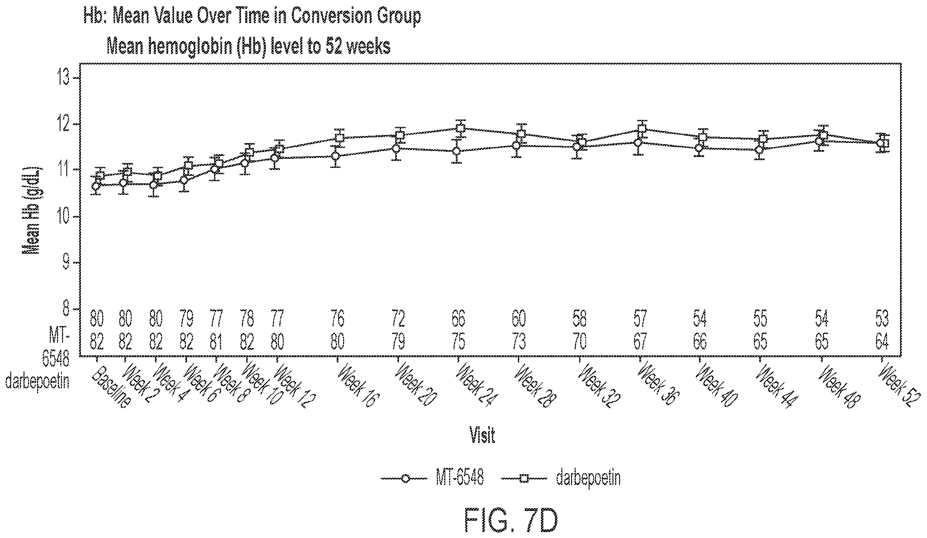

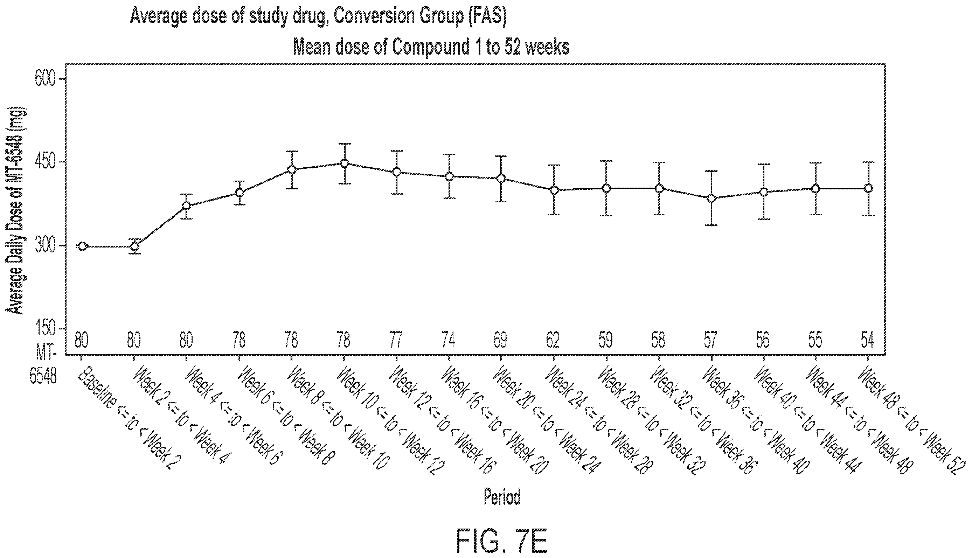

**FIGS. 7A-7E** provide graphical representations of the mean dosages and mean Hb levels of Compound 1 compared to darbepoetin alpha from baseline to week 24 for the conversion population of the NDD-CKD Conversion/Correction Study. **FIG. 7A** shows the Hb level from baseline to week 24 for the conversion population of NDD-CKD Conversion/Correction Study. **FIG. 7B** shows the proportion of patients within target Hb range from baseline to week 24 for the conversion population of the NDD-CKD Conversion/Correction Study. **FIG. 7C** shows the mean daily dose of Compound 1 and weekly dose of darbepoetin alpha from baseline to week 24 for the conversion population of the NDD-CKD Conversion/Correction Study. **FIG. 7D** shows the mean hemoglobin (Hb) level to week 52 for the conversion population of the NDD-CKD Conversion/Correction Study. **FIG. 7E** shows the mean daily dose of Compound 1 and weekly dose of darbepoetin alpha from baseline to week 52 for the conversion population of the NDD-CKD Conversion/Correction Study.
**FIG. 8A** shows the average daily dose of Compound **1** in conversion patients having a hemoglobin (Hb) level < 11 g/dL to week 52 of the study, and **FIG. 8B** shows the average daily dose of Compound **1** in conversion patients having a hemoglobin (Hb) level is ≥ 11 g/dL to week 52 of the study.
**FIG. 9** shows that the amount of an erythropoietin stimulating agent (ESA) therapy received can also influence the daily dose of Compound **1 over** a 52-week period. For example, the average dose of Compound **1 was** higher for patients who received weekly doses of ≥ 15 µg darbepoetin alfa as compared to patients who received weekly doses of < 15 µg darbepoetin alfa.
**FIG. 10A** shows the mean Hb over time in HD-CKD patients over 52 weeks who received therapy with Compound **1** (VDT) or darbepoetin alfa (DA). **FIG. 10B** shows the average dose of Compound **1** (VDT) for HD-CKD patients through week 52 of the study. **FIG. 10C** shows the average dose of darbepoetin alfa (DA) for HD-CKD patients through week 52 of the study. **FIG. 10D** shows the Proportion of patients with Hb levels within the target range.

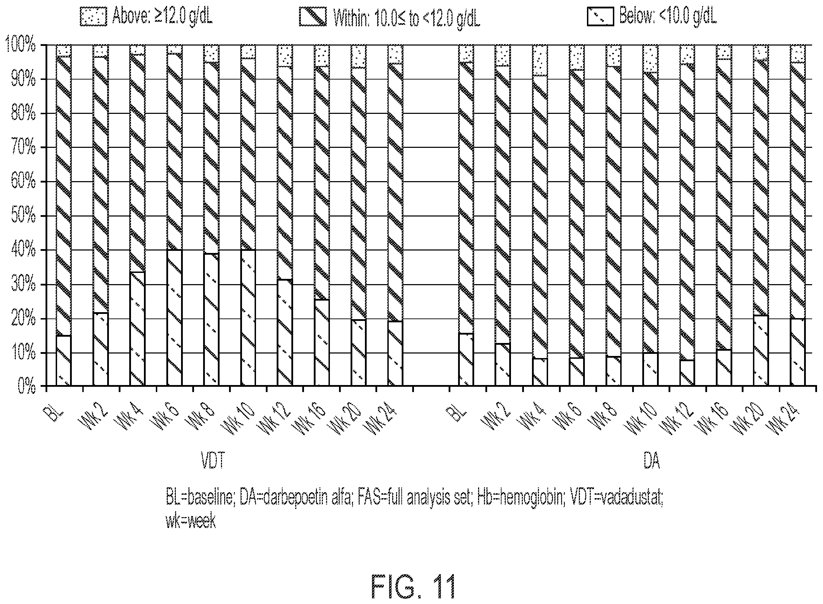

**FIG. 11** shows the proportion of patients within the target Hb range over 24 weeks who received therapy with Compound **1** (VDT) or darbepoetin alfa (DA).
**FIG. 12A** shows the mean Hb in HD-CKD patients treated with Compound **1** (VDT), **FIG. 12B** shows the mean Hb in patients treated with HD-CKD patients treated with darbepoetin alfa (DA), and **FIG. 12C** shows the mean dose of Compound **1** (VDT) in HD-CKD patients receiving this therapy through week 24 of the study. **FIG. 12D** shows the mean Hb value in HD-CKD patients over the 52-week study.

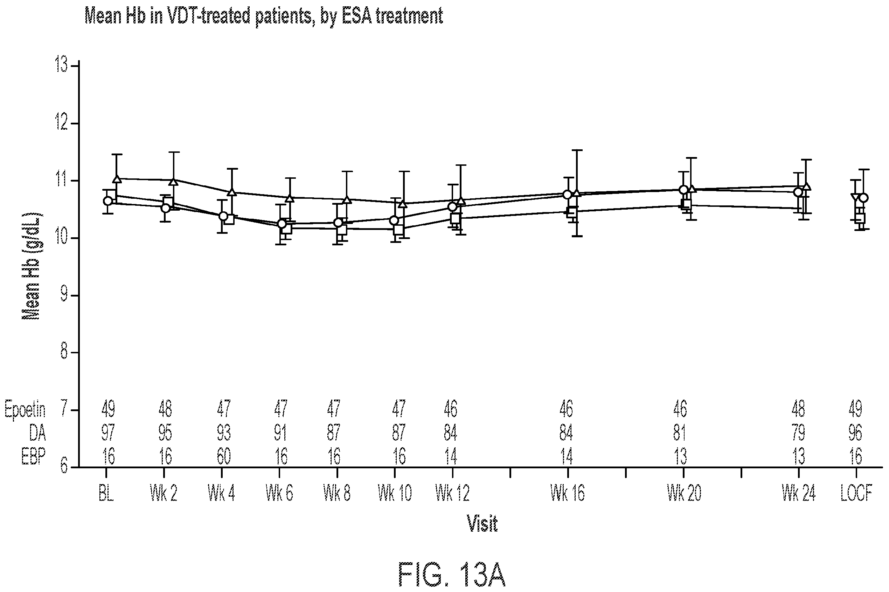

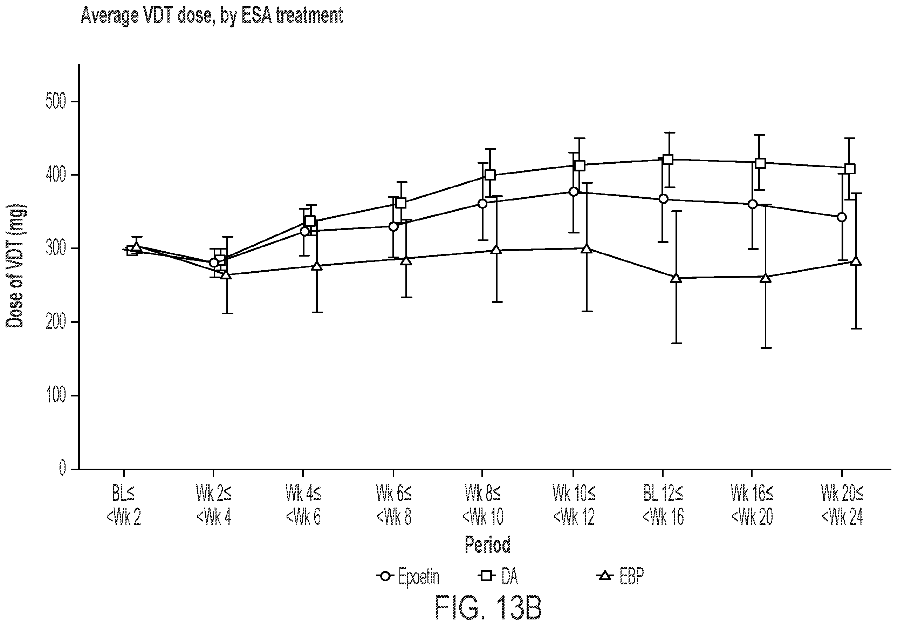

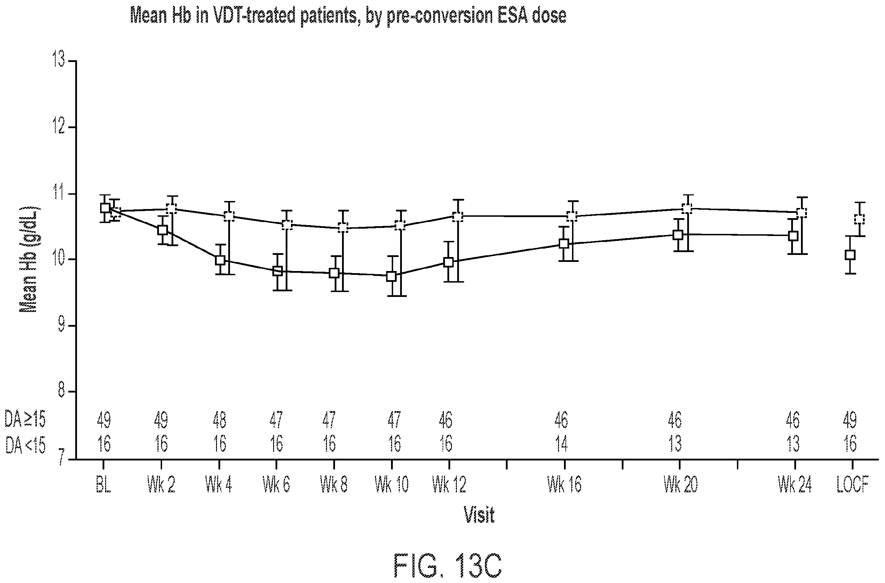

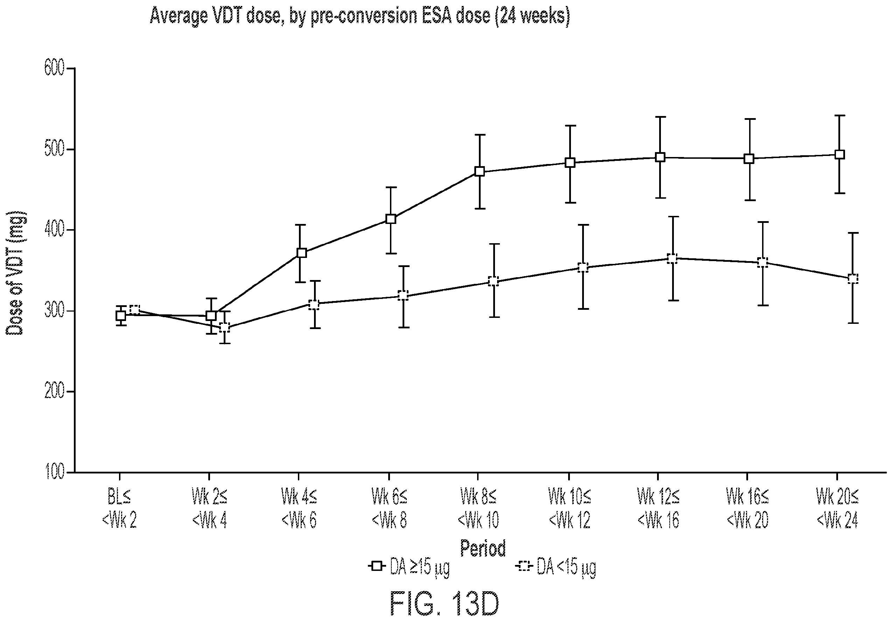

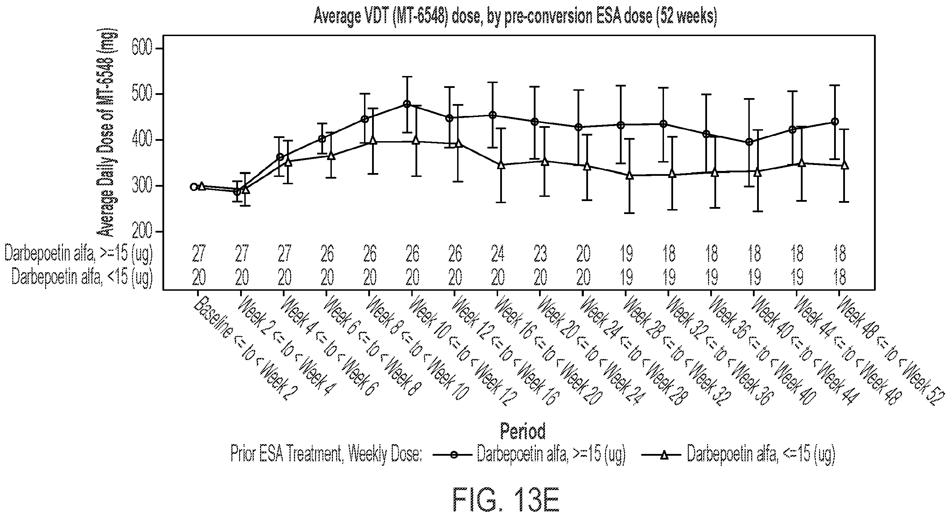

**FIGS. 13A-13D** show the effect of prior ESA treatment and dose on mean Hb over time in HD-CKD patients up to week 24 of the study. **FIG. 13A** shows the mean Hb in HD-CKD patients receiving Compound **1** (VDT) based on prior treatment with epoetin, darbepoetin alfa (DA), or epoetin beta pegol (EBP). **FIG. 13B** shows the average dose of Compound **1** (VDT) for HD-CKD patients based on prior treatment with epoetin, darbepoetin alfa (DA), or epoetin beta pegol (EBP). **FIG. 13C** shows the mean Hb in HD-CKD patients receiving Compound **1** (VDT) based on the pre-conversion dose of darbepoetin alfa (DA). **FIG. 13D** shows the average dose of Compound **1** (VDT) in HD-CKD patients based on the pre-conversion dose of darbepoetin alfa (DA) up to 24 weeks. **FIG. 13E** shows the average dose of Compound **1** (VDT, MT-6548) in HD-CKD patients based on the pre-conversion dose of darbepoetin alfa (DA) up to 52 weeks.
**FIG. 14** compares various iron related parameters for the overall HD-CKD population receiving therapy with Compound **1** and those receiving therapy with darbepoetin alfa (DA), including differences in serum ferritin (ng/mL) **(14A),** TSAT % **(14B),** TIBC (µg/dL) **(14C),** hepcidin (ng/mL) **(14D),** and the monthly dose of iron by any route (mg) **(14E).**
**FIG. 15** compares red blood cell indices MCV (mean corpuscular volume) **(15A);** MCH (mean corpuscular hemoglobin) **(15B);** MCHC (mean corpuscular hemoglobin concentration) **(15C);** and RDW (red cell distribution width) **(15D)** for Compound **1** and DA patients up to 52 weeks.
**FIG. 16** shows the average dose of Compound **1** in HD-CKD conversion subgroups based on the weekly dose of epoetin previously received by the patient.
**FIG. 17** shows the average dose of Compound **1** in HD-CKD conversion subgroups based on the weekly dose of darbepoetin alfa previously received by the patient.
**FIG. 18****.** shows the demographics and baseline characteristics of the overall patient population for the Phase III randomized, open-label, active-controlled NDD-CKD Conversion/Correction Study (J01).
**FIG. 19** shows the mean average dose of Compound **1** (MT-6548) in HD-CKD patients based on the pre-conversion dose of epoetin beta pegol.
**FIG. 20** shows the mean average dose of Compound **1** (MT-6548) in HD-CKD patients based on the pre-conversion dose of epoetin.
**FIG. 21** shows the mean Hb in HD-CKD patients receiving Compound **1** (VDT) based on the pre-conversion dose of epoetin.
**FIG. 22** shows the mean Hb in HD-CKD patients receiving Compound **1** (VDT) based on the pre-conversion dose of epoetin beta pegol.
**FIG. 23** shows that compound **1** maintained hemoglobin concentrations in subjects receiving hemodialysis who previously received ESAs.
**FIG. 24** shows the trial design for the treatment of anemia in patients with dialysis-dependent chronic kidney disease (DD-CKD).
**FIGS. 25A-25B** are the Compound **1** (vadadustat) dosing and Dose Adjustment Algorithms for the Correction/Conversion trial. **FIGS. 25C-25D** are the darbepoetin alfa dosing and Dose Adjustment Algorithms for the Correction/Conversion trial.
**FIGS. 26A-26B** are the Compound **1** (vadadustat) dosing and Dose Adjustment Algorithms for the Correction trial. **FIGS. 26C-26D** are the darbepoetin alfa dosing and Dose Adjustment Algorithms for the Correction Trial.
**FIGS. 27A-27B** show the median weekly dose of study treatment in safety population for Prevalent DD-CKD and Incident DD-CKD studies.
**FIGS. 28A-28B** show the mean change from baseline in hemoglobin levels in randomized population in Incident DD-CKD and Prevalent DD-CKD studies.
**FIG. 29** shows the trial design for the treatment of anemia in patients with nondialysis-dependent chronic kidney disease (NDD-CKD).

### DETAILED DESCRIPTION

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body by therapy. Described herein is an effective, durable method for the treatment of patients having anemia associated with chronic kidney disease (CKD), including methods suitable for conversion, correction, and maintenance therapy for patients. Any reference to methods or methods of treatment herein refer to the compound disclosed herein for use in such methods. For example, therapeutic benefits achieved with methods described herein are durable, with efficacy observed for long treatment periods such as about or at least six-months (24 weeks), about or at least a year (52 weeks), about or at least 5 years (260 weeks). Methods described herein can be generally useful for non-dialysis dependent patients (NDD-CKD) as well as patients receiving dialysis (DD-CKD). Additionally, methods described herein can be particularly beneficial for patients converting from a previous anemia treatment comprising administration of an erythropoietin stimulating agent (ESA), such as darbepoetin alfa (DA), and for patients with little or no exposure to ESA previously. Methods described herein also can be particularly effective for increasing as well as maintaining a target hemoglobin (Hb) level.

### Definitions

In order for the present invention to be more readily understood, certain terms are first defined below. Additional definitions for the following terms and other terms are set forth throughout the specification.

*Animal:* As used herein, the term "animal" refers to any member of the animal kingdom. In some embodiments, "animal" refers to humans, at any stage of development. In some embodiments, "animal" refers to non-human animals, at any stage of development. In embodiments, the non-human animal is a mammal (*e.g*., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically engineered animal, and/or a clone.

*Approximately or about:* As used herein, the term "approximately" or "about," as applied to one or more values of interest, refers to a value that is similar to a stated reference value. As described herein, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Dose(s):* As used herein, the term "dose(s)" means a quantity of the compound or a pharmaceutically acceptable salt, solvate, or hydrate thereof to be administered at one time. A dose may comprise a single unit dosage form, or alternatively may comprise more than a single unit dosage form (*e.g.*, a single dose may comprise two tablets), or even less than a single unit dosage form (*e.g*., a single dose may comprise half of a tablet). A dose described herein may be administered at various intervals. For example, a patient can receive a dose as described herein daily or weekly (*e.g*., once weekly or three times per week).

*Daily dose:* As used herein, the term "daily dose" means a quantity of the compound, or a pharmaceutically acceptable salt, solvate, or hydrate thereof that is administered in a 24-hour period. Accordingly, a daily dose may be administered all at once (*i.e*., once daily dosing) or alternatively the daily dosing may be divided such that administration of the compound is twice daily, three times daily, or even four times daily.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control sample or subject (or multiple control samples or subjects) in the absence of the treatment described herein. A "control subject" is a subject afflicted with the same form of disease as the subject being treated, who is about the same age as the subject being treated.

*In Vitro*: As used herein, the term "in vitro" refers to events that occur in an artificial environment, *e.g.,* in a test tube or reaction vessel, in cell culture, *etc.,* rather than within a multi-cellular organism.

*In Vivo*: As used herein, the term "in vivo" refers to events that occur within a multi-cellular organism, such as a human and a non-human animal. In the context of cell-based systems, the term may be used to refer to events that occur within a living cell (as opposed to, for example, in vitro systems).

*Patient*: As used herein, the term "patient" or "subject" refers to any organism to which a provided composition may be administered, *e.g*., for experimental, diagnostic, prophylactic, cosmetic, and/or therapeutic purposes. Typical patients include animals (*e.g*., mammals such as mice, rats, rabbits, non-human primates, and/or humans). In some embodiments, a patient is a human. A human includes pre- and post-natal forms.

*Pharmaceutically acceptable:* The term "pharmaceutically acceptable", as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Pharmaceutically acceptable salt*: Pharmaceutically acceptable salts are well known in the art. For example, S. M. Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid, or with organic acids such as acetic acid, trifluoroacetic acid, oxalic acid, maleic acid, tartaric acid, citric acid, succinic acid, or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄-alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, sulfonate, and aryl sulfonate. Further pharmaceutically acceptable salts include salts formed from the quarternization of an amine using an appropriate electrophile, *e.g*., an alkyl halide, to form a quarternized alkylated amino salt.

*Preventing*: The term "prevent," "preventing," or "prevention," as used herein refers to an effect that mitigates an undesired effect, *e.g*., an undesirable drug-drug interaction or the formation of a drug-iron chelate. Prevention does not require the 100% elimination of the possibility of an event. Rather, it denotes that the likelihood of the occurrence of the event has been reduced by the compound or method.

*Subject:* As used herein, the term "subject" refers to a human or any non-human animal (*e.g*., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutically effective amount*: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one-unit dose.

*Treating*: As used herein, the term "treat," "treatment," or "treating" refers to any method used to partially or completely alleviate, ameliorate, relieve, inhibit, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

As used herein, the term "HIF prolyl hydroxylase" is art-recognized and may be abbreviated as "PHD". HIF prolyl hydroxylase is also known as "prolyl hydroxylase domain-containing protein" which may be abbreviated as "PHD". In this regard, there are three different PHD isoforms, PHD1, PHD2, and PHD3, also referred to as EGLN2, EGLN1, and EGLN3, or HPH3, HPH2, and HPH1, respectively.

As used herein, the term "unit dosage form(s)" includes tablets; caplets; capsules, such as soft elastic gelatin capsules; sachets; cachets; troches; lozenges; dispersions; powders; solutions; gels; liquid dosage forms suitable for oral or mucosal administration to a patient, including suspensions (*e.g.,* aqueous or non-aqueous liquid suspensions), emulsions (*e.g.,* oil-in-water emulsions, or a water-in-oil liquid emulsion), solutions, and elixirs; and sterile solids (*e.g*., crystalline or amorphous solids) that can be reconstituted to provide liquid dosage forms suitable for oral or parenteral administration to a patient. The unit dosage form does not necessarily have to be administered as a single dose nor does a single unit dosage form necessarily constitute an entire dose.

Further abbreviations and acronyms are provided below.
- **ACTH**: adrenocorticotropic hormone
- **AE**: adverse event
- **ALT**: alanine aminotransferase (SGPT)
- **ANOVA**: analysis of variance
- **AST**: aspartate aminotransferase (SGOT)
- **BUN**: blood urea nitrogen
- **C**: Celsius
- **CBC**: complete blood count
- **CHF**: congestive heart failure
- **CKD**: chronic kidney disease
- **CKD-EPI**: Chronic Kidney Disease Epidemiology Collaboration
- **CMH**: Cochran-Mantel-Haenszel
- **CPK**: creatine phosphokinase
- **CRF**: case report form
- **CRO**: contract research organization
- **CS**: clinically significant
- **CV**: cardiovascular
- **CVD**: cardiovascular disease
- **dL**: deciliter
- **DVT**: deep venous thrombosis
- **EAC**: Endpoint Adjudication Committee
- **ECG**: electrocardiogram
- **EDC**: electronic data capture
- **eGFR**: estimated glomerular filtration rate
- **EOT**: end of treatment
- **EPO**: erythropoietin
- **ESA**: erythropoiesis-stimulating agent
- **ESRD**: end-stage renal disease
- **EU**: European Union
- **F**: Fahrenheit
- **FDA**: Food and Drug Administration
- **g**: gram
- **GCP**: Good Clinical Practice
- **GFR**: glomerular filtration rate
- **GMP**: Good Manufacturing Practice
- **HA**: health authority
- **HDL**: high-density lipoprotein
- **Hb**: hemoglobin
- **HIF**: hypoxia-inducible factor
- **HIFPH**: hypoxia-inducible factor prolyl-hydroxylase
- **HIF-PHI**: hypoxia-inducible factor prolyl-hydroxylase inhibitor
- **IC₅₀**: 50% inhibitory concentration
- **ICH**: International Conference on Harmonization
- **IDMC**: Independent Data Monitoring Committee
- **IDMS**: isotope dilution mass spectrometry
- **IEC**: independent ethics committee
- **INR**: international normalized ratio
- **IRB**: institutional review board
- **IV**: intravenous(ly)
- **IWR**: interactive web response
- **JSDT**: Japanese Society for Dialysis Therapy
- **JSN**: Japanese Society of Nephrology
- **KDIGO**: Kidney Disease: Improving Global Outcomes
- **kg**: kilogram
- **LDH**: lactate dehydrogenase
- **LDL**: low-density lipoprotein
- **LLN**: lower limit of normal
- **MACE**: major adverse cardiovascular events
- **MCH**: mean corpuscular (cell) hemoglobin
- **MCHC**: mean corpuscular (cell) hemoglobin concentration
- **MCV**: mean corpuscular (cell) volume
- **MedDRA**: Medical Dictionary for Regulatory Activities
- **µM**: micromolar
- **mg**: milligram
- **mL**: milliliter
- **mRNA**: messenger ribonucleic acid
- **MTD**: maximum tolerated dose
- **NDD-CKD**: non-dialysis dependent chronic kidney disease
- **ng**: nanogram
- **PD**: pharmacodynamics(s)
- **PE**: pulmonary embolism
- **PHD**: prolyl 4-hydroxylase domain
- **PK**: pharmacokinetic(s)
- **PP**: per protocol
- **PT**: prothrombin time
- **PTT**: partial thromboplastin time
- **QA**: quality assurance
- **QC**: quality control
- **RBC**: red blood cell
- **RDW**: red cell distribution width
- **ROW**: rest of world
- **SAE**: serious adverse event
- **SAP**: Statistical Analysis Plan
- **SC**: subcutaneous(ly)
- **SGOT**: serum glutamic oxaloacetic transaminase (AST)
- **SGPT**: serum glutamic pyruvic transaminase (ALT)
- **SmPC**: summary of product characteristics
- **SV**: Screening visit
- **TIBC**: total iron binding capacity
- **TREAT**: Trial to Reduce Cardiovascular Events with Aranesp Therapy
- **TSAT**: transferrin saturation
- **uACR**: urine albumin-to-creatinine ratio
- **ULN**: upper limit of normal
- **US**: United States
- **VEGF**: vascular endothelial growth factor
- **WBC**: white blood cell
- **WHO**: World Health Organization

### Anemia associated with chronic kidney disease (CKD)

Anemia commonly occurs in people with CKD-the permanent, partial loss of kidney function. Anemia might begin to develop in the early stages of CKD, when someone has 20 to 50 percent of normal kidney function. Anemia tends to worsen as CKD progresses. Most people who have total loss of kidney function, or kidney failure, have anemia. A person has kidney failure when he or she needs a kidney transplant or dialysis (*e.g*., hemodialysis or peritoneal dialysis) in order to live. When kidneys are diseased or damaged, they do not make enough EPO. As a result, the bone marrow makes fewer red blood cells, causing anemia. When blood has fewer red blood cells, it deprives the body of the oxygen it needs. Causes of anemia in people with kidney disease include blood loss from hemodialysis and low levels of the following nutrients found in food, such as iron, vitamin B12, and folic acid. Other causes of anemia in CKD patients include problems with bone marrow; inflammatory problems-such as arthritis, lupus, or inflammatory bowel disease-in which the body's immune system attacks the body's own cells and organs; chronic infections such as diabetic ulcers; and malnutrition.

Vadadustat ({[5-(3-chlorophenyl)-3-hydroxypyridine-2-carbonyl]amino}acetic acid; (Compound **1** or VDT or MT-6548) is a Hypoxia Inducible Factor Prolyl Hydroxylase inhibitor (HIF-PH inhibitor).

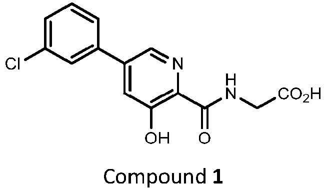

Compound **1** has emerged as a new drug that is highly useful for treating or preventing anemia secondary to or associated with chronic kidney disease, without prolonged, supraphysiologic erythropoietin (EPO) levels.

### Methods of Treatment and Prevention

As described herein, Compound **1** can result in effective and durable treatment of anemia in patients with CKD. For example, methods described herein can be effective in achieving and maintaining a target hemoglobin (Hb) level in patients receiving therapy with Compound **1.**

According to the invention, the patient with CKD has previously received treatment with an erythropoiesis-stimulating agent (ESA) within six weeks or eight weeks prior to or during a screening period before administering a dose of Compound **1.** ESAs include epoetin alfa, epoetin beta, epoetin beta pegol, and darbepoetin alfa (DA). In embodiments, a patient that will benefit from therapy with Compound **1** and/or an effective dose for a patient can be selected based on the identity and/or previously received dose amount of an ESA.

In embodiments, the patient with CKD is non-dialysis dependent (*i.e.*, a patient with NDD-CKD).

In embodiments, the patient with CKD is dialysis-dependent (*i.e*., a patient with DD-CKD).

In embodiments, the patient with CKD receives dialysis or previously has received dialysis. In embodiments, dialysis is hemodialysis (*e.g*., a patient with HD-CKD). In embodiments, dialysis is peritoneal dialysis (*e.g*., a patient with PD-CKD). In embodiments, the patient with CKD receives dialysis (*e.g*., hemodialysis or peritoneal dialysis). In embodiments, the patient with CKD previously received dialysis (*e.g*., hemodialysis or peritoneal dialysis).

In some examples, the patient receiving therapy with Compound **1** will experience sustained therapeutic benefit over a period of treatment that is at least about 24-52 weeks (*e.g*., at least about 24 weeks or at least about 52 weeks).

### Erythropoietin Stimulating Agents (ESA)

In some examples, a patient has not been previously treated with an erythropoiesis stimulating agent (ESA).

According to the invention, the methods described herein are beneficial to patients who have previously received treatment with an erythropoiesis-stimulating agent (ESA). Methods described herein can be particularly beneficial in achieving the desired therapeutic outcome while avoiding or reducing adverse effects associated with ESA therapy. Exemplary adverse effects may include cardiovascular events, rapid deterioration of kidney function, earlier requirement for dialysis, and vascular access failure.

*Darbepoetin alfa.* In embodiments, a patient previously received treatment with darbepoetin alfa (DA) (*e.g*., as described herein). In some embodiments, the DD-CKD patient was previously treated with 0.45 mcg/kg of darbepoetin alfa intravenously or subcutaneously weekly. In other embodiments the DD-CKD patient was previously treated with at least 0.75 mcg/kg of darbepoetin alfa intravenously or subcutaneously every 2 weeks. In some embodiments the NDD-CKD patient was previously treated with at least 0.45 mcg/kg of darbepoetin alfa intravenously or subcutaneously at 4-week intervals. Darbepoetin alfa is available in single dose vials as 25, 40, 60, 100, 200, 300, and 500 mcg/1 mL, and 150 mcg/0.75 mL. Darbepoetin alfa is also available as single-dose prefilled syringes as 25 mcg/0.42 mL, 40 mcg/0.4 mL, 60 mcg/0.3 mL, 100 mcg/0.5 mL, 150 mcg/0.3 mL, 200 mcg/0.4 mL, 300 mcg/0.6 mL, and 500 mcg/1 mL. In embodiments, a patient has been previously treated with darbepoetin alfa (DA) in a dosage amount of 0.45 mcg/kg to 0.75 mcg/kg once every four weeks. In embodiments, a patient has been previously treated with darbepoetin alfa (DA) in a dosage amount of 0.45 mcg/kg to 0.75 mcg/kg once every two weeks. In embodiments, a patient has been previously treated with darbepoetin alfa (DA) in a dosage amount of 0.45 mcg/kg to 0.75 mcg/kg once a week. In embodiments, a patient has been previously treated with darbepoetin alfa in a dosage amount of ≥ 15 µg weekly. In embodiments, a patient has been previously treated with darbepoetin alfa in a dosage amount of < 15 µg weekly.

*Epoetin alfa.* In embodiments, a patient previously received treatment with epoetin alfa (*e.g*., as described herein). In some embodiments, the DD-CKD patient or the NDD-CKD patient was previously treated with at least 50 to 100 Units/kg of epoetin alfa three times weekly. Preferably, intravenous route is recommended for patients on hemodialysis. Epoetin alfa is available as an injectable form as 2,000 Units/mL, 3,000 Units/mL, 4,000 Units/mL, and 10,000 Units/mL in single-dose vials; and as 20,000 Units/2 mL (10,000 Units/mL) and 20,000 Units/mL in multiple-dose vials containing benzyl alcohol. In embodiments, a patient has been previously treated with epoetin alfa in an amount of 10 U/kg to 500 U/kg 3 times weekly. In embodiments, a patient has been previously treated with epoetin alfa in an amount of 10 U/kg to 300 U/kg 3 times weekly. In embodiments, a patient has been previously treated with epoetin alfa in an amount of 50 U/kg to 300 U/kg 3 times weekly. In embodiments, a patient has been previously treated with epoetin alfa in an amount of 50 U/kg to 100 U/kg 3 times weekly.

*Epoetin beta.* In embodiments, a patient previously received treatment with epoetin beta (*e.g*., as described herein). In some embodiments, the DD-CKD patient or the NDD-CKD patient was previously treated with 20 IU/kg of epoetin beta three times weekly. In some embodiments, the DD-CKD patient or the NDD-CKD patient is at least 80 IU/kg of epoetin beta three times weekly. The preferable route for administration is intravenously. Epoetin beta is available as 500 IU, 2000 IU, 3000 IU, 4000 IU, 5000 IU, 6000 IU, 10,000 IU, 20,000 IU, and 30,000 IU solutions for injection as single-dose prefilled syringes.

*Epoetin beta pegol.* In embodiments, a patient previously received treatment with epoetin beta (*e.g*., as described herein). In some embodiments, the DD-CKD patient or the NDD-CKD patient was previously treated with 0.6 mcg/kg of epoetin beta pegol administered once every two weeks. The preferable route for administration is intravenously. Epoetin beta pegol is available for injection as prefilled syringes in 50, 75, 100, 150, 200, or 250 mcg in 0.3 mL solutions of epoetin beta pegol. In embodiments, a patient has been previously treated with epoetin beta pegol in a dosage amount of 0.6 mcg/kg to 1.20 mcg/kg once every two weeks. In embodiments, a patient has been previously treated with epoetin beta pegol in a dosage amount of 0.6 mcg/kg to 1.20 mcg/kg once monthly. In embodiments, a patient has been previously treated with epoetin beta pegol in a dosage amount of 0.6 mcg/kg once every two weeks. In embodiments, a patient has been previously treated with epoetin beta pegol in a dosage amount of 1.2 mcg/kg once every two weeks.

*Epoetin.* In embodiments, a patient previously received treatment with epoetin (*e.g.,* as described herein). In embodiments, a patient has been previously treated with epoetin at a dose of ≥ 4500 IU weekly. In embodiments, the patient has been previously treated with epoetin at a dose of <4500 IU.

### Dialysis Status

The methods described herein can be beneficial to patients of different dialysis status.

In embodiments, the patient's dialysis status can be used to select an initial dose of Compound **1.** In embodiments, the patient's dialysis status can be used to modify the dose of Compound **1** (*e.g.,* a dose increase within about six or eight weeks of commencing treatment with Compound **1).**

In embodiments, the patient is non-dialysis dependent. For example, in some embodiments, the patient with chronic kidney disease is non-dialysis dependent (a NDD-CKD patient).

In embodiments, the patient is dialysis-dependent. For example, in embodiments, the patient with chronic kidney disease is dialysis-dependent (a DD-CKD patient).

In embodiments, the patient receives or previously has received dialysis. In embodiments, the patient receives dialysis. In embodiments, the patient previously received dialysis.

In embodiments, dialysis is hemodialysis (HD). In embodiments, the patient with chronic kidney disease receives or previously received hemodialysis. In embodiments, the patient with chronic kidney disease receives hemodialysis. In embodiments, the patient with chronic kidney disease previously received hemodialysis.

In embodiments, dialysis is peritoneal dialysis (PD). In embodiments, the patient with chronic kidney disease receives or previously received peritoneal dialysis. In embodiments, the patient with chronic kidney disease receives peritoneal dialysis. In embodiments, the patient with chronic kidney disease previously received peritoneal dialysis.

### Correction and Maintenance

In one aspect, methods described herein are suitable for correction treatment and maintenance regimens. In embodiments, methods described herein are suitable for treating anemia associated with or secondary to chronic kidney disease (CKD). In embodiments, methods described herein are suitable for achieving, controlling and/or maintaining hemoglobin (Hb) levels within a target range.

In some examples, the correction regimen comprises administering Compound **1** to a patient who has not previously received treatment with an erythropoiesis-stimulating agent (ESA) or to a patient who has previously received treatment with an erythropoiesis-stimulating agent (ESA) but who has not received ESA treatment within eight (8) weeks of a screening period (*e.g*., a screening period that lasts no more than about four weeks) and/or receiving the first dose of Compound **1.**

In embodiments, the patient has previously received epoetin alfa (*e.g*., epoetin alfa in an amount ≤ 12,000 IU every two weeks), epoetin beta (*e.g*., epoetin beta in an amount ≤ 12,000 IU every two weeks), epoetin beta pegol (*e.g*., epoetin beta pegol in an amount ≤ 250 µg every four weeks), or darbepoetin alfa (DA) (*e.g*., darbepoetin alfa in an amount ≤ 120 µg every two weeks).

The subject can have an initial mean hemoglobin level that is ≥ 8.0 g/dL and < 11.0 g/dL.

In embodiments, the patient has non-dialysis dependent CKD (NDD-CKD).

In embodiments, the patient has dialysis dependent CKD (DD-CKD).

In embodiments, the patient with CKD receives dialysis or previously has received dialysis. In embodiments, dialysis is hemodialysis (HD-CKD). In embodiments, dialysis is peritoneal dialysis (PD-CKD). In embodiments, the patient with CKD receives dialysis (*e.g*., hemodialysis or peritoneal dialysis). In embodiments, the patient with CKD previously received dialysis (*e.g*., hemodialysis or peritoneal dialysis).

In embodiments, the patient has not received dialysis for at least about eight (8) weeks prior to commencing treatment with Compound **1.** In embodiments, the patient is not expected to receive dialysis during treatment with Compound **1.**

In embodiments, methods described herein are suitable for increasing, controlling, and/or maintaining hemoglobin (Hb) levels within a target range. In embodiments, the Hb range is 11-13 g/dL (*e.g*., for non-dialysis dependent CKD patients).

In embodiments, the patient receives a daily dose of 150-600 mg Compound **1.** In embodiments, Compound **1** is administered orally to the patient. In embodiments, Compound **1** is in a unit dosage form (*e.g*., a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, the dose is 600 mg Compound **1.**

In embodiments, the patient receives a dose of 150-600 mg Compound **1** about once weekly. In embodiments, Compound **1** is administered orally to the patient. In embodiments, Compound **1** is in a unit dosage form (*e.g*., a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, a dose is 600 mg Compound **1.**

In embodiments, the patient receives a dose of 150-600 mg Compound **1** about three times per week. In embodiments, Compound 1 is administered orally to the patient. In embodiments, Compound **1** is in a unit dosage form (*e.g*., a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, the dose is 600 mg Compound **1.**

In embodiments, the patient receives therapy with Compound **1** for at least 54, 56, 58, or 60 weeks. In some examples, the patient receives therapy with Compound **1** for at least about 6-24, or 6-12 months. In embodiments, the patient receives therapy with Compound **1** for at least 13, 14, 15, 16, 17, 18, 19, 20, 21, 22, 23, or 24 months. In some examples, the patient receives therapy with Compound **1** for at least about twenty-four (24) weeks or about six-months. In some examples, the patient receives therapy with Compound **1** for at least about fifty-two (52) weeks or about 12-months.

In embodiments, the patient receives an initial dose of Compound **1** that is 300 mg. In embodiments, a patient receives an initial dose of Compound **1** that is at least 300 mg (*e.g.,* 300 mg Compound **1,** 450 mg Compound **1,** or 600 mg Compound **1).** In embodiments, the patient receives a dose adjustment to a dose of at 450 mg or to at 600 mg within about four weeks of commencing therapy with Compound **1.** In embodiments, the patient receives a dose adjustment to a dose of at 450 mg or to at 600 mg within about six weeks of commencing therapy with Compound **1.** In embodiments, the patient receives a dose adjustment to a dose of at 450 mg or to at 600 mg within eight weeks of commencing therapy with Compound **1.** In embodiments, the patient receives a dose adjustment to a dose of at 450 mg or to at 600 mg within about ten weeks of commencing therapy with Compound **1.** In embodiments, the patient receives a dose adjustment to a dose of at 450 mg or to at 600 mg within twelve weeks of commencing therapy with Compound **1.** In embodiments, the patient receives a dose once daily (a daily dose). In embodiments, the patient receives a dose once weekly. In embodiments, the patient receives a dose three times per week.

In embodiments, the patient receives an initial daily dose of Compound **1** that is 300 mg. In embodiments, the patient receives an initial daily dose of Compound **1** that is at least 300 mg (*e.g.,* 300 mg Compound **1,** 450 mg Compound **1,** or 600 mg Compound **1).** In embodiments, the patient receives an initial daily dose of Compound **1** that is 300 mg. In embodiments, the patient receives a dose adjustment to a daily dose of at 450 mg or to at 600 mg within about four weeks of commencing therapy with Compound **1.** In embodiments, the patient receives a dose adjustment to a daily dose of at 450 mg or to at 600 mg within about six weeks of commencing therapy with Compound **1.** In embodiments, the patient receives a dose adjustment to a daily dose of at 450 mg or to at 600 mg within about eight weeks of commencing therapy with Compound **1.** In embodiments, the patient receives a dose adjustment to a daily dose of at 450 mg or to at 600 mg within about ten weeks of commencing therapy with Compound **1.** In embodiments, the patient receives a dose adjustment to a daily dose of at 450 mg or to at 600 mg within about twelve weeks of commencing therapy with Compound **1.**

In embodiments, the patient receives an initial dose of Compound **1** that is 150 mg. In embodiments, the patient receives an initial daily dose of Compound **1** that is 150 mg.

In embodiments, the patient receives an initial dose of Compound **1** that is 300 mg. In embodiments, the patient receives an initial daily dose of Compound **1** that is 300 mg.

In embodiments, the patient receives an initial dose of Compound **1** that is 450 mg. In embodiments, the patient receives an initial daily dose of Compound **1** that is 450 mg.

In embodiments, the patient receives an initial dose of Compound **1** that is 600 mg. In embodiments, the patient receives an initial daily dose of Compound **1** that is 600 mg.

In embodiments, the patient receives a dose of Compound **1** that is at least 300 mg (*e.g.,* 300 mg Compound **1,** 450 mg Compound **1,** or 600 mg Compound **1)** for at least four consecutive weeks. In embodiments, the patient receives a dose of Compound **1** that is at least 300 mg (*e.g.,* 300 mg Compound **1,** 450 mg Compound **1,** or 600 mg Compound **1)** for at least six consecutive weeks. In embodiments, the patient receives a dose of Compound **1** that is at least 300 mg (*e.g.,* 300 mg Compound **1, 450** mg Compound **1,** or 600 mg Compound **1)** for at least eight consecutive weeks. In embodiments, the patient receives a dose of Compound **1** that is at least 300 mg (*e.g.,* 300 mg Compound **1, 450** mg Compound **1,** or 600 mg Compound **1)** for ten consecutive weeks. In embodiments, the patient receives a dose of Compound **1** that is at least 300 mg (*e.g.,* 300 mg Compound **1,** 450 mg Compound **1,** or 600 mg Compound **1)** for at least twelve consecutive weeks. In embodiments, the period of consecutive weeks begins within four weeks of commencing therapy with Compound **1.** In embodiments, the period of consecutive weeks begins within six weeks of commencing therapy with Compound **1.** In embodiments, the period of consecutive weeks begins within eight weeks of commencing therapy with Compound **1.** In embodiments, the period of consecutive weeks begins within ten weeks of commencing therapy with Compound **1.** In embodiments, the period of consecutive weeks begins within twelve weeks of commencing therapy with Compound **1.** In embodiments, the patient receives a dose once daily (a daily dose). In embodiments, the patient receives a dose once weekly. In embodiments, the patient receives a dose three times per week. In embodiments, the dose is 300 mg Compound **1.** In embodiments, the dose is 450 mg Compound **1.** In embodiments, the dose is 600 mg Compound **1.**

In embodiments, the patient receives a daily dose of Compound **1** that is at least 300 mg (*e.g.,* 300 mg Compound **1,** 450 mg Compound **1,** or 600 mg Compound **1)** for at least four consecutive weeks. In embodiments, the patient receives a daily dose of Compound **1** that is at least 300 mg (*e.g.,* 300 mg Compound **1,** 450 mg Compound **1,** or 600 mg Compound **1)** for at least six consecutive weeks. In embodiments, the patient receives a daily dose of Compound **1** that is at least 300 mg (*e.g.,* 300 mg Compound **1,** 450 mg Compound **1,** or 600 mg Compound **1)** for at least eight consecutive weeks. In embodiments, the patient receives a daily dose of Compound **1** that is at least at least 300 mg (*e.g.,* 300 mg Compound **1,** 450 mg Compound **1,** or 600 mg Compound **1)** for ten consecutive weeks. In embodiments, the patient receives a daily dose of Compound **1** that is at least 300 mg (*e.g.,* 300 mg Compound **1,** 450 mg Compound **1,** or 600 mg Compound **1** for at least twelve consecutive weeks. In embodiments, the period of consecutive weeks begins within four weeks of commencing therapy with Compound **1.** In embodiments, the period of consecutive weeks begins within six weeks of commencing therapy with Compound **1.** In embodiments, the period of consecutive weeks begins within eight weeks of commencing therapy with Compound **1.** In embodiments, the period of consecutive weeks begins within ten weeks of commencing therapy with Compound **1.** In embodiments, the period of consecutive weeks begins within twelve weeks of commencing therapy with Compound **1.** In embodiments, the dose is 300 mg Compound **1.** In embodiments, the dose is 450 mg Compound **1.** In embodiments, the dose is 600 mg Compound **1.**

In another aspect, methods of the invention also include methods of selecting and/or treating patients for treatment that can particularly benefit from therapy with Compound **1.** In another aspect, methods of the invention also include methods of selecting and/or treating patients for treatment that can particularly benefit from higher doses of Compound **1** (*e.g*., therapy with doses of 450 mg or 600 mg of Compound **1** such as daily doses of 450 mg or 600 mg of Compound **1).**

For example, selecting and/or treating can be based on a previous ESA therapy (including a previous ESA therapy dose amount) received by a patient and/or the hemoglobin level of a patient.

In embodiments, the method comprises administering a dose of Compound **1** to the patient, wherein the dose is selected based on the initial hemoglobin (Hb) level of a patient. In embodiments, the method comprises selecting a patient based on the initial hemoglobin (Hb) level of a patient and administering to the patient a dose of Compound **1** based on said initial hemoglobin (Hb) level. In embodiments, the patient receives a dose once daily (a daily dose). In embodiments, the patient receives a dose once weekly. In embodiments, the patient receives a dose three times per week. In embodiments, the dose (*e.g*., a daily dose) is 300 mg Compound **1.** In embodiments, the dose (*e.g.,* a daily dose) is 450 mg Compound **1.** In embodiments, the dose (*e.g.,* a daily dose) is 600 mg Compound **1.**

In embodiments, the method comprises administering a daily dose of Compound **1** to the patient, wherein the daily dose is selected based on the initial hemoglobin (Hb) level of a patient. In embodiments, the method comprises selecting a patient based on the initial hemoglobin (Hb) level of a patient and administering to the patient a daily dose of Compound **1** based on said initial hemoglobin (Hb) level. In embodiments, the daily dose is 300 mg Compound **1.** In embodiments, the daily dose is 450 mg Compound **1.** In embodiments, the daily dose is 600 mg Compound **1.**

In embodiments, the patient has an initial hemoglobin level of ≤ 11 g/dL. In embodiments, the method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1 or** within 4, 6, 8, 10, or 12 weeks from the time of commencing treatment with Compound **1** as described herein. In some examples, the method comprises administering to a patient a dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient receives a dose about once daily (a daily dose). In embodiments, the patient receives a dose about once weekly. In embodiments, the patient receives a dose about three times per week. In embodiments, the dose (*e.g.,* a daily dose) is 300 mg Compound **1.** In embodiments, the dose (*e.g.,* a daily dose) is 450 mg Compound **1.** In embodiments, a dose (*e.g*., a daily dose) is 600 mg Compound **1.**

In embodiments, the patient has an initial hemoglobin level of ≤ 11 g/dL. In embodiments, the method comprises administering to a patient a daily dose of Compound **1** that is at least 300 mg (*e.g.,* 300 mg Compound **1,** 450 mg Compound **1,** or 600 mg Compound **1)** at the time of commencing treatment with Compound **1** or within 4, 6, 8, 10, or 12 weeks from the time of commencing treatment with Compound **1** as described herein. In some examples, the method comprises administering to a patient a daily dose of Compound **1** that is at least about 300 mg (*e.g.,* about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least twenty-four weeks (six-months) or at least fifty-two weeks (12-months) as described herein. In embodiments, the daily dose is 300 mg Compound **1.** In embodiments, a daily dose is 450 mg Compound **1.** In embodiments, the daily dose is 600 mg Compound **1.**

In embodiments, the method comprises administering a dose of Compound **1** to the patient, wherein the dose is selected based on a previous ESA therapy received by the patient. In embodiments, the method comprises administering a dose of Compound **1** to the patient, wherein the dose is selected based on the dose of a previous ESA therapy received by the patient. In embodiments, the patient receives a dose once daily (a daily dose). In embodiments, the patient receives a dose once weekly. In embodiments, the patient receives a dose three times per week.

In embodiments, the method comprises administering a daily dose of Compound **1** to the patient, wherein the daily dose is selected based on a previous ESA therapy received by the patient. In embodiments, the method comprises administering a daily dose of Compound **1** to the patient, wherein the daily dose is selected based on the dose of a previous ESA therapy received by the patient.

In embodiments, the method comprises selecting a patient based on a previous ESA therapy received by the patient and administering to the patient a daily dose of Compound **1** based on the previous ESA therapy received by the patient. In embodiments, the method comprises selecting a patient based on a previous ESA therapy received by the patient and administering to the patient a daily dose of Compound **1** based on the previous dose of ESA therapy received by the patient.

In embodiments, the patient has previously received epoetin alfa (*e.g*., epoetin alfa in an amount ≤ 12,000 IU every two weeks), epoetin beta (*e.g*., epoetin beta in an amount ≤ 12,000 IU every two weeks), epoetin beta pegol (*e.g*., epoetin beta pegol in an amount ≤ 250 µg every four weeks), or darbepoetin alfa (DA) (*e.g*., darbepoetin alfa in an amount ≤ 120 µg every two weeks).

In embodiments, the patient has previously received darbepoetin alfa (DA). In embodiments, the patient has previously received DA at a dose level of ≥ 15 µg (*e.g*., weekly). In embodiments, the method comprises administering to a patient a dose of Compound 1 that is at least 300 mg *(e.g.,* 300 mg Compound **1,** 450 mg Compound **1,** or 600 mg Compound 1) at the time of commencing treatment with Compound 1 or within about 4, 6, 8, 10, or 12 weeks from the time of commencing treatment with Compound 1 as described herein. In some examples, the method comprises administering to a patient a dose of Compound 1 that is at least about 300 mg (e.g., about 300 mg Compound **1,** about 450 mg Compound **1,** or about 600 mg Compound 1) for at least about 6, 8, 10, 12, 14, 16, 18, or 20 consecutive weeks as described herein. In embodiments, the patient receives treatment with Compound 1 for at least 54, 56, 58, or 60 weeks or at least 13, 14, 15, 16, 17, 18, 19, 20, 21, 22, 23, or 24 months as described herein. In some examples, the patient receives therapy with Compound 1 for at least about twenty-four weeks (about six-months) or at least about fifty-two weeks (about 12-months) as described herein. In embodiments, the patient has NDD-CKD. In embodiments, the patient has DD-CKD. In embodiments, the patient receives a dose once daily (a daily dose). In embodiments, the patient receives a dose once weekly. In embodiments, the patient receives a dose three times per week. In embodiments, the dose (*e.g*., a daily dose) is 300 mg Compound **1.** In embodiments, the dose (*e.g*., a daily dose) is 450 mg Compound **1.** In embodiments, the dose (*e.g*., a daily dose) is 600 mg Compound **1.**

In embodiments, the patient has previously received darbepoetin alfa (DA). In embodiments, the patient has previously received DA at a dose level of ≥ 15 µg (*e.g*., weekly). In embodiments, the method comprises administering to a patient a daily dose of Compound **1** that is at least 300 mg (*e.g.,* 300 mg Compound **1,** 450 mg Compound **1,** or 600 mg Compound **1)** at the time of commencing treatment with Compound **1 or** within 4, 6, 8, 10, or 12 weeks from the time of commencing treatment with Compound **1** as described herein. In some examples, the method comprises administering to a patient a daily dose of Compound **1** that is at least about 300 mg (*e.g.,* about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, the patient has DD-CKD. In embodiments, the daily dose is 300 mg Compound **1.** In embodiments, the daily dose is 450 mg Compound **1.** In embodiments, the daily dose is 600 mg Compound **1.**

In embodiments, the patient has previously received epoetin (*e.g*., epoetin alfa or epoetin beta). In embodiments, the patient has previously received epoetin (*e.g*., epoetin alfa or epoetin beta) at a dose level of ≥ 4500 IU (*e.g*., weekly). In embodiments, the method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, the method comprises administering to a patient a daily dose of Compound **1** that is at least about 300 mg (*e.g.*, about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at 13, 14, 15, 16, 17, 18, 19, 20, 21, 22, 23, or 24 months as described herein. In some examples, the patient receives therapy with Compound 1 for at least about twenty-four weeks (about six-months) or at least about fifty-two weeks (about 12-months) as described herein. In embodiments, the patient has NDD-CKD. In embodiments, the patient has DD-CKD. In embodiments, the patient receives a dose about once daily (a daily dose). In embodiments, the patient receives a dose once weekly. In embodiments, the patient receives a dose three times per week. In embodiments, the dose (*e.g*., a daily dose) is 300 mg Compound **1.** In embodiments, the dose (*e.g.,* a daily dose) is 450 mg Compound **1.** In embodiments, the dose (*e.g.,* a daily dose) is 600 mg Compound **1.**

In embodiments, the patient has previously received epoetin (*e.g.*, epoetin alfa or epoetin beta). In embodiments, the patient has previously received epoetin (*e.g*., epoetin alfa or epoetin beta) at a dose level of ≥ 4500 IU (*e.g*., weekly). In embodiments, the method comprises administering to a patient a daily dose of Compound **1** that is at least 450 mg (*e.g.,* 450 mg or 600 mg) at the time of commencing treatment with Compound **1** or within about 4, 6, 8, 10, or 12 weeks from the time of commencing treatment with Compound **1** as described herein. In some examples, the method comprises administering to a patient a daily dose of Compound **1** that is at least about 450 mg (*e.g*., about 450 mg or about 600 mg) for at least about 6, 8, 10, 12, 14, 16, 18, or 20 consecutive weeks as described herein. In embodiments, the patient receives treatment with Compound **1** for at least 54, 56, 58, or 60 weeks; or at 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, the patient has DD-CKD. In embodiments, the daily dose is 300 mg Compound **1.** In embodiments, the daily dose is 450 mg Compound **1.** In embodiments, the daily dose is 600 mg Compound **1.**

### Conversion and Maintenance

In embodiments, methods described herein are suitable for conversion treatment and maintenance regimens. In embodiments, methods described herein are suitable for treating anemia associated with or secondary to chronic kidney disease (CKD). In embodiments, methods described herein are suitable for achieving, controlling, and/or maintaining hemoglobin (Hb) levels within a target range.

In embodiments, a conversion regimen comprises administering Compound **1** to a patient who has previously received treatment with an erythropoiesis-stimulating agent (ESA) within eight (8) weeks of screening and/or receiving the first dose of Compound **1.** In embodiments, Compound **1** is administered to a patient who has previously received treatment with an erythropoiesis-stimulating agent (ESA) within eight (8) weeks of screening and/or receiving the first dose of Compound **1.**

In embodiments, the patient has previously received epoetin alfa (*e.g*., epoetin alfa in an amount ≤ 12,000 IU every two weeks for NDD-CKD patients or ≤ 9,000 IU weekly for DD-CKD patients), epoetin beta (*e.g*., epoetin beta in an amount ≤ 12,000 IU every two weeks for NDD-CKD patients or ≤ 9,000 IU weekly for DD-CKD patients), epoetin beta pegol (*e.g.*, epoetin beta pegol in an amount ≤ 250 µg every four weeks for NDD-CKD patients or DD-CKD patients), or darbepoetin alfa (DA) (*e.g*., darbepoetin alfa in an amount ≤ 120 µg every two weeks for NDD-CKD patients or ≤ 60 µg weekly for DD-CKD patients). In embodiments, the patient was previously treated with darbepoetin alfa.

In embodiments, the patient was previously treated with epoetin alfa in a dosage amount of 50 U/kg to 300 U/kg three times weekly. In embodiments, the patient was previously treated with darbepoetin alfa in a dosage amount of 0.45 mcg/kg to 0.75 mcg/kg once every four weeks. In embodiments, the patient was previously treated with darbepoetin alfa in a dosage amount of 0.45 mcg/kg to 0.75 mcg/kg once every two weeks. In embodiments, the patient was previously treated with darbepoetin alfa in a dosage amount of 0.45 mcg/kg to 0.75 mcg/kg once a week.

In embodiments, the patient has non-dialysis dependent CKD (NDD-CKD). In embodiments, a subject has an initial mean hemoglobin level that is ≥ 8.0 g/dL and < 11.0 g/dL.

In embodiments, the patient has dialysis-dependent CKD (DD-CKD). In embodiments, a patient has an initial mean hemoglobin level that is ≥ 8.5 g/dL and < 12.0 g/dL.

In embodiments, the patient has CKD and receives dialysis or previously has received dialysis. In embodiments, the patient receives dialysis. In embodiments, the patient has previously received dialysis.

In embodiments, dialysis is hemodialysis (HD). In embodiments, the patient receives hemodialysis (HD). In embodiments, the patient has previously received hemodialysis (HD).

In embodiments, dialysis is peritoneal dialysis (PD). In embodiments, the patient receives peritoneal dialysis (PD). In embodiments, the patient has previously received peritoneal dialysis (PD).

In embodiments, the patient has CKD and receives or previously has received dialysis. In embodiments, the patient has an initial mean hemoglobin level that is ≥ 8.5 g/dL and < 12.0 g/dL.

In embodiments, the patient has CKD and receives or previously has received hemodialysis (HD-CKD). In embodiments, the patient has an initial mean hemoglobin level that is ≥ 8.5 g/dL and < 12.0 g/dL.

In embodiments, the patient has CKD and receives or previously has received peritoneal dialysis (PD-CKD). In embodiments, the patient has an initial mean hemoglobin level that is ≥ 8.5 g/dL and < 12.0 g/dL.

In embodiments, the patient has not received dialysis for at least eight (8) weeks prior to commencing treatment with Compound **1** or prior to a screening period (*e.g*., a screening period that lasts no more than about four weeks) prior to commencing treatment with Compound **1.** In embodiments, the patient is not expected to receive dialysis during treatment with Compound **1.**

In embodiments, methods described herein are suitable for achieving, controlling, and/or maintaining hemoglobin (Hb) levels within a target range. In embodiments, the Hb range is 11-13 g/dL (*e.g*., for non-dialysis dependent CKD patients). In embodiments, the Hb range is 10-12 g/dL (*e.g.,* for DD-CKD patients). In embodiments, the Hb range is 10-12 g/dL (*e.g.,* for CKD patients receiving dialysis such as hemodialysis or peritoneal dialysis).

In embodiments, the patient receives a daily dose of 150-600 mg Compound **1.** In embodiments, Compound **1** is administered orally to the patient. In embodiments, Compound **1** is in a unit dosage form (*e.g*., a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, the dose is 600 mg Compound **1.**

In embodiments, the patient receives a dose of 150-600 mg Compound **1** about once weekly. In embodiments, Compound **1** is administered orally to the patient. In embodiments, Compound **1** is in a unit dosage form (*e.g*., a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, the dose is 600 mg Compound **1.**

In embodiments, the patient receives a dose of 150-600 mg Compound **1** three times per week. In embodiments, Compound **1** is administered orally to the patient. In embodiments, Compound **1** is in a unit dosage form (*e.g.*, a unit dosage form formulated for oral administration such as tablets or capsules). In embodiments, the unit dosage form comprises 150 mg or 300 mg of Compound **1.** In embodiments, the dose is 150 mg Compound **1.** In embodiments, the dose is 300 mg Compound **1.** In embodiments, the dose is 450 mg Compound **1.** In embodiments, the dose is 600 mg Compound **1.**

In embodiments, the patient receives therapy with Compound **1** for at least 54, 56, 58, or 60 weeks. In some examples, the patient receives therapy with Compound **1** for at least about 6-24, 6-12, or 12-24 months. In embodiments, the patient receives therapy with Compound **1** for at least 13, 14, 15, 16, 17, 18, 19, 20, 21, 22, 23, or 24 months. In some examples, the patient receives therapy with Compound **1** for at least about twenty-four (24) weeks or about six-months. In some examples, the patient receives therapy with Compound **1** for at least about fifty-two (52) weeks or about 12-months.

In embodiments, the patient receives an initial dose of Compound **1** that is 300 mg. In embodiments, the patient receives an initial dose of Compound **1** that is at least 300 mg (*e.g.,* 300 mg Compound **1,** 450 mg Compound **1,** or 600 mg Compound **1).** In embodiments, the patient receives a dose adjustment to a dose of at 450 mg or to at 600 mg within four weeks of commencing therapy with Compound **1.** In embodiments, the patient receives a dose adjustment to a dose of at 450 mg or to at 600 mg within six weeks of commencing therapy with Compound **1.** In embodiments, the patient receives a dose adjustment to a dose of at 450 mg or to at 600 mg within eight weeks of commencing therapy with Compound **1.** In embodiments, the patient receives a dose adjustment to a dose of at 450 mg or to at 600 mg within ten weeks of commencing therapy with Compound **1.** In embodiments, the patient receives a dose adjustment to a dose of at 450 mg or to at 600 mg within twelve weeks of commencing therapy with Compound **1.** In embodiments, the patient receives a dose once daily (a daily dose). In embodiments, the patient receives a dose once weekly. In embodiments, the patient receives a dose three times per week.

In embodiments, the patient receives an initial daily dose of Compound **1** that is 300 mg. In embodiments, the patient receives an initial daily dose of Compound **1** that is at least 300 mg (*e.g.,* 300 mg Compound **1,** 450 mg Compound **1,** or 600 mg Compound **1).** In embodiments, a patient receives an initial daily dose of Compound **1** that is 300 mg. In embodiments, the patient receives a dose adjustment to a daily dose of at 450 mg or to at 600 mg within four weeks of commencing therapy with Compound **1.** In embodiments, the patient receives a dose adjustment to a daily dose of at 450 mg or to at 600 mg within six-weeks of commencing therapy with Compound **1.** In embodiments, the patient receives a dose adjustment to a daily dose of at 450 mg or to at 600 mg within eight-weeks of commencing therapy with Compound **1.** In embodiments, the patient receives a dose adjustment to a daily dose of at 450 mg or to at 600 mg within ten weeks of commencing therapy with Compound **1.** In embodiments, the patient receives a dose adjustment to a daily dose of at 450 mg or to at 600 mg within about twelve weeks of commencing therapy with Compound **1.**

In embodiments, the patient receives an initial dose of Compound **1** that is 150 mg. In embodiments, the patient receives an initial daily dose of Compound **1** that is 150 mg.

In embodiments, the patient receives an initial dose of Compound **1** that is 300 mg. In embodiments, the patient receives an initial daily dose of Compound **1** that is 300 mg.

In embodiments, the patient receives an initial dose of Compound **1** that is 450 mg. In embodiments, the patient receives an initial daily dose of Compound **1** that is 450 mg.

In embodiments, the patient receives an initial dose of Compound **1** that is 600 mg. In embodiments, the patient receives an initial daily dose of Compound **1** that is 600 mg.

In some examples, the patient receives a dose of Compound **1** that is at least about 300 mg (*e.g*., about 300 mg Compound **1,** about 450 mg Compound **1,** or about 600 mg Compound **1)** for at least about four consecutive weeks. In some examples, the patient receives a dose of Compound **1** that is at least about 300 mg (*e.g*., about 300 mg Compound **1,** about 450 mg Compound **1,** or about 600 mg Compound **1)** for at least about six consecutive weeks. In some examples, the patient receives a dose of Compound **1** that is at least about 300 mg (*e.g*., about 300 mg Compound **1,** about 450 mg Compound **1,** or about 600 mg Compound **1)** for at least about eight consecutive weeks. In some examples, the patient receives a dose of Compound **1** that is at least about 300 mg (*e.g*., about 300 mg Compound **1,** about 450 mg Compound **1,** or about 600 mg Compound **1)** for about ten consecutive weeks. In some examples, the patient receives a dose of Compound **1** that is at least about 300 mg (*e.g*., about 300 mg Compound **1,** about 450 mg Compound **1,** or about 600 mg Compound **1)** for at least about twelve consecutive weeks. In embodiments, the period of consecutive weeks begins within about four weeks of commencing therapy with Compound **1.** In embodiments, the period of consecutive weeks begins within about six weeks of commencing therapy with Compound **1.** In embodiments, the period of consecutive weeks begins within about eight weeks of commencing therapy with Compound **1.** In embodiments, the period of consecutive weeks begins within about ten weeks of commencing therapy with Compound **1.** In embodiments, the period of consecutive weeks begins within about twelve weeks of commencing therapy with Compound **1.** In embodiments, the patient receives a dose about once daily (a daily dose). In embodiments, the patient receives a dose about once weekly. In embodiments, the patient receives a dose about three times per week. In embodiments, the dose is 300 mg Compound **1.** In embodiments, the dose is 450 mg Compound **1.** In embodiments, the dose is 600 mg Compound **1.**

In some examples, the patient receives a daily dose of Compound **1** that is at least about 300 mg (*e.g*., about 300 mg Compound **1,** about 450 mg Compound **1,** or about 600 mg Compound **1)** for at least about four consecutive weeks. In some examples, the patient receives a daily dose of Compound **1** that is at least about 300 mg (*e.g*., about 300 mg Compound **1,** about 450 mg Compound **1,** or about 600 mg Compound **1)** for at least about six consecutive weeks. In some examples, the patient receives a daily dose of Compound **1** that is at least about 300 mg (*e.g*., about 300 mg Compound **1,** about 450 mg Compound **1,** or about 600 mg Compound **1)** for at least about eight consecutive weeks. In some examples, the patient receives a daily dose of Compound **1** that is at least at least about 300 mg (*e.g*., about 300 mg Compound **1,** about 450 mg Compound **1,** or about 600 mg Compound **1)** for about ten consecutive weeks. In some examples, the patient receives a daily dose of Compound **1** that is at least about 300 mg (*e.g*., about 300 mg Compound **1,** about 450 mg Compound **1,** or about 600 mg Compound **1** for at least about twelve consecutive weeks. In embodiments, the period of consecutive weeks begins within about four weeks of commencing therapy with Compound **1.** In embodiments, the period of consecutive weeks begins within about six weeks of commencing therapy with Compound **1.** In embodiments, the period of consecutive weeks begins within about eight weeks of commencing therapy with Compound **1.** In embodiments, the period of consecutive weeks begins within about ten weeks of commencing therapy with Compound **1.** In embodiments, the period of consecutive weeks begins within about twelve weeks of commencing therapy with Compound **1.** In embodiments, the dose is 300 mg Compound **1.** In embodiments, the dose is 450 mg Compound **1.** In embodiments, the dose is 600 mg Compound **1.**

In another example, methods are described that include methods of selecting and/or treating patients for treatment that can particularly benefit from therapy with Compound **1.** In another example, methods are described that also include methods of selecting and/or treating patients for treatment that can particularly benefit from higher doses of Compound **1** (*e.g.*, therapy with doses of about 450 mg or about 600 mg of Compound **1** such as daily doses of about 450 mg or about 600 mg of Compound **1).**

For example, selecting and/or treating can be based on a previous ESA therapy (including a previous ESA therapy dose amount) received by a patient and/or the hemoglobin level of a patient.

In some examples, the method comprises administering a dose of Compound **1** to the patient, wherein the dose is selected based on the initial hemoglobin (Hb) level of a patient. In some examples, the method comprises selecting a patient based on the initial hemoglobin (Hb) level of a patient and administering to the patient a dose of Compound **1** based on said initial hemoglobin (Hb) level. In embodiments, a patient receives a dose about once daily (a daily dose). In embodiments, the patient receives a dose about once weekly. In embodiments, the patient receives a dose about three times per week. In embodiments, the dose (*e.g*., a daily dose) is 300 mg Compound **1.** In embodiments, the dose (*e.g.,* a daily dose) is 450 mg Compound **1.** In embodiments, the dose (*e.g.,* a daily dose) is 600 mg Compound **1.**

In some examples, the method comprises administering a daily dose of Compound **1** to the patient, wherein the daily dose is selected based on the initial hemoglobin (Hb) level of a patient. In some examples, the method comprises selecting a patient based on the initial hemoglobin (Hb) level of a patient and administering to the patient a daily dose of Compound **1** based on said initial hemoglobin (Hb) level. In embodiments, the daily dose is 300 mg Compound **1.** In embodiments, the daily dose is 450 mg Compound **1.** In embodiments, the daily dose is 600 mg Compound **1.**

In embodiments, the patient has an initial hemoglobin level of ≤ 11 g/dL. In embodiments, the method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, the method comprises administering to a patient a dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient receives a dose about once daily (a daily dose). In embodiments, the patient receives a dose once weekly. In embodiments, the patient receives a dose three times per week. In embodiments, the dose (*e.g*., a daily dose) is 300 mg Compound **1.** In embodiments, the dose (*e.g*., a daily dose) is 450 mg Compound 1. In embodiments, the dose (*e.g*., a daily dose) is 600 mg Compound **1.**

In embodiments, the patient has an initial hemoglobin level of ≤ 11 g/dL. In embodiments, the method comprises administering to a patient a daily dose of Compound **1** that is at least 300 mg (*e.g.,* 300 mg Compound **1,** 450 mg Compound **1,** or 600 mg Compound **1)** at the time of commencing treatment with Compound **1** or within 4, 6, 8, 10, or 12 weeks from the time of commencing treatment with Compound **1** as described herein. In some examples, the method comprises administering to a patient a daily dose of Compound 1 that is at least about 300 mg (*e.g.,* about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the daily dose is 300 mg Compound **1.** In embodiments, the daily dose is 450 mg Compound **1.** In embodiments, the daily dose is 600 mg Compound **1.**

In embodiments, the method comprises administering a dose of Compound **1** to the patient, wherein the daily dose is selected based on a previous ESA therapy received by the patient. In embodiments, the method comprises administering a dose of Compound **1** to the patient, wherein the dose is selected based on the dose of a previous ESA therapy received by the patient. In embodiments, the patient is administered Compound **1** once daily. In embodiments, the patient is administered Compound **1** once weekly. In embodiments, the patient is administered Compound **1** three times per week.

In embodiments, the method comprises administering a daily dose of Compound **1** to the patient, wherein the daily dose is selected based on a previous ESA therapy received by the patient. In embodiments, the method comprises administering a daily dose of Compound **1** to the patient, wherein the daily dose is selected based on the dose of a previous ESA therapy received by the patient.

In some examples, the method comprises selecting a patient based on a previous ESA therapy received by the patient and administering to the patient a dose of Compound **1** based on the previous ESA therapy received by the patient. In some examples, the method comprises selecting a patient based on a previous ESA therapy received by the patient and administering to the patient a dose of Compound **1** based on the previous dose of ESA therapy received by the patient. In embodiments, the patient is administered Compound **1** about once daily. In embodiments, the patient is administered Compound **1** about once weekly. In embodiments, the patient is administered Compound **1** about three times per week.

In some examples, the method comprises selecting a patient based on a previous ESA therapy received by the patient and administering to the patient a daily dose of Compound **1** based on the previous ESA therapy received by the patient. In some examples, the method comprises selecting a patient based on a previous ESA therapy received by the patient and administering to the patient a daily dose of Compound **1** based on the previous dose of ESA therapy received by the patient.

In embodiments, the patient has previously received epoetin alfa (*e.g*., epoetin alfa in an amount ≤ 12,000 IU every two weeks for NDD-CKD patients or ≤ 9,000 IU weekly for DD-CKD patients), epoetin beta (*e.g*., epoetin beta in an amount ≤ 12,000 IU every two weeks for NDD-CKD patients or ≤ 9,000 IU weekly for DD-CKD patients), epoetin beta pegol (*e.g*., epoetin beta pegol in an amount ≤ 250 µg every four weeks for NDD-CKD patients or DD-CKD patients), or darbepoetin alfa (DA) (*e.g*., darbepoetin alfa in an amount ≤ 120 µg every two weeks for NDD-CKD patients or ≤ 60 µg weekly for DD-CKD patients).

In embodiments, the patient has previously received darbepoetin alfa (DA). In embodiments, the patient has previously received DA at a dose level of ≥ 15 µg (*e.g*., weekly). In embodiments, the method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, the method comprises administering to a patient a dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, the patient has DD-CKD. In embodiments, the patient receives a dose about once daily (a daily dose). In embodiments, the patient receives a dose about once weekly. In embodiments, the patient receives a dose about three times per week. In embodiments, the dose (*e.g.,* a daily dose) is 300 mg Compound **1.** In embodiments, the dose (*e.g.,* a daily dose) is 450 mg Compound **1.** In embodiments, the dose (*e.g*., a daily dose) is 600 mg Compound **1.**

In embodiments, the patient has previously received darbepoetin alfa (DA). In embodiments, the patient has previously received DA at a dose level of ≥ 15 µg (*e.g*., weekly). In embodiments, the method comprises administering to a patient a daily dose of Compound **1** that is at least 300 mg (*e.g.,* 300 mg Compound **1,** 450 mg Compound **1,** or 600 mg Compound **1)** at the time of commencing treatment with Compound **1 or** within about 4, 6, 8, 10, or 12 weeks from the time of commencing treatment with Compound **1** as described herein. In some examples, the method comprises administering to a patient a daily dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, the patient has DD-CKD. In embodiments, the daily dose is 300 mg Compound **1.** In embodiments, the daily dose is 450 mg Compound **1.** In embodiments, the daily dose is 600 mg Compound **1.**

In embodiments, the patient has previously received epoetin beta pegol. In embodiments, a patient has previously received epoetin beta pegol at a dose level of ≥ 15 µg (*e.g*., weekly). In embodiments, the method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, the method comprises administering to a patient a dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, the patient receives treatment with Compound **1** for at least 54, 56, 58, or 60 weeks; or at least 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, a patient has DD-CKD. In embodiments, the patient receives a dose about once daily (a daily dose). In embodiments, the patient receives a dose once weekly. In embodiments, the patient receives a dose three times per week. In embodiments, the dose (*e.g*., a daily dose) is 300 mg Compound **1.** In embodiments, the dose (*e.g*., a daily dose) is 450 mg Compound **1.** In embodiments, the dose (*e.g*., a daily dose) is 600 mg Compound **1.**

In embodiments, a patient has previously received epoetin beta pegol. In embodiments, a patient has previously received epoetin beta pegol at a dose level of ≥ 15 µg (*e.g*., weekly). In embodiments, a method comprises administering to a patient a daily dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, a method comprises administering to a patient a daily dose of Compound **1** that is at least about 300 mg (*e.g.,* about 300 mg Compound **1,** about 450 mg Compound **1,** or about 600 mg Compound **1)** for at least about 6, 8, 10, 12, 14, 16, 18, or 20 consecutive weeks as described herein. In embodiments, a patient receives treatment with Compound **1** for at least 54, 56, 58, or 60 weeks; or at least 13, 14, 15, 16, 17, 18, 19, 20, 21, 22, 23, or 24 months as described herein. In some examples, a patient receives therapy with Compound **1** for at least about twenty-four weeks (about six-months) or at least about fifty-two weeks (about 12-months) as described herein. In embodiments, a patient has NDD-CKD. In embodiments, a patient has DD-CKD. In embodiments, a daily dose is 300 mg Compound **1.** In embodiments, a daily dose is 450 mg Compound **1.** In embodiments, a daily dose is 600 mg Compound **1.**

In embodiments, a patient has previously received epoetin (*e.g*., epoetin alfa or epoetin beta). In embodiments, a patient has previously received epoetin (*e.g*., epoetin alfa or epoetin beta) at a dose level of ≥ 4500 IU (*e.g*., weekly). In embodiments, a method comprises administering to a patient a dose of Compound **1** that is at least 300 mg (*e.g.,* 300 mg Compound **1,** 450 mg Compound **1,** or 600 mg Compound **1)** at the time of commencing treatment with Compound **1** or within about 4, 6, 8, 10, or 12 weeks from the time of commencing treatment with Compound **1** as described herein. In some examples, a method comprises administering to a patient a daily dose of Compound **1** that is at least about 300 mg (*e.g*., about 300 mg Compound **1,** about 450 mg Compound **1,** or about 600 mg Compound **1)** for at least about 6, 8, 10, 12, 14, 16, 18, or 20 consecutive weeks as described herein. In embodiments, a patient receives treatment with Compound **1** for at least 54, 56, 58, or 60 weeks; or at least 13, 14, 15, 16, 17, 18, 19, 20, 21, 22, 23, or 24 months as described herein. In some examples, a patient receives therapy with Compound **1** for at least about twenty-four weeks (about six-months) or at least about fifty-two weeks (about 12-months) as described herein. In embodiments, a patient has NDD-CKD. In embodiments, a patient has DD-CKD. In embodiments, a patient receives a dose about once daily (a daily dose). In embodiments, a patient receives a dose about once weekly. In embodiments, a patient receives a dose about three times per week. In embodiments, a dose (*e.g*., a daily dose) is 300 mg Compound **1.** In embodiments, a dose (*e.g.,* a daily dose) is 450 mg Compound **1.** In embodiments, a dose (*e.g.,* a daily dose) is 600 mg Compound **1.**

In embodiments, the patient has previously received epoetin (*e.g*., epoetin alfa or epoetin beta). In embodiments, a patient has previously received epoetin (*e.g*., epoetin alfa or epoetin beta) at a dose level of ≥ 4500 IU (*e.g*., weekly). In embodiments, the method comprises administering to a patient a daily dose of Compound **1** that is at least 450 mg (*e.g.,* 450 mg or 600 mg) at the time of commencing treatment with Compound **1** or within about 4, 6, 8, 10, or 12 weeks from the time of commencing treatment with Compound **1** as provided herein. In some examples, the method comprises administering to a patient a daily dose of Compound **1** that is at least about 450 mg (*e.g.,* about 450 mg or about 600 mg) for at least about 6, 8, 10, 12, 14, 16, 18, or 20 consecutive weeks as described herein. In embodiments, the patient receives treatment with Compound **1** for at least 54, 56, 58, or 60 weeks; or at 13, 14, 15, 16, 17, 18, 19, 20, 21, 22, 23, or 24 months as described herein. In some examples, the patient receives therapy with Compound **1** for at least about twenty-four weeks (about six-months) or at least about fifty-two weeks (about 12-months) as described herein. In embodiments, the patient has NDD-CKD. In embodiments, the patient has DD-CKD. In embodiments, the daily dose is 300 mg Compound **1.** In embodiments, the daily dose is 450 mg Compound **1.** In embodiments, the daily dose is 600 mg Compound **1.**

In certain embodiments, the patient does not have endogenous EPO circadian circulation expression patterns. In certain embodiments, the Compound **1** is administered to mimic the normal and endogenous circadian pattern of the EPO (*i.e.,* of a healthy person), such that the peak of the EPO expression occurs between 6 p.m. and midnight. In certain embodiments, the compound is administered at a time such that the EPO peak is earlier than the cortisol peak, specifically, such that the EPO peak precedes the cortisol peak by 1 hour, by 2 hours, by 3 hours, by 4 hours, by 5 hours, by 6 hours, by 7 hours, or by 8 hours. In certain embodiments, the cortisol peak is in the morning. In certain embodiments, the compound is administered at 8 a.m., 9 a.m., 10 a.m., 11 a.m., 12 p.m., 1 p.m., or 2 p.m. In certain embodiments, Compound **1** is administered after breakfast. In certain embodiments, compound **1** is administered between breakfast and 8 a.m., 9 a.m., 10 a.m., 11 a.m., 12 p.m., 1 p.m., or 2 p.m. In certain embodiments, compound **1** is administered before lunch. In certain embodiments, Compound **1** is administered between breakfast and lunch. In certain embodiments Compound **1** is administered after lunch. In certain embodiments, Compound **1** is administered between lunch and 2 p.m. In certain embodiments, Compound **1** is administered every day at the same time.

### Safety Assessment

Methods described herein can also avoid or reduce adverse events and/or adverse drug reactions, including those typically associated with ESA therapy. For example, methods described herein can avoid or reduce adverse events related to cardiovascular events, retinal disorders, and/or malignancy.

In embodiments, the methods described herein avoid or reduce the risk of thrombosis. In some such embodiments, the thrombosis is thromboembolism. The term "thromboembolism" refers to the formation in a blood vessel of a clot (thrombus) that breaks loose and is carried by the blood stream to plug another vessel. The clot may plug a vessel in the lungs, brain, gastrointestinal tract, kidneys, or leg. Thromboembolism can be fatal. Such thromboembolic conditions include, but are not limited to, cerebral infarctions, myocardial infarctions, and pulmonary embolisms. Accordingly, the methods described herein can avoid or reduce adverse events related to thromboembolism such as cerebral infarction, myocardial infarction, and/or pulmonary embolism.

### Iron-Related Parameters and Red Blood Cell Indices

Methods described herein can also achieve favorable results in iron-related parameters (*e.g*., serum ferritin, transferrin saturation (TSAT), total iron-binding capacity (TIBC), hepcidin, serum iron, and/or monthly dose of iron by any route of administration) and/or red blood cell indices (*e.g*., mean corpuscular volume (MCV), mean corpuscular hemoglobin (MCH), mean corpuscular hemoglobin concentration (MCHC), and/or red cell distribution width (RDW)). For example, methods described herein can achieve favorable results in these parameters and indices as compared to other methods of treating anemia, including ESA therapy.

### Total iron binding capacity (TIBC)

Total iron binding capacity (TIBC) is a measure of the blood's capacity to bind iron with transferrin and is performed by drawing blood and measuring the maximum amount of iron that the blood can carry. Accordingly, the TIBC is representative of the amount of circulating transferrin, which contains two binding sites for transporting iron from iron storage sites to erythroid progenitor cells.

Phase 2a clinical trials showed that, in stage 3, 4, or 5 CKD patients, Compound **1** was able to increase total iron binding capacity (TIBC) levels, at 6 weeks post administration as compared to placebo treated patients. Unexpectedly, the increase in TIBC levels was not associated with an increase in serum iron levels. Further, it was also discovered that Compound **1** resulted in a dose-related increase in TIBC and a decrease in transferrin saturation (TSAT), suggesting administration of Compound **1** results in enhanced iron mobilization.

In some examples, a patient has an increase in total iron binding capacity (TIBC) relative to a baseline level. In some examples, methods described herein raise the TIBC relative to a baseline TIBC in a patient, without significantly increasing the serum iron level relative to a baseline.

In certain examples, the TIBC increases by about 10 µg/dL, about 20 µg/dL, about 30 µg/dL, about 40 µg/dL, about 50 µg/dL about 60 µg/dL, about 70 µg/dL, about 80 µg/dL, about 90 µg/dL or about 100 µg/dL relative to a baseline TIBC. In certain examples, the TIBC increases by at least about 10 µg/dL, at least about 20 µg/dL, at least about 30 µg/dL, at least about 40 µg/dL, at least about 50 µg/dL, at least about 60 µg/dL, at least about 70 µg/dL, at least about 80 µg/dL, at least about 90 µg/dL or at least about 100 µg/dL. In certain examples, the TIBC increases by between about 10 µg/dL and about 60 µg/dL, between about 10 µg/dL and about 50 µg/dL, between about 10 µg/dL and about 40 µg/dL, between about 10 µg/dL and about 30 µg/dL, or between about 10 µg/dL and about 20 µg/dL. In certain examples, the TIBC increases by between 20 µg/dL and about 60 µg/dL, between about 30 µg/dL and about 60 µg/dL, between 40 µg/dL and about 60 µg/dL, or between about 50 µg/dL and about 60 µg/dL.

In certain such examples, the TIBC increase occurs over about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, or about 24 weeks relative to a baseline TIBC.

In certain examples administration of Compound **1** is suitable to increase the total iron binding capacity in the patient by at least 5%, 10%, 15%, 20%, 25%, 30%, 35%, 40%, 45%, or at least 50%. In more specific examples, the pharmaceutically effective amount is suitable to increase the total iron binding capacity in the patient by at least 5%, 10%, 15%, 20%, 25%, 30%, 35%, 40%, 45%, or at least 50% while the total serum iron levels are not increased, or are increased by at most 1%, 2%, 3%, 4%, 5%, 6%, 7%, 8%, 9%, 10%, 15%, 20%, or at most 25%.

In certain examples, unsaturated iron binding capacity (UIBC) may be determined by adding serum to an alkaline buffer/reductant solution containing a known concentration of iron to saturate the available binding sites on transferrin. The Ferrozine chromogen reacts only with the Fe²⁺; therefore, an iron reductant is added to ensure that all iron is present in the ferrous state. The excess unbound divalent iron reacts with Ferrozine chromogen to form a magenta complex, which is measured spectrophotometrically. The unsaturated iron binding capacity (UIBC) is equal to the difference measured in the concentrations of the added iron solution and the excess unbound iron. Serum TIBC is equal to total serum iron plus UIBC and may therefore be calculated using the results of the UIBC and serum iron determinations.

### Serum Iron

In certain examples, serum iron may be determined using a test based on the FerroZine method without deproteinization. Specimens are analyzed on the Roche Modular Instrument utilizing Roche Diagnostics Reagents. Under acidic conditions, iron is liberated from transferrin. The detergent clarifies lipemic samples. Ascorbate reduces the released Fe3+ ions to Fe2+ ions, which then react with FerroZine to form a colored complex. The color intensity is directly proportional to the iron concentration and can be measured photometrically.

Serum iron level measurements determine how much iron is in the plasma. The amount of iron that is found in serum is dependent on the ability to mobilize the iron that is stored in cells. This process of iron mobilization is controlled by ferroportin and hepcidin which work in concert to regulate the amount of iron that is exported to the plasma. Ferroportin moves iron in and out of cells, while hepcidin regulates the action of ferroportin, thereby determining whether iron is released into the plasma or retained in the cell. Accordingly, it is possible to have large amounts of iron stored in cells, but relatively low levels of serum iron depending on the activity of ferroportin and hepcidin.

In certain examples, the serum iron level increases by less than about 20 µg/dL, less than about 15 µg/dL, less than about 10 µg/dL, or less than about 5 µg/dL relative to a baseline serum iron level. In certain examples, the serum iron level increases by between about 0 µg/dL and about 20 µg/dL, between about 0 µg/dL and about 15 µg/dL, between about 0 µg/dL and about 10 µg/dL, or between about 0 µg/dL and about 5 µg/dL.

### Hepcidin Levels

In examples, a patient has a decrease in hepcidin level relative to a baseline level.

In certain examples, hepcidin level decreases less than about 20%, less than about 15%, less than about 10%, less than about 5%, less than about 4%, less than about 3%, less than about 2%, or less than about 1% relative to the baseline hepcidin level. In certain examples, hepcidin level decreases by between about 0% and about 20%, between about 0% and about 15%, between about 0% and about 10%, or between about 0% and about 5%, between about 0% and about 4%, between about 0% and about 3%, between about 0% and about 2%, or between about 0% and about 1% relative to the baseline hepcidin expression level. In certain embodiments, hepcidin level decreases by about 20%, about 15%, about 10%, about 5%, about 4%, about 3%, about 2%, or about 1% relative to the baseline hepcidin level.

In examples, methods described herein can increase the peak levels of serum EPO during the circadian cycle by at least 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, or at least 150% relative to the trough levels of serum EPO without decreasing the serum levels of hepcidin by more than 1%, 2%, 3%, 4%, 5%, 6%, 7%, 8%, 9%, 10%, 11%, 12%, 13%, 14%, 15%, 16%, 17%, 18%, 19%, or by more than 20% relative to hepcidin levels prior to administration of Compound **1.**

In some examples, methods described herein can be suitable to increase the peak levels of hemoglobin levels by at least 2%, 3%, 4%, 5%, 6%, 7%, 8%, 9%, 10%, 11%, 12%, 13%, 14%, 15%, 16%, 17%, 18%, or at least 20%, relative to hepcidin levels prior to the treatment without decreasing the serum levels of hepcidin by more than 1%, 2%, 3%, 4%, 5%, 6%, 7%, 8%, 9%, 10%, 11%, 12%, 13%, 14%, 15%, 16%, 17%, 18%, 19%, or by more than 20% relative to hepcidin levels prior to administration of Compound **1.**

In certain examples, hepcidin level may be determined, as described in Ganz, T. et al., "Immunoassay for human serum hepcidin" Blood 112: 4292-97 (2008). Briefly, the antibody to human hepcidin was purified on staphylococcal protein A columns according to the manufacturer's protocol; 96-well plates were coated with the antibody and incubated with 100 µL (standard samples) or 200 µL (samples with very low concentration of hepcidin) of 1:20 dilution of serum or 1:10 dilution of urine in Tris-buffered saline containing 0.05% Tween-20 (TBS-Tween 20), with 10 ng/mL of biotinylated hepcidin-25 added as the tracer. Standard curves were prepared by serial 2-fold dilution of synthetic hepcidin 4000 ng/mL in TBS-Tween 20 buffer containing the tracer. The integrity and bioactivity of synthetic hepcidin and biotinylated hepcidin were verified by mass spectrometry and by bioassay with ferroportin-green fluorescent protein expressing HEK-293 cells. After washing, the assay was developed with streptavidin-peroxidase and tetramethyl benzidine. The enzymatic reaction was stopped by sulfuric acid, and the plate was read at 450 nm on a DTX 880 microplate reader. Standard curves were fitted with 12-point fit using GraphPad Prism software. The fitted curve was then used to convert sample absorbance readings to hepcidin concentrations.

### Serum Hemoglobin

In certain embodiments, hemoglobin values are adjusted for altitude, gender, and age of the patient.

In embodiments, a patient has a baseline hemoglobin level of <10 g/dL. In embodiments, a patient has a baseline hemoglobin level of ≤9 g/dL. In embodiments, a patient has a baseline hemoglobin level of ≤8 g/dL.

In embodiments, methods described herein maintain the level of hemoglobin within a target range.

In embodiments, a target range is 11.0-13.0 g/dL (e.g., for NDD-CKD patients). In embodiments, a target range is 10.0-12.0 g/dL (e.g., for DD-CKD patients).

In some examples, the hemoglobin levels of the patient are maintained or controlled at a level of 8.0 g/dL and at or below about 13.0 g/dL, at least about 8.5 g/dL and at or below 13.0 g/dL, at least about 9.0 g/dL and at or below 13.0 g/dL, or at least about 9.5 g/dL and at or below 13.0 g/dL. In certain such embodiments, hemoglobin levels are maintained or controlled at a level of at least 11.0 g/dL and at or below 13.0 g/dL. In certain such embodiments, hemoglobin levels are maintained or controlled at a level of at least 11.0 g/dL and at or below 12.0 g/dL. In embodiments, hemoglobin levels are maintained or controlled at 10.0 - 13.0 g/dL. In embodiments, hemoglobin levels are maintained or controlled at 10.0 - 12.0 g/dL. In embodiments, hemoglobin levels are maintained or controlled at 11.0 - 13.0 g/dL. In embodiments, hemoglobin levels are maintained or controlled at 11.0 - 12.0 g/dL.

In embodiments, a patient has a hemoglobin level of 8.0 g/dL to 13.0 g/dL. In embodiments, a patient has a hemoglobin level of 8.0 g/dL to 12.0 g/dL. In embodiments, a patient has a hemoglobin level of 8.0 g/dL to 11.0 g/dL. In embodiments, a patient has a hemoglobin level of 9.0 g/dL to 12.0 g/dL. In embodiments, a patient has a hemoglobin level of 9.5 g/dL to 12.0 g/dL. In embodiments, a patient has a hemoglobin level of 9.0 g/dL to 12.5 g/dL. In embodiments, a patient has a hemoglobin level of <11.0 g/dL. In embodiments, a patient has a hemoglobin level of >11.5 g/dL. In embodiments, a patient has a hemoglobin level of ≥9.5 g/dL to <11.0 g/dL. In embodiments, a patient has a hemoglobin level of ≥8.0 g/dL to <11.0 g/dL. In embodiments, a patient has a hemoglobin level of ≥12.0 g/dL. In embodiments, a patient has a hemoglobin level of ≥13.0 g/dL.

In embodiments, methods describe herein increase the level of hemoglobin by at least 0.2 g/dL, by at least 0.3 g/dL, by at least 0.4 g/dL, by at least 0.5 g/dL, by at least 0.6 g/dL, by at least 0.7 g/dL, by at least 0.8 g/dL, by at least 0.9 g/dL, by at least 1.0 g/dL, by at least 1.2 g/dL, or by at least 1.5 g/dL relative to a baseline hemoglobin level in the patient.

In embodiments, hemoglobin levels are increased to 10.0-12.0 g/dL. In embodiments, hemoglobin levels are increased to 10.0-11.0 g/dL. In embodiments, hemoglobin levels are increased to 11.0-13.0 g/dL.

In certain embodiments, the level of serum hemoglobin is raised by between 0.1 and 1.0 g/dL, between 0.1 and 0.9 g/dL, 0.1 and 0.8 g/dL, 0.1 and 0.7 g/dL, 0.1 and 0.6 g/dL, or 0.1 and 0.5 g/dL over a period of time, such as one week, two weeks, three weeks, four weeks, five weeks, or six weeks relative to the baseline hemoglobin level. In certain embodiments, the level of hemoglobin is raised by at least 0.1 g/dL, 0.2 g/dL, 0.3 g/dL, 0.4 g/dL, 0.5 g/dL, 0.6 g/dL, 0.7 g/dL, 0.8 g/dL, 0.9, or 1.0 g/dL over a period of time, such as one week, two weeks, three weeks, four weeks, five weeks, or six weeks relative to the baseline hemoglobin level.

In certain embodiments, the level of hemoglobin is raised by 0.1 g/dL over a period of one week relative to the baseline hemoglobin level. In certain embodiments, the level of hemoglobin is raised by 0.1 g/dL over a period of two weeks relative to the baseline hemoglobin level. In certain embodiments, the level of hemoglobin is raised by 0.5 g/dL over a period of three weeks relative to the baseline hemoglobin level. In certain embodiments, the level of hemoglobin is raised by 0.6 g/dL over a period of four weeks relative to the baseline hemoglobin level. In certain embodiments, the level of hemoglobin is raised by 0.6 g/dL over a period of five weeks relative to the baseline hemoglobin level. In certain embodiments, the level of hemoglobin is raised by 0.6 g/dL over a period of six weeks relative to the baseline hemoglobin level.

Serum hemoglobin levels may be determined, for example using standard approach CBC where red cells are lysed and potassium ferricyanide oxidizes hemoglobin to methemoglobin, which combines with potassium cyanide forming cyanmethemoglobin. The brown color is measured spectrophotometrically and the corresponding hemoglobin reported.

### Serum Ferritin and Transferrin Saturation (TSAT)

In some examples, a patient has a decrease in serum ferritin level relative to a baseline level.

In some examples, a patient has a ferritin level of at least 50 ng/mL or even at least 100 ng/mL. In embodiments, serum ferritin is maintained at a level of between 50 ng/mL and 300 ng/mL. In some examples, a patient has a serum ferritin level of ≥ 100 ng/mL.

In some examples, transferrin saturation (TSAT) decreases relative to a baseline TSAT. In some examples, a patient has a transferrin saturation (TSAT) of at least about 15%, at least about 18%, or even at least about 20%. In some examples, a patient has a serum ferritin level of about ≥ 100 ng/mL and/or a transferrin saturation (TSAT) of ≥ 20%.

In some examples, a patient has a serum ferritin level of about ≥ 100 ng/mL and/or a transferrin saturation (TSAT) of ≥ 20%. In some examples, a patient has a serum ferritin level of about ≥ 100 ng/mL and a transferrin saturation (TSAT) of about ≥ 20%.

### Patient Populations

The dose and dosage regimens described herein may be used in methods for treating anemia secondary to CKD, comprising administering to a patient having anemia an effective amount of Compound **1,** or a pharmaceutically acceptable salt thereof.

As described herein, methods can be useful for the treatment of patients of having various dialysis statuses (*e.g*., a dialysis status as described herein).

In certain embodiments, the patient has non-dialysis-dependent chronic kidney disease (NDD-CKD). The NDD-CKD patients include those CKD patients who do not yet require the life-supporting treatments for kidney failure, such as kidney replacement therapy (RRT, including maintenance dialysis or kidney transplantation).

In other embodiments, the patient is a dialysis dependent CKD patient (DD-CKD). DD-CKD patients are in end-stage kidney disease. ESKD is typically the irreversible conclusion of the NDD-CKD. Even though the NDD-CKD status refers to the status of persons with earlier stages of CKD (stages 1 to 4), people with advanced stage of CKD (stage 5), who have not yet started kidney replacement therapy, are also referred to as NDD-CKD. Therefore, in some embodiments, the NDD-CKD patient will need or begin dialysis during the administration of Compound **1.** In embodiments, dialysis is hemodialysis (HD). In embodiments, dialysis is peritoneal dialysis (PD).

In some embodiments, the patient had been previously treated with an ESA, such as an erythropoietin mimetic. In certain embodiments the ESA is an rhEPO product, including, but not limited to, epoetin alfa, epoetin beta, darbepoetin, or peginesatide. (*e.g*., epoetin alfa, epoetin beta, darbepoetin, or peginesatide). In one example, at least eight (8) weeks prior to being administered Compound 1, the patient discontinued use of the ESA. In certain embodiments, the patient is refractory or resistant to treatment with an ESA.

In some examples, the patient has a ferritin level of at least about 100 ng/mL. In other examples, the patient has a transferritin saturation of at least about 20%.

In some embodiments, the patient has a body mass index (BMI) of less than about 30 kg/m².

In embodiments, a patient is an adult. In embodiments, a patient is ≥ 18 years old. In embodiments, a patient is ≥ 20 years old. In certain embodiments, the patient is between the ages of 20 years old and 90 years old, for example between the ages of 25 years old and 90 years old, between the ages of 30 years old and 90 years old, between the ages of 35 years old and 90 years old, between the ages of 40 years old and 90 years old, between the ages of 45 years old and 90 years old, between the ages of 50 years old and 90 years old, between the ages of 55 years old and 90 years old, between the ages of 60 years old and 90 years old, between the ages of 60 years old and 85 years old, or between the ages of 60 years old and 80 years old. In certain embodiments, the patient is between the ages of 60 years old and 80 years old. In certain embodiments, the patient is 70 years old.

In some embodiments, the patient is at least 20 years old, at least 25 years old, at least 30 years old, at least 35 years old, at least 40 years old, at least 45 years old, at least 50 years old, at least 55 years old, at least 60 years old, at least 65 years old, at least 70 years old, at least 75 years old, at least 80 years old, at least 85 years old, or at least 90 years old. In some embodiments, the patient is 20 years old, 21 years old, 22 years old, 23 years old, 24 years old, 25 years old, 26 years old, 27 years old, 28 years old, 29 years old, 30 years old, 31 years old, 32 years old, 33 years old, 34 years old, 35 years old, 36 years old, 37 years old, 38 years old, 39 years old, 40 years old, 41 years old, 42 years old, about 43 years old, about 44 years old, about 45 years old, about 46 years old, 47 years old, 48 years old, 49 years old, 50 years old, 51 years old, 52 years old, 53 years old, 54 years old, 55 years old, 56 years old, 57 years old, 58 years old, 59 years old, 60 years old, 61 years old, 62 years old, 63 years old, 64 years old, 65 years old, 66 years old, 67 years old, 68 years old, 69 years old, 70 years old, 71 years old, 72 years old, 73 years old, 74 years old, 75 years old, 76 years old, 77 years old, 78 years old, 79 years old, 80 years old, 81 years old, 82 years old, 83 years old, 84 years old, 85 years old, 86 years old, 87 years old, 88 years old, 89 years old, or 90 years old.

The patient may be a member of any racial or ethnic subpopulation, including White, Black, Hispanic, and Asian. The patient may also be male or female.

### Glomerular Filtration Rate

A glomerular filtration rate (GFR) ≥ 60 ml/min/1.73 m² is considered normal without chronic kidney disease if there is no kidney damage present.

Kidney damage is defined signs of damage seen in blood, urine, or imaging studies which includes lab albumin/creatinine ratio (ACR) ≥ 30. All people with a GFR <60 ml/min/1.73 m² for 3 months are defined as having chronic kidney disease.

Protein in the urine is regarded as an independent marker for worsening of kidney function and cardiovascular disease.

There are five (5) stages for CKD as shown in **Table 1.**

**Table 1. Chronic Kidney Disease (CKD) Staging**

| **CKD G1-5 A1-3 glomerular filtration rate (GFR) and albumin/creatinine ratio (ACR)** | | | | | |
|---|---|---|---|---|---|
| | | | **ACR** | | |
| | | | A1 | A2 | A3 |
| | | | Normal to mildly increased | Moderately increased | Severely increased |
| | | | <30 | 30-300 | >300 |
| G1 | Normal | 90+ | 1 if kidney damage present | 1 | 2 |
| G2 | Mildly decreased | 60-89 | 1 if kidney damage present | 1 | 2 |
| G3a | Mildly to moderately decreased | 45-59 | 1 | 2 | 3 |
| G3b | Moderately to severely decreased | 30-44 | 2 | 3 | 3 |
| G4 | Severely decreased | 15-29 | 3 | 4+ | 4+ |
| G5 | Kidney failure | <15 | 4+ | 4+ | 4+ |
| Numbers 1-4 indicates risk of progression as well as frequency of monitoring (number of times a year). | | | | | |
| Kidney Disease Improving Global Outcomes - KDIGO 2012 Clinical Practice Guideline for the Evaluation and Management of Chronic Kidney Disease | | | | | |

Stage 1: Slightly diminished function; kidney damage with normal or relatively high GFR (≥90 mL/min/1.73 m²) and persistent albuminuria. Kidney damage is defined as pathological abnormalities or markers of damage, including abnormalities in blood or urine tests or imaging studies.

Stage 2: Mild reduction in GFR (60-89 mL/min/1.73 m²) with kidney damage. Kidney damage is defined as pathological abnormalities or markers of damage, including abnormalities in blood or urine tests or imaging studies.

Stage 3: Moderate reduction in GFR (30-59 mL/min/1.73 m²). British guidelines distinguish between stage 3A (GFR 45-59) and stage 3B (GFR 30-44) for purposes of screening and referral.

Stage 4: Severe reduction in GFR (15-29 mL/min/1.73 m²). Preparation for kidney replacement therapy.

Stage 5: Established kidney failure (GFR <15 mL/min/1.73 m²), permanent kidney replacement therapy, or end-stage kidney disease.

GFR can be estimated based on a serum creatinine level. Creatinine is a muscle waste product that is filtered from the blood by the kidneys and released into the urine at a relatively steady rate. When kidney function decreases, less creatinine is eliminated and concentrations increase in the blood. With the creatinine test, a reasonable estimate of the actual GFR can be determined. Different equations may be used to calculate eGFR. In some examples, eGFR is calculated based on Chronic Kidney Disease Epidemiology Collaboration (CKD-EPI) creatinine equation (2009). In other examples, eGFR is calculated by Equation 1 (Eq.1):$eGFR \left(mL/min/1.73 {m}^{2}\right) = 194 \times serum creatinine -1 . 094 \times age - 0.287 \left(females: \times 0.739\right)$ (Eq. 1)

In some examples, the patient has an eGFR of less than about 60 mL/min/1.73 m², less than about 45 mL/min/1.73 m², less than about 30 mL/min/1.73 m², less than about 15 mL/min/1.73 m², or less than about 15 mL/min/1.73 m².

In certain embodiments, the CKD is stage 1, 2, 3, 4, or 5 chronic kidney disease. In certain such embodiments, the CKD is stage 3, 4, or 5 chronic kidney disease. In certain embodiments, the CKD is stage 1 chronic kidney disease. In certain embodiments, the CKD is stage 2 chronic kidney disease. In certain embodiments, the CKD is stage 3 chronic kidney disease. In certain embodiments, the CKD is stage 4 chronic kidney disease. In certain embodiments, the CKD is stage 5 chronic kidney disease. In certain embodiments, the chronic kidney disease is pre-dialysis chronic kidney disease. In certain embodiments, the patient is a dialysis patient and these patients may be referred to as having end stage renal disease (ESRD).

### Doses and Dosing Regimens

The specific doses for uses of a Compound **1** can be administered in any manner known to the skilled artisan. Exemplary doses are provided herein, including in the Examples.

Exemplary doses of Compound **1** include doses of 150-600 mg of Compound **1.** In embodiments, a dose of Compound **1** is 150 mg. In embodiments, a dose of Compound **1** is 300 mg. In embodiments, a dose of Compound **1** is 450 mg. In embodiments, a dose of Compound **1** is 600 mg.

In embodiments, any exemplary dose described herein (*e.g*., a dose of Compound **1** that is 150 mg, 300 mg, 450 mg, or 600 mg) is administered to a patient about once daily. In embodiments, a dose is 150 mg Compound **1** administered once daily. In embodiments, a dose is 300 mg Compound **1** administered once daily. In embodiments, a dose is 450 mg Compound 1 administered once daily. In embodiments, a dose is 600 mg Compound **1** administered once daily.

In embodiments, any exemplary dose described herein (*e.g*., a dose of Compound **1** that is 150 mg, 300 mg, 450 mg, or 600 mg) is administered to a patient about once weekly. In embodiments, a dose is 150 mg Compound **1** administered once weekly. In embodiments, a dose is 300 mg Compound **1** administered once weekly. In embodiments, a dose is 450 mg Compound **1** administered once weekly. In embodiments, a dose is 600 mg Compound **1** administered once weekly.

In embodiments, any exemplary dose described herein (*e.g*., a dose of Compound **1** that is 150 mg, 300 mg, 450 mg, or 600 mg) is administered to a patient about three times per week. In embodiments, a dose is 150 mg Compound **1** administered three times per week. In embodiments, a dose is 300 mg Compound **1** administered three times per week. In embodiments, a dose is 450 mg Compound **1** administered three times per week. In embodiments, a dose is 600 mg Compound **1** administered three times per week.

A patient can receive any exemplary dose according to any exemplary dosing frequency for a certain number of consecutive weeks.

In some examples of any methods described herein, said patient receives a dose of Compound **1** (e.g., about 150 mg, about 300 mg, about 450 mg, or about 600 mg) for a period that is at least about 24, 28, 32, 36, 40, 44, 48, or 52 weeks (e.g., a period that is at least about 52 weeks). In one example, a dose of Compound **1** is administered to the patient for at least about 24 weeks. In one example, a dose of Compound **1** is administered to the patient for at least about 28 weeks. In one example, a dose of Compound **1** is administered to the patient for at least about 32 weeks. In one example, a dose of Compound **1** is administered to the patient for at least about 36 weeks. In one example, a dose of Compound **1** is administered to the patient for at least about 40 weeks. In one example, a dose of Compound **1** is administered to the patient for at least about 44 weeks. In one example, a dose of Compound **1** is administered to the patient for at least about 48 weeks. In one example, a dose of Compound **1** is administered to the patient for at least about 52 weeks. In embodiments, a dose is 150 mg (e.g., administered once daily, once a week, or three times per week). In embodiments, a dose is 300 mg (e.g., administered once daily, once a week, or three times per week). In embodiments, a dose is 450 mg (e.g., administered once daily, once a week, or three times per week). In embodiments, a dose is 600 mg (e.g., administered once daily, once a week, or three times per week).

In embodiments of any methods described herein, said patient receives a dose of Compound **1** (e.g., 150 mg, 300 mg, 450 mg, or 600 mg) for a period that is 53-260 weeks. In embodiments, a dose of Compound **1** is administered to the patient for at least 53 weeks. In embodiments, a dose of Compound **1** is administered to the patient for at least 64 weeks. In embodiments, a dose of Compound **1** is administered to the patient for at least 76 weeks. In embodiments, a dose of Compound **1** is administered to the patient for at least 88 weeks. In embodiments, a dose of Compound **1** is administered to the patient for at least 104 weeks. In embodiments, a dose of Compound **1** is administered to the patient for at least 116 weeks. In embodiments, a dose of Compound **1** is administered to the patient for at least 128 weeks. In embodiments, a dose of Compound **1** is administered to the patient for at least 140 weeks. In embodiments, a dose of Compound **1** is administered to the patient for at least 156 weeks. In embodiments, a dose of Compound **1** is administered to the patient for at least 168 weeks. In embodiments, a dose of Compound **1** is administered to the patient for at least 180 weeks. In embodiments, a dose of Compound **1** is administered to the patient for at least 192 weeks. In embodiments, a dose of Compound **1** is administered to the patient for at least 208 weeks. In embodiments, a dose of Compound **1** is administered to the patient for 260 weeks. In embodiments, a dose is 150 mg (e.g., administered once daily, once a week, or three times per week). In embodiments, a dose is 300 mg (e.g., administered once daily, once a week, or three times per week). In embodiments, a dose is 450 mg (e.g., administered once daily, once a week, or three times per week). In embodiments, a dose is 600 mg (e.g., administered once daily, once a week, or three times per week).

In embodiments of any methods described herein, said patient receives a dose of Compound 1 that is at least 300 mg for at least 53, 64, 76, 88, 104, 116, 128, 140, 156, 168, 180, 192, 208, or 260 weeks. In embodiments of any methods described herein, said patient receives a dose of Compound **1** that is at least 300 mg for 53-260 weeks. In embodiments of any methods described herein, said patient receives a dose of Compound 1 that is at least 300 mg for 53, 64, 76, 88, 104, 116, 128, 140, 156, 168, 180, 192, 208, or 260 weeks. In some examples of any methods described herein, said patient receives a dose of Compound **1** that is at least about 300 mg for at least about 6-52, 6-48, 6-42, 6-36, 6-30, 6-24, 6-18, or 6-12 consecutive weeks. In some examples, said patient receives a dose of Compound **1** that is at least about 300 mg for at least about 8, 10, 12, 14, 16, 18, 20, 22, or 24 consecutive weeks. In some examples, said patient receives a dose of Compound **1** that is at least about 300 mg for at least about 26, 28, 30, 32, 34, 36, 38, 40, 42, 44, 46, 48, 50, or 52 consecutive weeks. In one example, said patient receives a dose of Compound **1** that is at least about 300 mg for at least about 8 consecutive weeks. In another example, said patient receives a dose of Compound **1** that is at least about 300 mg for at least about 12 consecutive weeks. In another example, said patient receives a dose of Compound **1** that is at least about 300 mg for at least about 24 consecutive weeks. In one example, said patient receives a dose of Compound **1** that is at least about 300 mg for at least about 36 consecutive weeks. In another example, said patient receives a dose of Compound **1** that is at least about 300 mg for at least about 52 consecutive weeks. In embodiments, said patient receives a dose of Compound **1** that is at least 300 mg for at least 53 consecutive weeks. In embodiments, said patient receives a dose of Compound **1** that is at least 300 mg for at least 64 consecutive weeks. In embodiments, said patient receives a dose of Compound **1** that is at least 300 mg for at least 76 consecutive weeks. In embodiments, said patient receives a dose of Compound **1** that is at least 300 mg for at least 88 consecutive weeks. In embodiments, said patient receives a dose of Compound **1** that is at least 300 mg for at least 104 consecutive weeks. In embodiments, said patient receives a dose of Compound **1** that is at least 300 mg for at least 116 consecutive weeks. In embodiments, said patient receives a dose of Compound **1** that is at least 300 mg for at least 128 consecutive weeks. In embodiments, said patient receives a dose of Compound **1** that is at least 300 mg for at least 140 consecutive weeks. In embodiments, said patient receives a dose of Compound **1** that is at least 300 mg for at least 156 consecutive weeks. In embodiments, said patient receives a dose of Compound **1** that is at least 300 mg for at least 168 consecutive weeks. In embodiments, said patient receives a dose of Compound **1** that is at least 300 mg for at least 180 consecutive weeks. In embodiments, said patient receives a dose of Compound **1** that is at least 300 mg for at least 192 consecutive weeks. In embodiments, said patient receives a dose of Compound **1** that is at least 300 mg for at least 208 consecutive weeks. In embodiments, said patient receives a dose of Compound **1** that is at least 300 mg for 260 consecutive weeks. In embodiments, a dose is 300 mg of Compound **1.** In embodiments, a dose is 450 mg of Compound **1.** In embodiments, a dose is 600 mg of Compound **1.**

A patient can receive any exemplary dose according to any exemplary dosing frequency for a threshold treating period. In embodiments, a treating period comprises uninterrupted treatment with Compound **1.** In embodiments, a treating period comprises interruption (*e.g*., a patient does not receive Compound **1** for a period of 1-7 days, about 1 week, or about 2 weeks).

In embodiments of any methods described herein, the patient receives a dose of Compound **1** that is at least 300 mg up to any of weeks 1-12, 1-6, 24-32, 24-40, or 24-52 of a treating period. In embodiments of any methods described herein, the patient receives a dose of Compound **1** that is at least 300 mg up to week 24, week 28, or week 32 of treatment with Compound **1.** In embodiments of any methods described herein, the patient receives a dose of Compound **1** that is at least 300 mg up to week 46, week 48, week 50, or week 52 of treatment with Compound **1.** In embodiments, the dose is 300 mg of Compound **1.** In embodiments, the dose is 450 mg of Compound **1.** In embodiments, the dose is 600 mg of Compound **1.**

In embodiments, a suitable dosing regimen is based on the identity of an erythropoietin stimulating agent (ESA) therapy, the dosage amount of the ESA, and/or dialysis (*e.g*., hemodialysis or peritoneal dialysis) previously received by a subject.

In embodiments, a suitable dosing regimen (*e.g*., as described herein) is selected based on the identity of an ESA therapy (*e.g.*, darbepoetin alfa) previously received by a subject.

In embodiments, a suitable dosing regimen (*e.g*., as described herein) is selected based on the dosage amount of an ESA therapy (*e.g*., a weekly dose amount of darbepoetin alfa) previously received by a subject.

In embodiments, a suitable dosing regimen (*e.g.*, as described herein) is selected based on the dialysis status of a subject (*e.g*., a patient who receives or previously receives dialysis such as hemodialysis or peritoneal dialysis). In embodiments, a patient has DD-CKD. In embodiments, a patient has NDD-CKD.

In embodiments, a dosing regimen comprises administering to a patient a dose of Compound **1** that is at least 300 mg (*e.g.,* a dose that is 450 mg or 600 mg) as described herein. In embodiments, the dosing regimen is determined for a patient that has previously received darbepoetin alfa (DA) (*e.g*., within about eight weeks of commencing treatment with Compound **1** or being screened for treatment with Compound **1).** In embodiments, the patient has previously received a dose of darbepoetin alfa that is ≥ about 15 µg. In embodiments, the patient is dialysis-dependent (a DD-CKD patient). In embodiments, the patient receives dialysis such as hemodialysis (a HD-CKD patient) or peritoneal dialysis (PD-CKD). In embodiments, the patient is non-dialysis dependent (a NDD-CKD patient). In embodiments, the dose is 300 mg of Compound **1.** In embodiments, the dose is 450 mg of Compound **1.** In embodiments, the dose is 600 mg of Compound **1.** In embodiments, Compound **1** is administered daily. In embodiments, Compound **1** is administered once weekly. In embodiments, Compound **1** is administered three times per week.

In embodiments, a dosing regimen comprises administering to a patient a dose of Compound **1** that is at least 300 mg (*e.g.,* a dose that is at 450 mg or 600 mg) as described herein. In embodiments, the dosing regimen is determined for a patient that has previously received darbepoetin alfa (DA) (*e.g*., within about eight weeks of commencing treatment with Compound **1** or being screened for treatment with Compound **1).** In embodiments, the patient has previously received a dose of darbepoetin alfa that is ≥ 15 µg. In embodiments, the patient is dialysis-dependent (a DD-CKD patient). In embodiments, the patient receives dialysis such as hemodialysis (a HD-CKD patient) or peritoneal dialysis (PD-CKD). In embodiments, the patient is non-dialysis dependent (a NDD-CKD patient). In embodiments, the dose is 300 mg of Compound **1.** In embodiments, the dose is 450 mg of Compound **1.** In embodiments, the dose is 600 mg of Compound **1.** In embodiments, Compound **1** is administered daily. In embodiments, Compound **1** is administered once weekly. In embodiments, Compound **1** is administered three times per week.

In embodiments, a dosing regimen comprises administering to a patient a dose of Compound **1** that is at least 300 mg (*e.g.,* a dose that is 450 mg or 600 mg) as described herein. In embodiments, the dosing regimen is determined for a patient that has previously received an epoetin such as epoetin alfa or epoetin beta (*e.g*., within about eight weeks of commencing treatment with Compound **1** or being screened for treatment with Compound **1).** In embodiments, the patient has previously received a dose of an epoetin such as epoetin alfa or epoetin beta that is ≥ 4500 IU. In embodiments, the patient is dialysis-dependent (a DD-CKD patient). In embodiments, the patient receives dialysis such as hemodialysis (a HD-CKD patient) or peritoneal dialysis (a PD-CKD patient). In embodiments, the dose is 300 mg of Compound **1.** In embodiments, the dose is 450 mg of Compound **1.** In embodiments, the dose is 600 mg of Compound **1.** In embodiments, Compound **1** is administered daily. In embodiments, Compound **1** is administered once weekly. In embodiments, Compound **1** is administered three times per week.

Doses of Compound **1** are taken orally. Doses of Compound **1** may be taken while fasting, together with fluids, or together with food of any kind. In specific embodiments, doses of Compound **1** may be taken or 1, 2, 3, 4, 5, 6, 7, 8, 9, 10, 11, or 12 hours after a meal, or 1, 2, 3, 4, 5, 6, 7, 8, 9, 10, 11, or 12 hours before a meal. Doses of Compound **1** may be taken at any time of day. In certain embodiments, repeat doses are administered at the same time during the day. In certain embodiments, the dose doses are administered in the morning, around mid-day, or in the evening. In certain embodiments, the doses are administered between 4.00 am and 2.00 pm. In certain embodiments, the doses are administered between 5.00 am and 1.00 pm. In certain embodiments, the doses are administered between 6.00 am and 12.00 noon. In certain embodiments, the doses are administered between 7.00 am and 11.00 am. In certain embodiments, the doses are administered between 8.00 am and 10.00 am. In certain embodiments, the doses are administered before, during, or after breakfast. Administration and dosing regimens may be adjusted as described herein.

In a specific embodiment, the patient is initially treated with an 300 mg dose of Compound **1.** Dose levels of the compound include 150, 300, 450, and 600 mg. Thereafter, the medication is taken once daily during the course of treatment. After the initial dose, patients receive a maintenance dose of Compound 1 that is between 150 mg to 600 mg per day. The patient should take the study medication with 4 ounces of water or other oral beverage, regardless of food intake. The dose is taken at approximately the same time each day, preferably between 7 AM and 2 PM.

This section provides several exemplary doses for Compound **1.** In certain embodiments, such a dose is the initial dose at the beginning of a treatment. In other embodiments, such a dose is the adjusted dose at a later time during the course of treatment.

In embodiments of any methods described herein, a dose of Compound **1** can be adjusted or maintained based on a patient's hemoglobin (Hb) level. In embodiments, a dose of Compound **1** is adjusted (e.g., by 150 mg of Compound **1**) if a patient's hemoglobin (Hb) level is < 11.0 g/dL or > 11.5 g/dL. In embodiments, a dose is increased. In embodiments, a dose is decreased. In embodiments, a method comprises adjusting the dose by 150 mg of Compound **1** if a patient's hemoglobin (Hb) level is < 10.0 g/dL or > 11.5 g/dL. In embodiments, a method comprises adjusting the dose by 150 mg of Compound **1** if a patient's hemoglobin (Hb) level is < 10.0 g/dL or > 12.5 g/dL. In embodiments, a method comprises adjusting the dose by 150 mg of Compound 1 if a patient's hemoglobin (Hb) level is < 10.0 g/dL. In embodiments, a method comprises adjusting the dose comprises increasing the dose by 150 mg of Compound **1.** In embodiments, a method comprises adjusting the dose by 150 mg of Compound 1 if the patient's hemoglobin (Hb) level is > 11.5 g/dL. In methods a method comprises adjusting the dose comprises decreasing the dose by 150 mg of Compound 1. In embodiments, a method comprises adjusting the dose by 150 mg of Compound **1** if the patient's hemoglobin (Hb) level is < 10.0 g/dL. In embodiments, a method comprises adjusting the dose comprises increasing the dose by 150 mg of Compound **1.** In embodiments, a method comprises adjusting the dose by 150 mg of Compound **1** if the patient's hemoglobin (Hb) level is > 11.5 g/dL. In embodiments, a method comprises adjusting the dose comprises decreasing the dose by 150 mg of Compound **1.**

In embodiments of any methods described herein, a dose of Compound **1** can be adjusted or maintained based on a change in a patient's hemoglobin (Hb) level. In embodiments, a dose is decreased by 150 mg of Compound **1** if the patient's hemoglobin (Hb) level increases by > 1.0 g/dL in a 2-week period or by > 2.0 g/dL in about a 4-week period.

In embodiments of any methods described herein, adjusting the dose occurs no more than once in at least every 2 weeks. In embodiments of any methods described herein, adjusting the dose occurs no more than once in at least every 4 weeks. In embodiments of any methods described herein, adjusting the dose occurs no more than once in at least every 6 weeks. In embodiments of any methods described herein, decreasing the dose occurs no more than once in at least every 2 weeks.

### Formulations (Pharmaceutical Compositions) of Compound 1

In certain embodiments, Compound **1** may be provided as a formulation (pharmaceutical composition). In embodiments, a formulation is an oral dosage form (*e.g*., a tablet or capsule).

Exemplary formulations of Compound **1** are described in WO 2014/200773 and WO 2016/161094. Still further exemplary formulations are described herein.

Also described herein are anhydrous pharmaceutical compositions and dosage forms since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g*., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

An anhydrous pharmaceutical composition should be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

Also described herein are pharmaceutical compositions and dosage forms that comprise one or more compounds that reduce the rate by which an active ingredient will decompose. Such compounds, which are referred to herein as "stabilizers", include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients.

### Excipients

A formulation comprising Compound **1** may comprise one or more excipients.

In certain examples, pharmaceutical compositions and dosage forms comprise one or more excipients. Suitable excipients are well known to those skilled in the art of pharmacy, and nonlimiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, provided are pharmaceutical compositions and dosage forms that contain little, if any, lactose other mono- or disaccharides. As used herein, the term "lactose-free" means that the amount of lactose present, if any, is insufficient to substantially increase the degradation rate of an active ingredient.

Lactose-free compositions can comprise excipients that are well known in the art and are listed, for example, in the U.S. Pharmacopeia (USP) 25 NF20 (2002). In general, lactose-free compositions comprise active ingredients, a binder/filler, and a lubricant in pharmaceutically compatible and pharmaceutically acceptable amounts. In one example, lactose-free dosage forms comprise active ingredients, microcrystalline cellulose, pre-gelatinized starch, and magnesium stearate.

Examples of excipients that can be used in formulations described herein include, but are not limited to, insoluble diluents, binders, fillers, disintegrants, glidants, carriers, and lubricants.

In examples, formulations of Compound **1** comprise: one or more diluents and/or filler; one or more disintegrants; one or more lubricants; and/or one or more glidants.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methylcellulose, (*e.g*., Nos. 2208, 2906, 2910), microcrystalline cellulose, and mixtures thereof.

Examples of insoluble diluents and carriers suitable for use in the pharmaceutical compositions and dosage forms provided herein include, but are not limited to, dibasic calcium phosphate and microcrystalline cellulose. Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC-581. Suitable anhydrous or low moisture excipients or additives include AVICEL-PH-103^{™} and Starch 1500 LM. Other suitable forms of microcrystalline cellulose include, but are not limited to, silicified microcrystalline cellulose, such as the materials sold as PROSOLV 50, PROSOLV 90, PROSOLV HD90, PROSOLV 90 LM, and mixtures thereof.

Examples of diluents / fillers suitable for use in the pharmaceutical compositions and dosage forms provided herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, hydroxypropyl methylcellulose (HPMC or hypromellose) (*e.g*., Methocel E5 Premium LV) and mixtures thereof. In certain example, fillers may include, but are not limited to block copolymers of ethylene oxide and propylene oxide. Such block copolymers may be sold as POLOXAMER or PLURONIC, and include, but are not limited to POLOXAMER 188 NF, POLOXAMER 237 NF, POLOXAMER 338 NF, POLOXAMER 437 NF, and mixtures thereof. In certain examples, fillers may include, but are not limited to isomalt, lactose, lactitol, mannitol, sorbitol xylitol, erythritol, and mixtures thereof.

Disintegrants may be used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients may be used to form solid oral dosage forms. The amount of disintegrant used varies based upon the type of formulation, and is readily discernible to those of ordinary skill in the art.

Disintegrants that can be used in pharmaceutical compositions and dosage forms include, but are not limited to, agar-agar, alginic acid, calcium carbonate, microcrystalline cellulose, croscarmellose sodium, povidone, crospovidone, polacrilin potassium, sodium starch glycolate (*e.g*., Explotab^{®}), potato or tapioca starch, other starches, pre-gelatinized starch, other starches, clays, other algins, other celluloses, gums, and mixtures thereof.

Glidants that can be used in pharmaceutical compositions and dosage forms include, but are not limited to fumed silica, magnesium carbonate, magnesium stearate, colloidal silicon dioxide (*e.g*., Aerosil, Cab-O-Sil), starch and talc.

Lubricants that can be used in pharmaceutical compositions and dosage forms include, but are not limited to, calcium stearate, magnesium stearate (*e.g*. Hyqual^{®} 5712), mineral oil, light mineral oil, glycerin, sorbitol, mannitol, polyethylene glycol, other glycols, stearic acid, sodium stearyl fumarate, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic colloidal silicon dioxide product sold by Cabot Co. of Boston, MA), and mixtures thereof.

In some examples, formulations of Compound **1** can comprise intra-granular components, extra-granular components, and film coating components, wherein the intra-granular components comprise Compound **1,** an insoluble diluent or carrier, a disintegrant, and a diluent or filler; wherein the extra-granular components comprise a disintegrant, a glidant, and/or a lubricant; and wherein the film coating components comprise a tablet coating.

In certain examples, provided herein are formulations of Compound **1** that comprise about 10%, about 15%, about 20%, about 25%, about 30%, about 35%, about 40%, about 45%, about 50%, about 55%, about 60%, about 65%, about 70%, about 75%, about 80%, about 85%, or about 90% by weight of Compound **1,** wherein the weight is the total weight of all intra-granular and extra-granular components of a tablet.

In certain examples, provided herein are formulations of Compound **1** that comprise about 5%, about 10%, about 15%, about 20%, about 25%, about 30%, about 35%, about 40%, about 45%, or about 50%, by weight of an insoluble diluent or carrier, wherein the weight is the total weight of all intra-granular and extra-granular components of a tablet.

In certain examples, provided herein are formulations of Compound **1** that comprise about 1%, about 1.5%, about 2.0%, about 2.5%, about 3%, about 3.5%, about 4%, about 4.5%, about 5.0%, about 5.5%, about 6.0%, about 6.5%, about 7%, about 7.5%, about 8%, about 8.5%, about 9.0%, about 9.5%, or about 10%, by weight of a disintegrant, wherein the weight is the total weight of all intra-granular and extra-granular components of a tablet.

In certain examples, provided herein are formulations of Compound **1** that comprise about 0.05%, about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, or about 0.8%, by weight of a glidant, wherein the weight is the total weight of all intra-granular and extra-granular components of a tablet.

In certain examples, provided herein are formulations of Compound **1** that comprise about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, or about 0.8%, about 0.85%, about 0.9%, about 0.95%, about 1.0%, about 1.05%, about 1.1%, about 1.15%, about 1.2%, about 1.2%, about 1.25%, about 1.3%, about 1.35%, about 1.4%, about 1.45%, or about 1.5%, by weight of a lubricant, wherein the weight is the total weight of all intra-granular and extra-granular components of a tablet.

In certain examples, described herein are formulations of Compound **1** comprising intra-granular components, extra-granular components, and film coating components, wherein the intra-granular component comprises about 50% to about 80% by weight of Compound 1, about 10% to about 40% by weight of an insoluble diluent or carrier, about 1.5% to about 4.5% by weight of a disintegrant, and about 1% to about 5% by weight of a diluent or filler; wherein the extra-granular component comprises about 1.5% to about 4.5% by weight of a disintegrant, about 0.1% to about 0.4% by weight of a glidant, and about 0.15% to about 1.35% by weight of a lubricant; and wherein the film coating component comprises about 1.0% to about 8% by weight of a tablet coating; and wherein the weight is the total weight of all intra-granular and extra-granular components.

In certain examples, described herein are formulations of Compound **1** comprising intra-granular components, extra-granular components, and film coating components, wherein the intra-granular component comprises about 55% to about 75% by weight of Compound 1, about 15% to about 35% by weight of an insoluble diluent or carrier, about 2.0% to about 4.0% by weight of a disintegrant, and about 1.8% to about 3.8% by weight of a diluent or filler; wherein the extra-granular component comprises about 2.0% to about 4.0% by weight of a disintegrant, about 0.15% to about 0.35% by weight of a glidant, and about 0.35% to about 1.15% by weight of a lubricant; and wherein the film coating component comprises about 1.0% to about 8% by weight of a tablet coating; and wherein the weight is the total weight of all intra-granular and extra-granular components.

In certain examples, described herein are formulations of Compound **1** comprising intra-granular components, extra-granular components, and film coating components, wherein the intra-granular component comprises about 60% to about 70% by weight of Compound **1,** about 20% to about 30% by weight of an insoluble diluent or carrier, about 2.5% to about 3.5% by weight of a disintegrant, and about 2.3% to about 3.3% by weight of a diluent or filler; wherein the extra-granular component comprises about 2.5% to about 3.5% by weight of a disintegrant, about 0.2% to about 0.3% by weight of a glidant, about 0.55% to about 0.95% by weight of a lubricant; and wherein the film coating component comprises about 1.0% to about 8% by weight of a tablet coating; and wherein the weight is the total weight of all intra-granular and extra-granular components.

In certain examples, described herein are formulations of Compound **1** comprising intra-granular components, extra-granular components, and film coating components, wherein the intra-granular component comprises about 65% by weight of Compound **1,** about 25% by weight of an insoluble diluent or carrier, about 3% by weight of a disintegrant, and about 2.8% by weight of a diluent or filler; wherein the extra-granular component comprises about 3% by weight of a disintegrant, about 0.25% by weight of a glidant, about 0.75% by weight of a lubricant; and wherein the film coating component comprises about 2.0% to about 6.0% by weight of a tablet coating; and wherein the weight is the total weight of all intra-granular and extra-granular components.

In one examples, formulations of Compound **1** comprise one or more diluents/fillers (*e.g*., microcrystalline cellulose and/or HPMC (hypromellose)) and a disintegrant (*e.g*., sodium starch glycolate). In another example, a formulation further comprises one or more glidants (*e.g*., colloidal silicon dioxide and/or magnesium stearate).

In examples, formulations of Compound **1** comprise one or more diluents/fillers (*e.g*., microcrystalline cellulose and/or isomalt), one or more disintegrants (*e.g*., sodium starch glycolate and/or povidone), and one or more lubricants (*e.g*., sodium lauryl sulfate). In examples, a formulation further comprises one or more glidants (*e.g*., colloidal silicon dioxide and/or magnesium stearate). In examples, a formulation comprises one or more excipients selected from the group consisting of microcrystalline cellulose, sodium starch glycolate, and HPMC (hypromellose). In examples, a formulation comprises microcrystalline cellulose, sodium starch glycolate, and HPMC (hypromellose).

In examples, the formulations of Compound **1** comprise a film-coating components comprising Opadry^{®}. Opadry^{®} is a commercial film-coating that is a formulated powder blend provided by Colorcon. Opadry^{®} combines polymer, plasticizer and pigment in a dry concentrate. Examples of Opadry^{®} include, but are not limited to, Opadry^{®} I (HPC/HPMC), Opadry^{®} 20A18334, Opadry^{®} II, Opadry^{®} II HP (PVA-PEG), or another suitable Opadry^{®} suspension (such as polyvinyl alcohol, polyethylene glycol, titanium dioxide, and talc, with or without colorants).

In certain examples, described herein are formulations of Compound **1** comprising intra-granular components, extra-granular components, and film coating components, wherein the intra-granular components comprise Compound **1,** microcrystalline cellulose, sodium starch glycolate, and hydroxypropyl methylcellulose, wherein the extra-granular components comprise sodium starch glycolate, colloidal silicon dioxide, and magnesium stearate; and wherein the film-coating components comprise Opadry^{®}.

In certain examples, described herein are formulations of Compound **1** comprising intra-granular components, extra-granular components, and film coating components, wherein the intra-granular component comprises about 50% to about 80% by weight of Compound **1,** about 10% to about 40% by weight of microcrystalline cellulose, about 1.5% to about 4.5% by weight of sodium starch glycolate, and about 1% to about 5% by weight of a hydroxypropyl methylcellulose; wherein the extra-granular component comprises about 1.5% to about 4.5% by weight of a sodium starch glycolate, about 0.1% to about 0.4% by weight of colloidal silicon dioxide, and about 0.15% to about 1.35% by weight of magnesium stearate; wherein the film coating component comprises about 1.0% to about 8% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 55% to about 75% by weight of Compound **1,** about 15% to about 35% by weight of microcrystalline cellulose, about 2.0% to about 4.0% by weight of sodium starch glycolate, and about 1.8% to about 3.8% by weight of a hydroxypropyl methylcellulose; wherein the extra-granular component comprises about 2.0% to about 4.0% by weight of a sodium starch glycolate, about 0.15% to about 0.35% by weight of colloidal silicon dioxide, and about 0.35% to about 1.15% by weight of magnesium stearate; wherein the film coating component comprises about 1.0% to about 8% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 60% to about 70% by weight of Compound **1,** about 20% to about 30% by weight of microcrystalline cellulose, about 2.5% to about 3.5% by weight of sodium starch glycolate, and about 2.3% to about 3.3% by weight of a hydroxypropyl methylcellulose; wherein the extra-granular component comprises about 2.5% to about 3.5% by weight of a sodium starch glycolate, about 0.2% to about 0.3% by weight of colloidal silicon dioxide, and about 0.55% to about 0.95% by weight of magnesium stearate; wherein the film coating component comprises about 1.0% to about 8% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 60% by weight of Compound **1,** about 30% by weight of microcrystalline cellulose, about 3% by weight of sodium starch glycolate, and about 2.8% by weight of a hydroxypropyl methylcellulose; wherein the extra-granular component comprises about 3% by weight of a sodium starch glycolate, about 0.25% by weight of colloidal silicon dioxide, and about 0.75% by weight of magnesium stearate; wherein the film coating component comprises about 2.0% to about 6.0% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 65% by weight of Compound **1,** about 25% by weight of microcrystalline cellulose, about 3% by weight of sodium starch glycolate, and about 2.8% by weight of a hydroxypropyl methylcellulose; wherein the extra-granular component comprises about 3% by weight of a sodium starch glycolate, about 0.25% by weight of colloidal silicon dioxide, and about 0.75% by weight of magnesium stearate; wherein the film coating component comprises about 2.0% to about 6.0% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 70% by weight of Compound **1,** about 20% by weight of microcrystalline cellulose, about 3% by weight of sodium starch glycolate, and about 2.8% by weight of a hydroxypropyl methylcellulose; wherein the extra-granular component comprises about 3% by weight of a sodium starch glycolate, about 0.25% by weight of colloidal silicon dioxide, and about 0.75% by weight of magnesium stearate; wherein the film coating component comprises about 2.0% to about 6.0% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 75% by weight of Compound **1,** about 15% by weight of microcrystalline cellulose, about 3% by weight of sodium starch glycolate, and about 2.8% by weight of a hydroxypropyl methylcellulose; wherein the extra-granular component comprises about 3% by weight of a sodium starch glycolate, about 0.25% by weight of colloidal silicon dioxide, and about 0.75% by weight of magnesium stearate; wherein the film coating component comprises about 2.0% to about 6.0% by weight of Opadry^{®}; and wherein the weight is the total weight of all intra-granular and extra-granular components.

In certain examples, described herein are formulations of Compound **1** comprising intra-granular components, extra-granular components, and film coating components, wherein the intra-granular component comprises about 80% by weight of Compound **1,** about 10% by weight of microcrystalline cellulose, about 3% by weight of sodium starch glycolate, and about 2.8% by weight of a hydroxypropyl methylcellulose; wherein the extra-granular component comprises about 3% by weight of a sodium starch glycolate, about 0.25% by weight of colloidal silicon dioxide, and about 0.75% by weight of magnesium stearate; wherein the film coating component comprises about 2.0% to about 6.0% by weight of Opadry^{®}; and wherein the weight is the total weight of all intra-granular and extra-granular components.

### Oral Dosage Forms

Pharmaceutical compositions that are suitable for oral administration can be provided as discrete dosage forms, such as, but not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. See generally, Remington's The Science and Practice of Pharmacy, 21st Ed., Lippincott Williams & Wilkins (2005).

Oral dosage forms provided herein are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g*., powders, tablets, capsules, and caplets) include, but are not limited to, starches, sugars, microcrystalline cellulose, diluents, granulating agents, lubricants, binders, and disintegrating agents.

In one example, oral dosage forms are tablets or capsules, in which case solid excipients are employed. In another example, tablets can be coated by standard aqueous or non-aqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

### Liquid Dosage Forms

Liquid dosage forms of Compound **1** for oral administration include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents, and emulsifiers such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols, and fatty acid esters of sorbitan, and mixtures thereof.

Besides inert diluents, the oral compositions can also include adjuvants such as wetting agents, emulsifying and suspending agents, sweetening, flavoring, coloring, perfuming, and preservative agents.

Suspensions, in addition to the active inhibitor(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

### Amounts of Compound 1

In certain other embodiments, provided herein are unit dosage forms of Compound **1** that comprise between 150 mg and 600 mg of a compound having a structure of Compound **1,** or a pharmaceutically acceptable salt thereof.

In certain other embodiments, provided herein are unit dosage forms of Compound **1** that comprise 150 mg, 200 mg, 250 mg, 300 mg, 350 mg, 400 mg, 450 mg, 500 mg, 550 mg, or even 600 mg of a compound having a structure Compound **1.** In certain embodiments, the unit dosage form comprises 150 mg, 185 mg, 200 mg, 250 mg, 300 mg, or even 315 mg of a compound having a structure of Compound **1,** or a pharmaceutically acceptable salt thereof. In certain such embodiments, the unit dosage form is a capsule comprising 185 mg, 200 mg, 200, 250 mg, or even 300 mg of the compound.

In embodiments, a unit dosage form comprises 150 mg of Compound **1.** In embodiments, a unit dosage form is a tablet (*e.g*., a film-coated tablet). In embodiments, a unit dosage form is a capsule. In embodiments, a unit dosage form comprises excipients that are microcrystalline cellulose, sodium starch glycolate, and/or hydroxypropyl methylcellulose. In embodiments, a unit dosage form comprises excipients that are sodium starch glycolate, colloidal silicon dioxide, and/or magnesium stearate.

In embodiments, a unit dosage form comprises 300 mg of Compound **1.** In embodiments, a unit dosage form is a tablet (*e.g*., a film-coated tablet). In embodiments, a unit dosage form is a capsule. In embodiments, a unit dosage form comprises excipients that are microcrystalline cellulose, sodium starch glycolate, and/or hydroxypropyl methylcellulose. In embodiments, a unit dosage form comprises excipients that are sodium starch glycolate, colloidal silicon dioxide, and/or magnesium stearate.

While embodiments of the present invention have been shown and described herein, it will be obvious to those skilled in the art that such embodiments are provided by way of example only. It should be understood that various alternatives to the embodiments of the invention described herein may be employed in practicing the invention. It is intended that the following claims define the scope of the invention and that methods and structures within the scope of these claims and their equivalents be covered thereby.

### EXEMPLIFICATION

### Example 1: Vadadustat (Compound 1) for the Treatment of Anemia in Subjects with Non-Dialysis-Dependent Chronic Kidney Disease.

This example describes the first open-label, active-controlled Phase 3 study of Compound **1** (Vadadustat or VDT or MT-6548) for treatment of anemia in subjects with Non-Dialysis-Dependent Chronic Kidney Disease (NDD-CKD) for up to 52 weeks. Efficacy and safety of Compound **1** was compared with darbepoetin alfa (DA) for the treatment of anemia in subjects with NDD-CKD. A subset of the subjects was switching to treatment with Compound **1** from an ESA therapy. These studies showed that Compound **1** is effective for controlling hemoglobin (Hb) levels (including within a target range) for patient groups who (1) did not receive erythropoietin stimulating agent (ESA) therapy within eight weeks prior to commencing vadadustat therapy ("correction" patient group) and (2) did receive ESA therapy within eight weeks of commencing vadadustat therapy ("conversion" patient group). These findings support the use of vadadustat as a durable, effective therapy for anemia in patients with NDD-CKD.

The study population consisted of subjects ≥20 years of age with NDD-CKD, estimated glomerular filtration rate (eGFR) < 60 mL/min/1.73 m², and hemoglobin (Hb) values greater than or equal to 8.0 g/dL and less than 11.0 g/dL (correction group) or greater than or equal to 9.0 g/dL and less than 12.5 g/dL (conversion group), who were currently treated with an erythropoiesis stimulating agent (ESA) for anemia.

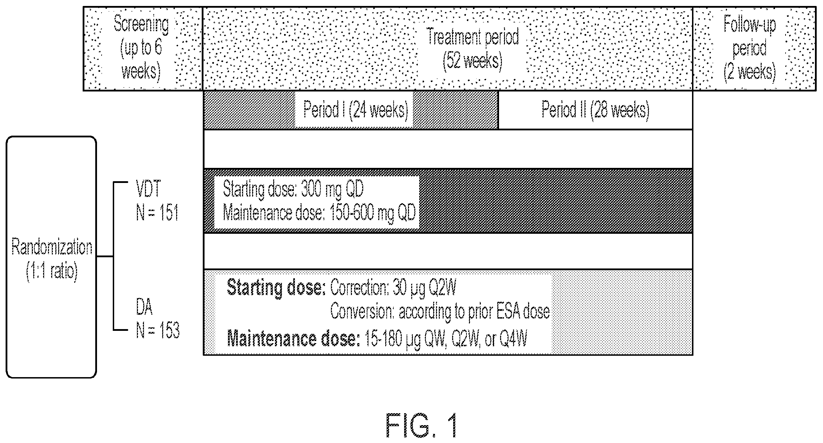

As shown in FIG. 1, following a screening period of up to 6 weeks, subjects who met all inclusion and no exclusion criteria described below were randomized 1:1 to Compound 1 or darbepoetin alfa. Following randomization, patients were treated for a period of up to 52 weeks. Patients were then observed post treatment for a period of two (2) weeks ("follow up" period).

### Selection and Withdrawal of Subjects

Subjects are selected for the study based on the following inclusion and exclusion criteria.

### Key Inclusion Criteria

In order to be eligible for this study, patients were required to meet *all* of the following inclusion criteria:
- Be at least 20 years of age, inclusive;
- Have diagnosis of Chronic Kidney Disease with an estimated glomerular filtration rate (eGFR) < 60 mL/minute/1.73 m² [eGFR (mL/min/1.73 m²) = 194 x (serum creatinine)^{-1.094} x (age)^{-0.287} (females: x 0.739)] at screening;
- Not have received dialysis within 8 weeks prior to the screening period nor expect to start dialysis during the treatment period;
- ESA Treatment
   ∘ ***Correction group*:** not receiving ESA for at least eight (8) weeks just before screening;
   ∘ ***Conversion group*:** receiving the same formulation of ESA using the same administration route and the same dose interval (the dose interval should be the one described in the package insert for the ESA formulation) for at least 8 weeks just before screening using the dosages described below:
      Epoetin alfa and epoetin beta: ≤12,000 IU every 2 weeks
      Darbepoetin alfa: ≤120 µg every 2 weeks
      Epoetin beta pegol: ≤250 µg every 4 weeks
- Baseline Hb:
   Correction group: ≥ 8.0 to < 11.0 g/dL
   Conversion group: ≥ 9.0 to ≤ 12.0 g/dL
- Have a difference of <1.5 g/dL in Hb for the latest two tests in the screening period;
- Have serum ferritin values of ≥100 ng/mL or transferrin saturation of ≥20% during the screening period; and
- Have folic acid and vitamin B12 values above standard minimum values during the screening period.

### Key Exclusion Criteria

Subjects deemed ineligible for the study included those who:
- Have anemia due to a cause other than CKD;
- Have active bleeding or blood loss during the eight (8) weeks just before screening;
- Have received a red blood cell transfusion during the eight (8) weeks just before screening;
- Have uncontrolled hypertension;
- Have active ocular fundus disease or cannot undergo an ocular fundus examination;
- Have been diagnosed with cardiovascular disease, malignancy, or hemisiderosis;
- Have a history of adverse drug reactions or a drug allergy; and
- Have previously received Compound **1.**

### Key Efficacy Endpoints

Efficacy endpoints for this study were defined as follows:

### Primary

- Average Hb level at weeks 20 and 24 of the treatment period.

### Secondary

- Hb level at each treatment visit; and
- The proportion of patients within the target Hb range (11.0-13.0 g/dL).

### Key Safety Endpoints

Safety endpoints for this study were defined as follows:
- Adverse events (AEs);
- Adverse drug reactions; and

### Other Efficacy Endpoints

Other endpoints for this study were defined as follows:
- Iron-related parameters: serum ferritin, transferrin saturation (TSAT), total iron-binding capacity (TIBC), hepcidin, serum iron, and monthly dose of iron by any route of administration (oral and intravenous); and
- Red blood cell indices.

### Statistical Analysis

### Primary Efficacy Endpoints

*Noninferiority:* Compound 1 was considered noninferior to Darbepoetin alfa if the 95% confidence interval (CI) lower limit for the difference between Compound 1 and Darbepoetin alfa in the Least Squares (LS) Mean of the average Hb at weeks 20 and 24 was more than or equal to the predefined noninferiority margin of -0.75 g/dL. Difference in LS Mean refers to mixed-model repeated measures (MMRM) with an unstructured covariance matrix within patients.

### Treatment of Subjects

Subjects were randomized 1:1 to either
- Compound **1** with an initial dose of 300 mg/day; or
- Darbepoetin alfa (subcutaneous) initial dose is as follows:

Dosing information for all subjects is shown in **FIG. 1****.**

For subjects already on darbepoetin, the initial dosing regimen in the study was based on the prior dosing regimen.

For subjects in the correction group, the initial dosing regimen was 30 µg of darbepoetin once every two weeks.

For subjects taking other ESAs, the initial dose of darbepoetin was based on the approved local product label.

For all subjects, no additional ESA doses were administered after Screening visit 2 (SV2) and prior to the Randomization visit.

### Dose Adjustment Guidelines

Dosing was initiated at the baseline visit, and the first dose of study medication (Compound **1** or darbepoetin alfa) was administered at the investigative site after other baseline procedures have been completed. For all subjects, no additional ESA doses were administered after SV2 and prior to the Randomization visit. Hemoglobin was monitored via HemoCue^{®} point of care device throughout the study to determine if the dose of study medication (Compound **1** or darbepoetin alfa) needed to be adjusted or suspended.

The aim is to maintain a Hb level of 11.0-13.0 g/dL throughout the study.

The Dose Adjustment Algorithm for Compound **1** and darbepoetin alfa is as follows (see below).

When adjusting therapy, Hb rate of rise, rate of decline, and variability as well as the subject's clinical condition (*i.e.,* recent illness, volume depletion, volume overload, *etc.*) were considered.

### Key Dose Adjustment Algorithm:

- Increases in the dose were not allowed to occur more frequently than once every 4 weeks. Decreases in dose were allowed to occur more frequently. Frequent dose adjustments were avoided.
- If the Hb rose rapidly, the dose was reduced.
- If the Hb fell below 11.0 g/dL, the dose was increased.
- If the Hb level exceeded 13.0 g/dL, the dose was reduced.

For subjects randomized to darbepoetin alfa, the initial dose is determined as follows:
- For subjects already on darbepoetin, the initial dosing regimen in the study was based on the prior dosing regimen.
- For subjects taking other ESAs, the initial dose of darbepoetin was based on the approved local product label.

### Dosing Instructions

### Compound 1

All subjects start with an initial dose of 300 mg at baseline visit. Dose levels of Compound **1** include 150, 300, 450, and 600 mg. Each subject took his/her first dose of study medication at the investigative site at the baseline visit. Thereafter, study medication was taken on an outpatient basis. Subjects took Compound **1** with or without food. The dose was taken at approximately the same time each day. The subject was instructed to take any oral iron supplements at least 2 hours before or 2 hours after the dose of Compound 1.

### Darbepoetin alfa

Darbepoetin alfa was administered, stored, and dispensed according to the approved local product label.

### Iron Supplementation

Investigators prescribed iron supplementation as needed during the study to maintain ferritin ≥100 ng/mL or TSAT ≥20%. Important: Because of the potential for oral iron to reduce the bioavailability of Compound 1, the study medication was not administered concurrently with an oral iron supplement. The subject was instructed to take any oral iron supplements at least two (2) hours before or two (2) hours after the dose of Compound 1.

### Results

304 patients were randomized 1:1 into groups receiving vadadustat (Compound **1**) therapy and darbepoetin alfa (DA) therapy. Of 151 patients in the vadadustat group, 130 completed treatment period 1. Of 153 patients in the DA group, 141 completed treatment period 1.

Patient characteristics of the correction population at baseline are shown in **Table 2.**

**Table 2. Patient Characteristics of the Correction Population at Baseline.**

| | | Compound **1** (N=71) | Darbepoetin alfa (N=71) |
|---|---|---|---|
| Sex (male), n (%) | | 34 (47.9) | 30 (42.3) |
| Age (year) | | 70.8 (10.8) | 71.7 (8.8) |
| Body weight (kg) | | 58.35 (13.29) | 57.89 (10.16) |
| BMI (kg/m²) | | 23.71 (3.56) | 23.58 (3.74) |
| Hb (g/dL) | | 10.17 (0.87) | 10.10 (0.77) |
| eGFR(mL/min/1.73 m²) | | 24.33 (12.40) | 25.22 (12.39) |

| eGFR category, n (%) | | | |
|---|---|---|---|
| <15 | | 22 (31.0) | 19 (26.8) |

| | | Compound **1** (N=71) | Darbepoetin alfa (N=71) |
|---|---|---|---|
| 15≤ to <30 | | 24 (33.8) | 31 (43.7) |
| 30 ≤ to <60 | | 25 (35.2) | 20 (28.2) |
| ≥60 | | 0 (0.0) | 1 (1.4) |
| CRP (mg/dL) | | 0.315 (0.783) | 0.196 (0.424) |
| Serum ferritin (ng/dL) | | 167.85 (146.65) | 142.35 (108.18) |
| TSAT(%) | | 29.3 (10.3) | 27.3 (8.3) |
| Prior ESA, n (%) | | | |
| | Epoetin | - | - |
| | Darbepoetin alpha | - | - |
| | Epoetin beta pegol | - | - |

| Comorbidities, n (%) | | | |
|---|---|---|---|
| | Hypertension | 67 (97.2) | 67 (94.4) |
| | Diabetes mellitus | 28 (39.4) | 27 (38.0) |
| | Dyslipidemia | 44 (62.0) | 47 (66.2) |

### Overall Population

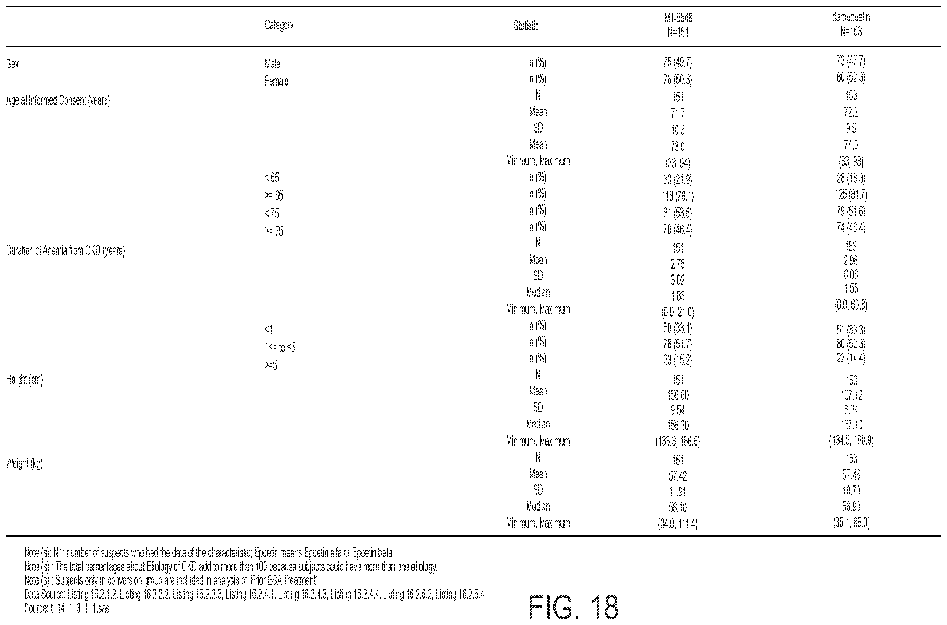

A description of the demographics and baseline characteristics of the overall patient population is provided in **FIG. 18****.**

In the Compound **1** group-overall, mean Hb increased from baseline, reached the target range at week 8, and thereafter remained within the target; durability of efficacy was confirmed up to week 52.

Indeed, the primary efficacy endpoint was as measured by average Hb at weeks 20 and 24. See **Table 3.** The 95% confidence intervals (Cls) of the average Hb at weeks 20 and 24 for both Compound 1 and darbepoetin alfa groups were within the target range of 11.0-13.0 g/dL. The 95% CI lower limit of the difference between groups (VDT-DA) was above the predefined noninferiority margin of -0.75 g/dL. That is, at week 24 the primary endpoint was met: The difference in mean hemoglobin (Hb) was -0.26 g/dL (95% CI -0.50, -0.02 g/dL), thus achieving the pre-specified non-inferiority criterion of -0.75 g/dL.

**Table 3. LSMean of the average* Hb at weeks 20 and 24 and the difference between Compound 1 and Darbepoetin alfa**

| Treatment Group | N | Average Hb, weeks 20 and 24 | |
|---|---|---|---|
| | | LSMean | 95% CI |
| Compound 1 | 151 | 11.66 | 11.49, 11.84 |
| Darbepoetin alfa | 153 | 11.93 | 11.76, 12.10 |
| Difference (Compound 1 - Darbepoetin alfa) | - | -0.26 | -0.50, -0.02 |
| *This MMRM model includes treatment group, visits, subject population (correction, conversion), interaction of treatment group and visits, interaction of visits and subject population (correction, conversion) as fixed effects, baseline values as covariate effects, and subject as a random effect (covariance matrix: unstructured). | | | |

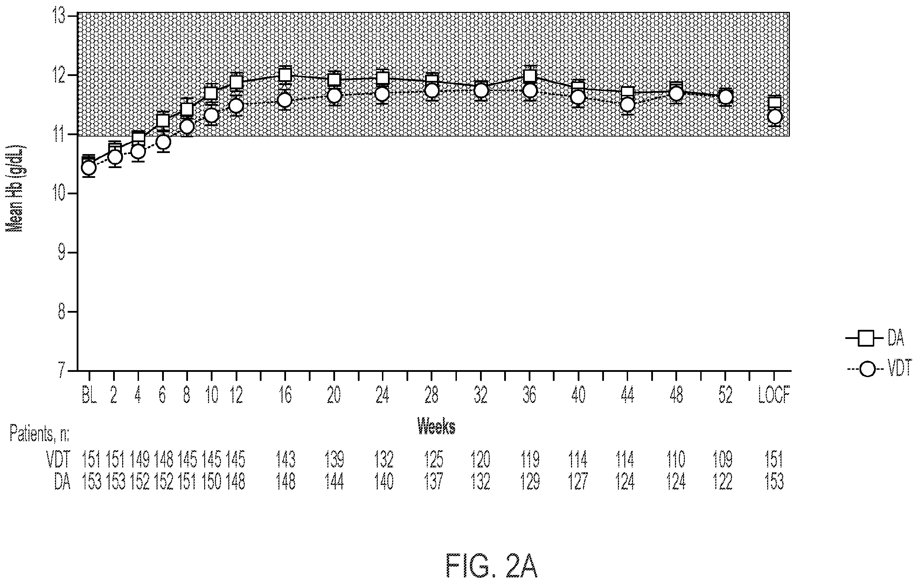

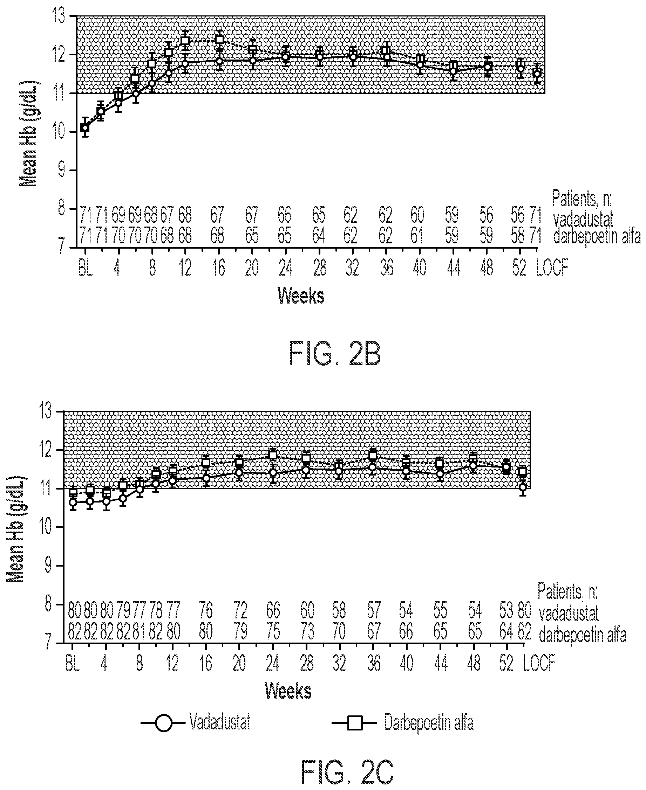

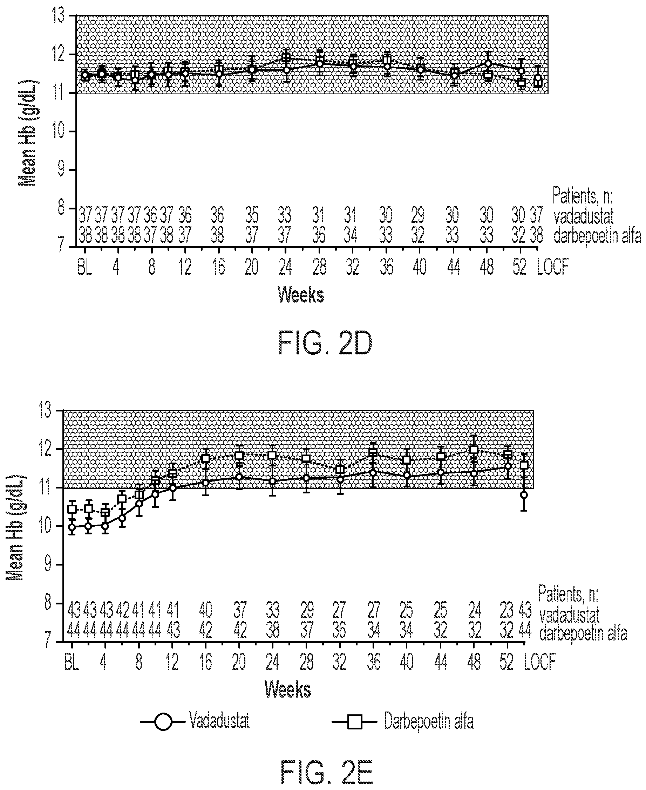

In the (Compound 1) vadadustat group, mean Hb increased from baseline, reached the target range at Week 8, and was maintained within the target range up to Week 52 (**FIG. 2A**). In ESA non-users, mean Hb increased from baseline, reached the target range at Week 8 and at Week 6 with vadadustat and darbepoetin alfa, respectively, then remained within the target range thereafter (**FIG. 2B**). The proportion of patients with Hb levels within the target range was 15.5% at baseline and increased to 71.4% at Week 52 with vadadustat and was 9.9% at baseline and increased to 84.5% at Week 52 with darbepoetin alfaHb, which confirms the durability of efficacy of treatment with Compound **1** (**FIG. 2**). Specifically, the mean Hb level at week 52 was 11.51 g/dL (95% CI 11.35, 11.67 g/dL) for Compound **1**-treated subjects compared to 11.58 g/dL (95% CI 11.43, 11.74 g/dL) for darbepoetin alfa-treated subjects (**FIG. 2**). The average dose for Compound **1** in the overall population is shown in **FIG. 3A** (24 weeks) and **FIG. 3B** (52 weeks). From Weeks 48 to 52, the mean dose was 335.65 mg/day (95% CI, 286.72-384.58) for Compound 1 and 16.37 µg/week (95% CI, 13.09-19.65) for darbepoetin alfa (**FIG. 3C**).

In ESA users, mean Hb levels were almost stable after conversion from a previous ESA in both groups. Mean Hb reached the target range at Week 8 and at Week 6 for vadadustat and darbepoetin alfa, respectively, and remained within the target range thereafter (**FIG. 2C**). The proportion of patients with Hb levels within the target range was 45.0% at baseline and increased to 79.2% at Week 52 with vadadustat and was 52.4% at baseline and increased to 76.6% at Week 52 with darbepoetin alfa, respectively. From Weeks 48 to 52, the mean dose was 403.67 mg/day (95% CI, 355.84-451.50) for Compound 1 and was 23.15 µg/week (95% CI, 18.29-28.00) for darbepoetin alfa (**FIG. 3D**).

In the Compound 1 group, ESA users were analyzed by mean Hb levels during the screening period (<11.0 g/dl, Hb ≥11.0 g/dl). For the Hb ≥11.0 g/dl subgroup, Hb levels were maintained within the target range throughout the treatment period (**FIG. 2D**). For the Hb <11.0 g/dl subgroup, mean Hb increased from baseline, reached the target range at 12 weeks, and remained within the target range thereafter (**FIG. 2E**). The proportion of patients with Hb levels within the target range at baseline and Week 52 was 91.9% and 80.0% for the Hb ≥11.0 g/dl subgroup, respectively and 4.7% and 78.3% for the Hb <11.0 g/dl subgroup, respectively.

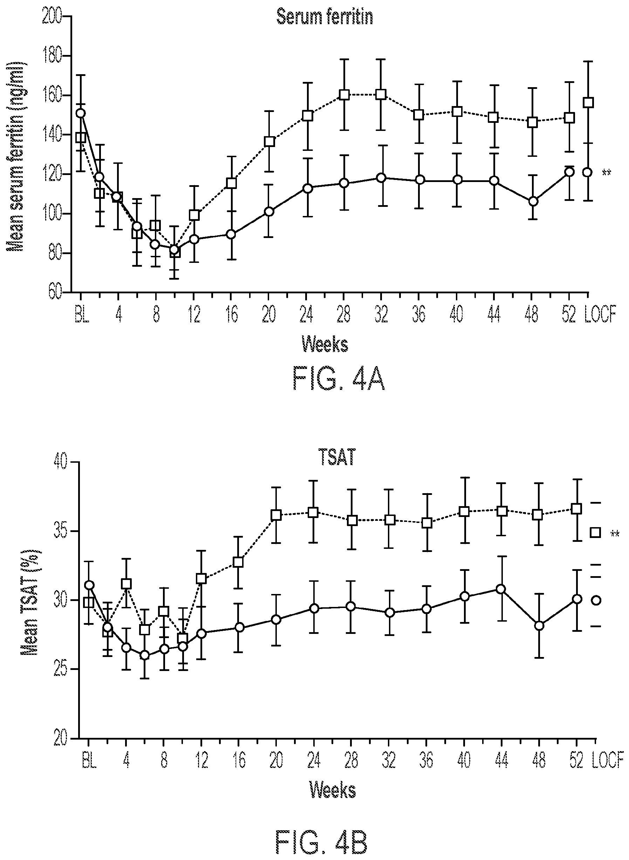

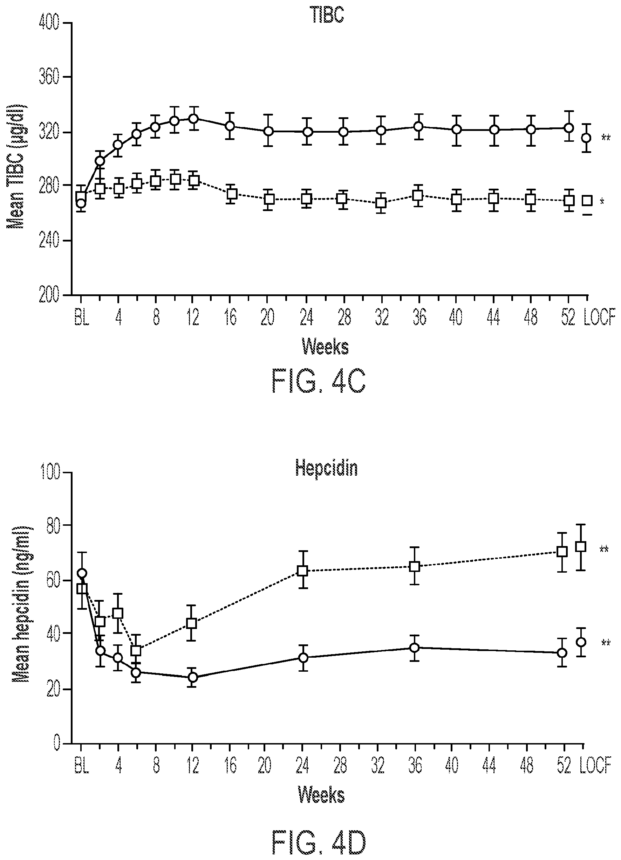

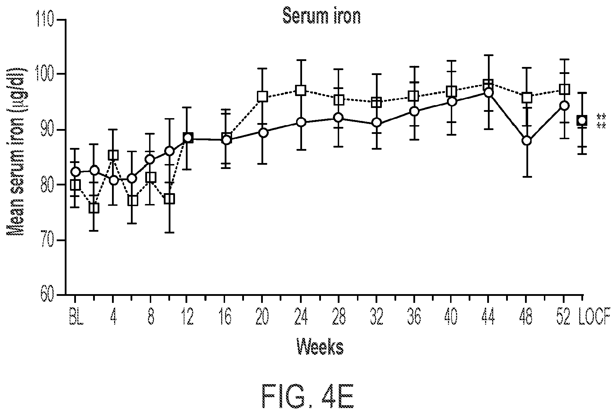

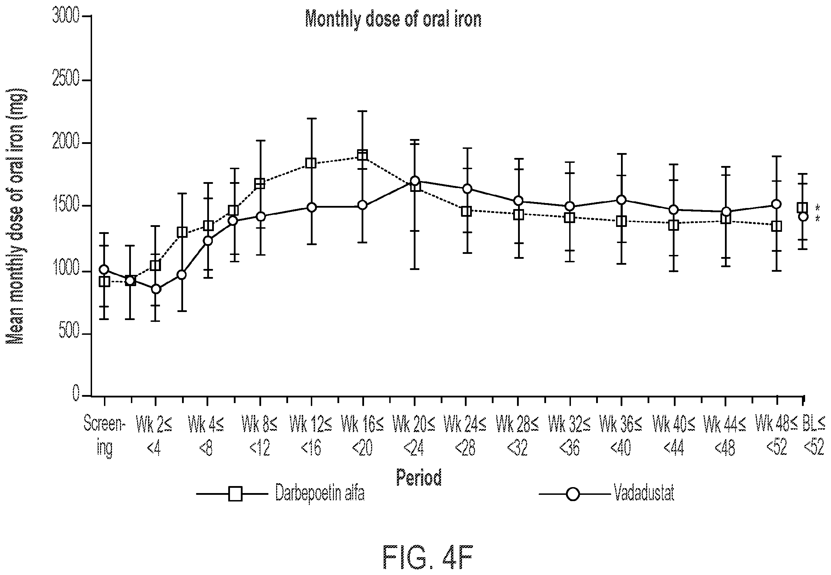

**FIG. 4** compares various iron related parameters for the overall population receiving therapy with Compound **1** and those receiving therapy with darbepoetin alfa (DA), including differences in serum ferritin (ng/mL), TSAT %, TIBC (µg/dL), hepcidin (ng/mL), serum iron (µg/dL), and the monthly dose of iron by any route (mg).

At 52 weeks LOCF compared with baseline, there were significant decreases in serum ferritin and hepicidin with vadadustat and no difference in serum ferritin and significant increase in hepcidin with darbepoetin alfa **(****FIGS. 4A****, 4D).** For TSAT, there was no significant difference with vadadustat at 52 weeks LOCF compared with baseline, but a significant increase with darbepoetin alfa (**FIG. 4B**). For TIBC, there was a significant increase at 52 weeks LOCF compared with baseline with vadadustat and a significant decrease with darbepoetin alfa (**FIG. 4C**). The proportions of patients receiving oral iron during the screening period and at Week 48 to 52 were 23.8% and 33.6%, respectively, in the Compound 1 group and 18.3% and 29.0%, respectively, in the darbepoetin alfa group.

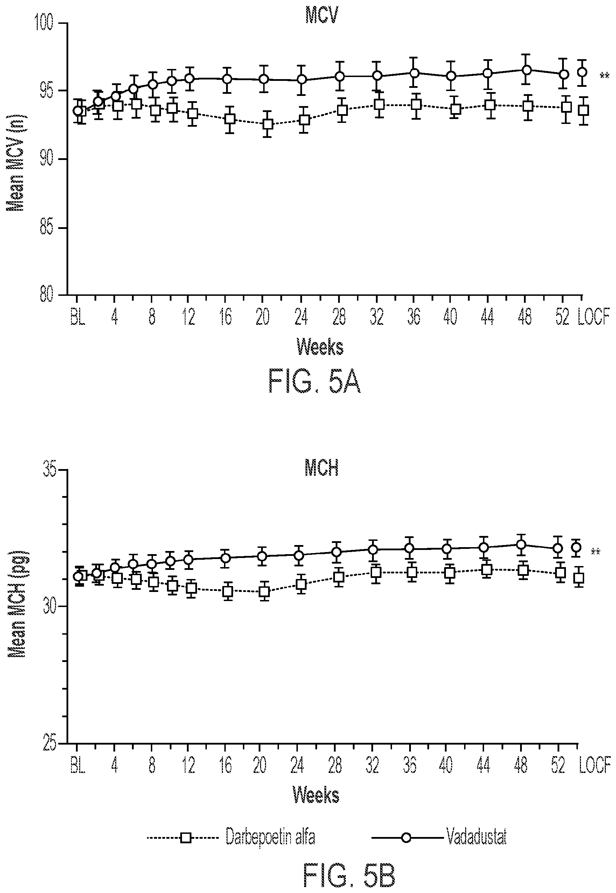

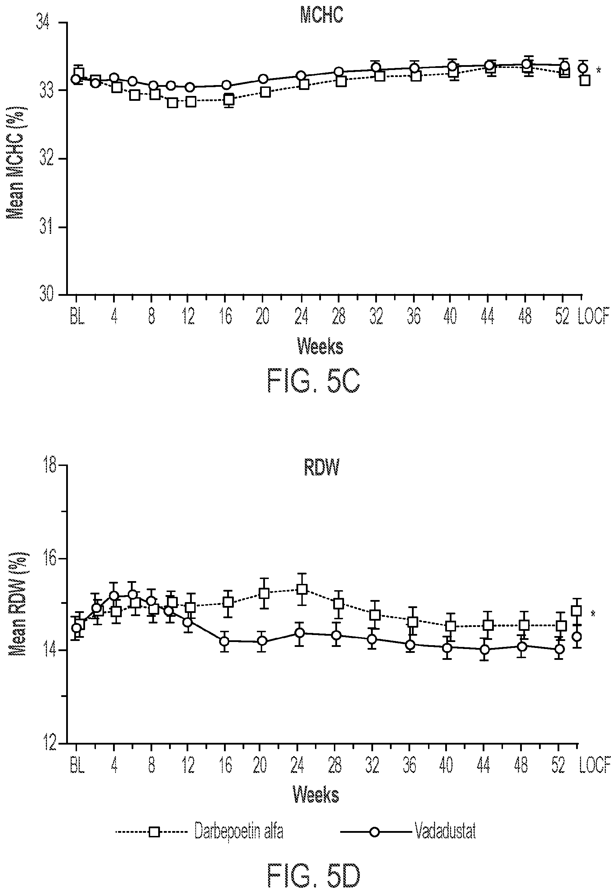

**FIG. 5** compares red blood cell indices MCH (mean corpuscular hemoglobin), MCHC (mean corpuscular hemoglobin concentration), MCV (mean corpuscular volume); and RDW (red cell distribution width) for Compound **1** and DA patients up to 52 weeks. In the Compound 1 group, MCV, MCH, and MCHC were significantly increased at 52 weeks LOCF compared with baseline (**FIGS. 5A, 5B****,** **5C**) and there were no significant differences in RDW (**FIG. 5D**). In the darbepoetin alfa group, there were no differences from baseline except for RDW, which was slightly but significantly higher than baseline (**FIG. 5D**).

*Safety assessment.* The proportions of patients reporting AEs and serious AEs were similar between the treatment groups. Up to week 52, at least one AE was reported by 90.1% and 92.2% of patients in the VDT and the DA groups, respectively (**Table 4**)**.** The proportions of patients reporting AEs and serious AEs were similar between groups. None of the serious AEs were considered related to study drug. The most commonly reported AEs with VDT were nasopharyngitis, diarrhea, and constipation. Although diarrhea was reported more frequently in the VDT group, most of the AEs were reported as mild in severity. No deaths were reported in the VDT group. The AEs of special interest include cardiovascular event, retinal disorder, malignancy, hyperkalemia, pulmonary hypertension, and cardiac failure.

**Table 4. Overview Adverse Events**

| **Overview of AEs, 52 weeks** | | | |
|---|---|---|---|
| **Type of AE** | | **VDT (N = 151)** | **DA (N** = **153)** |
| | | **n (%)** | **n (%)** |
| | Subjects with ≥1 AE | 136 (90.1) | 141 (92.2) |
| | Adverse drug reaction | 20 (13.2) | 7 (4.6) |
| | Serious AEs | 42 (27.8) | 49 (32.0) |
| | Serious adverse drug reaction | 0 (0.0) | 0 (0.0) |
| | Discontinuation due to AEs | 10 (6.6) | 6 (3.9) |
| | Dose reduction or interruption of study drug due to AEs | 11 (7.3) | 4 (2.6) |
| | Deaths due to AEs | 0 (0.0) | 1 (0.7) |

### Most Common AEs with VDT, 52 weeks

| **AE (preferred term)** | | **VDT (N = 151)** | **DA (N = 153)** |
|---|---|---|---|
| | | **n (%)** | **n (%)** |
| | Nasopharyngitis | 37 (24.5) | 43 (28.1) |
| | Diarrhea | 18 (11.9) | 8 (5.2) |
| | Constipation | 14 (9.3) | 11 (7.2) |

### AEs of Special Interest, 52 weeks

| **AE of special interest** | **VDT (N = 151)** | **DA (N = 153)** |
|---|---|---|
| | **n (%)** | **n (%)** |
| Cardiovascular event | 3 (2.0) | 4 (2.6) |
| Retinal disorder | 4 (2.6) | 12 (7.8) |
| Malignancy | 2 (1.3) | 6 (3.9) |
| Hyperkalemia | 1 (0.7) | 5 (3.3) |
| Pulmonary hypertension | 0 (0.0) | 0 (0.0) |
| Cardiac failure | 6 (4.0) | 2 (1.3) |

As shown in **Table 4,** the incidence of adverse events (AEs) was 90.1% in the vadadustat-treated group compared to 92.2% in the darbepoetin alfa-treated group. The top three most common AEs reported in vadadustat-treated subjects were nasopharyngitis (24.5%), diarrhea (11.9%), and constipation (9.3%). The incidence of serious adverse events (SAEs) was 27.8% in the Compound **1**-treated group compared to 32.0% in the darbepoetin alfa-treated group; no SAE was considered related to study drug.

Compound **1** was identified as effective for controlling Hb within the target Hb range for both correction and conversion patients, and no new safety concerns were identified. Treatment with Compound **1** is durable, with efficacy confirmed up to week 52. Further, treatment with Compound **1** was associated with significant increases in TIBC, MCV, and MCH, and decreases in hepcidin, suggesting an improvement in iron metabolism.

### Correction Group

For the correction group, the mean Hb increased from baseline, reached the target range at week 8 and remained within the target range thereafter (**FIG. 6A**). For the correction group, Compound **1** improved average Hb from baseline at week 24 from 10.17 to 11.85 g/dL in correction patients.

At week 24, the proportion of patients within the target Hb range of 11.0-13.0 g/dL, was 69.7% and 72.3% for correction patients in the Compound 1 and Darbepoetin alfa groups, respectively (**FIG. 6B**). **FIG. 6D** shows the mean hemoglobin level of patients through week 52 of the study.

**FIG. 6C** shows the mean dose of Compound **1** (VDT) through week 24 of the study. **FIG. 6E** shows the average dose of Compound **1** (VDT) through week 52 of the study.

### Conversion Group

For the conversion group, mean Hb levels were almost stable after conversion from a previous ESA in both groups. The mean Hb reached the target range at week 8 and remained within the target range thereafter (**FIGS. 7A****,** **7D**). Specifically, Compound 1 improved average Hb from baseline at week 24 from 10.68 to 11.27 g/dL in conversion patients. At week 24, the proportion of patients in the conversion group with the target Hb range was 66.7% for the Compound 1 group and 82.7% for DA group (**FIG. 7B**). **FIG. 7C** shows the mean dose of Compound **1** (VDT) through week 24 of the study. **FIG.7D** shows the mean hemoglobin (Hb) level in the conversion group to week 52 of the study, and **FIG 7E** shows the average dose of Compound **1** (VDT) through week 52 of the study.

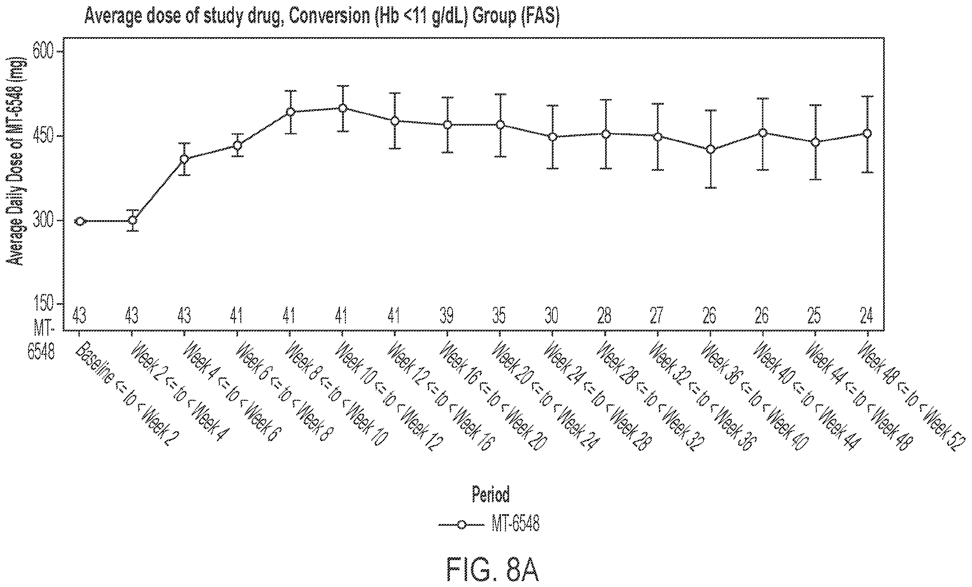

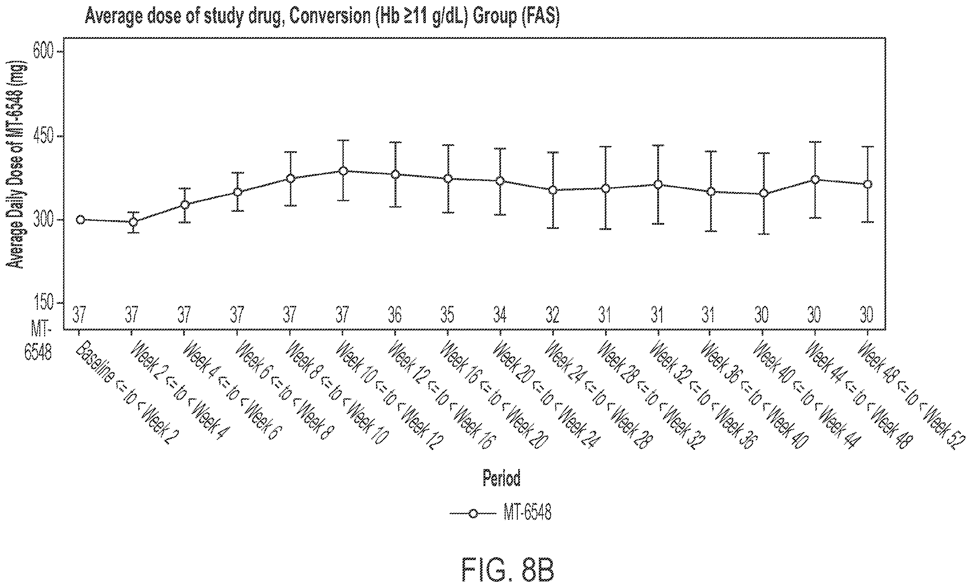

Compared to the correction group, the conversion group had a higher average dose of Compound **1.** Differences in average dose were also observed in further subpopulations of the conversion group. For example, patients with a hemoglobin (Hb) level that was < 11 g/dL received a higher average daily dose of Compound **1** than patients with a hemoglobin (Hb) level that was ≥ 11 g/dL **(****FIGS. 8A-8B****).**

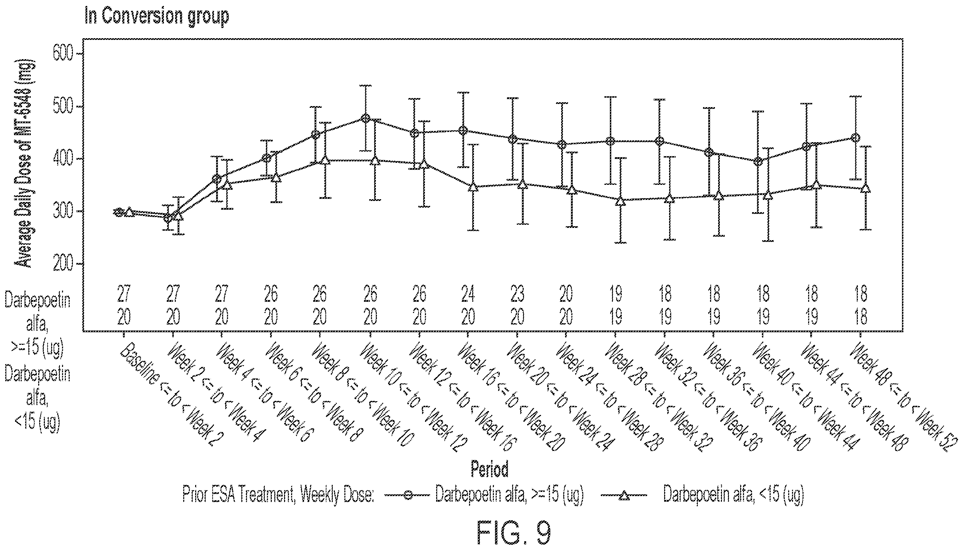

**FIG. 9** shows that the amount of an erythropoietin stimulating agent (ESA) therapy received can also influence the daily dose of Compound **1** over a 52-week period. For example, the average dose of Compound **1** was higher for patients who received weekly doses of ≥ 15 µg darbepoetin alfa as compared to patients who received weekly doses of < 15 µg darbepoetin alfa.

Compound **1** is effective for treatment of anemia in correction and conversion cohorts of NDD-CKD patients. Moreover, higher doses of Compound **1** may be particularly beneficial for NDD-CKD conversion patients with lower Hb levels and/or who have previously received higher doses of darbepoetin alfa (DA): for example, such patients can benefit from, *e.g*., higher doses of 450 or 600 mg of Compound **1.** Additionally, therapy using Compound **1** has shown to be effective (*e.g*., in maintaining target Hb levels) and durable over the course of the 52-week study period.

### Discussion

This is the first phase 3, randomized controlled trial to demonstrate noninferiority of once-daily, oral vadadustat to darbepoetin alfa administered by subcutaneous injection for treatment of anemia in patients with NDD-CKD. With Compound 1, mean Hb increased to the predefined target of 11.0-13.0 g/dl regardless of whether patients were previously treated with an ESA or not, and was maintained within the target range up to 52 weeks. Overall, Compound 1 was as safe as darbepoetin alfa and no major safety concerns were observed during the 52-week treatment period.

Overall, the findings from this study suggest that Compound 1, which was initiated at a dose of 300 mg and adjusted according to patient Hb levels to maintain Hb within the target range, can be used effectively and safely to treat anemia in patients with CKD who are in the predialysis phase. As the baseline characteristics of patients enrolled in this study were similar to those of large post-marketing studies in patients with anemia in NDD-CKD who started treatment with darbepoetin alfa or epoetin beta pegol^{1,2}, these findings can be generalized to treatment of anemia in patients with NDD-CKD in clinical practice. In clinical practice, many patients who are in the predialysis phase have not yet started treatment for anemia and, of those who are treated with an ESA, Hb levels can be well-controlled, but for many are below the target recommended by the guidelines.^{3,4,5} Therefore, because Applicants assessed the efficacy of Compound 1 in both ESA non-users and ESA users, these findings have further relevance to clinical practice. In this study, Compound 1 was effective at maintaining Hb within the target range in ESA non-users who had mean Hb at baseline of 8.0-11.0 g/dl, which is the level at which a guideline recommend anemia treatment is started.⁵ In addition, for ESA users, treatment with Compound 1 improved Hb levels in patients who had Hb <11.0 g/dl at screening and maintained Hb levels in those who had Hb ≥11.0 g/dl.

In addition to the decrease of erythropoietin in patients with CKD, inefficiency of iron utilization, which to a certain degree is affected by increased levels of hepcidin, is also a contributing factor of the onset of amemia.⁶ Stabilization of HIF decreases hepcidin production and increases the expression of multiple genes involved in iron transport and absorption, including genes for divalent metal transporter 1, duodenal cytochrome b, and transferrin.⁷ Additionally, there were beneficial changes in iron-related parameters in this study that were not seen with darbepoetin alfa. In particular, the significant increase in TIBC and significant decrease in serum ferritin and hepcidin that were observed with Compound 1 suggests that iron uptake and utilization had improved during treatment. Although red blood cell-related parameters were within the normal range during the study period in both treatment groups, significant increases from baseline in MCV, MCH, and MCHC were observed with Compound 1, which may reflect an improvement in iron utilization during treatment with vadadustat. In both treatment groups, there were significant increases in iron supplementation, which most likely were a result of the protocol to ensure that serum ferritin and TSAT were maintained at ≥100 ng/ml and ≥20%, respectively.

The rates of AE and SAEs in this study were similar between treatment groups and no SAEs were attributable to vadadustat or darbepoetin alfa. The most frequently reported AEs were gastrointestinal disorders and, although a higher frequency of patients reported diarrhea with vadadustat compared with darbepoetin alfa, most events of diarrhea were mild or moderate in severity. Although hypertension is a known adverse effect associated with ESA treatment,¹² the frequency of hypertension was low in both treatment groups in this study and importantly, there was no numerical increase in the frequency of hypertension with vadadustat compared with darbepoetin alfa.

The AEs of special interest with Compound 1 that were assessed in this study were those known to be associated with the mechanism of action of HIF-PHIs or previously associated with the HIF-PHI class.¹³ Overall, few AEs of special interest were reported and there were no clinically relevant differences between the treatment groups. Although one patient reported a case of retinal hemorrhage that was possibly related to Compound 1, the event was mild and the patient did not appear to have any clinically relevant changes in serum VEGF. An increased incidence of hyperkalemia has been reported in clinical trials of other HIF-PHIs for anemia in patients not on dialysis and in those undergoing dialysis.^{14,15} However, in this study, there was only one case of hyperkalemia in the Compound 1 group, which was not considered to be related to Compound 1.

In conclusion, this study is the first to demonstrate noninferiority of Compound 1 to darbepoetin alfa and the durability of efficacy of Compound 1 for up to 52 weeks of treatment for anemia in patients with NDD-CKD. Importantly, the safety of vadadustat in this study was consistent with previous reports in both Caucasian^{8,10,11} and Japanese⁹ patients and no new safety concerns were identified. Overall, these findings can support vadadustat for treatment of anemia in patients in the predialysis phase.

### References

1. Tanaka T, Nangaku M, Imai E, Tsubakihara Y, Kamai M, Wada M, et al.: Safety and effectiveness of long-term use of darbepoetin alfa in non-dialysis patients with chronic kidney disease: A post-marketing surveillance study in Japan. Clin Exp Nephrol 23: 231-243, 2019.
2. Hayashi T, Uemura Y, Kumagai M, Kimpara M, Kanno H, Ohashi Y, et al.: Effect of achieved hemoglobin level on renal outcome in non-dialysis chronic kidney disease (CKD) patients receiving epoetin beta pegol: MIRcerA Clinical Evidence on Renal Survival in CKD patients with renal anemia (MIRACLE-CKD Study). Clin Exp Nephrol 23: 349-361, 2019.
3. Akizawa T, Makino H, Matsuo S, Watanabe T, Imai E, Nitta K, et al.: Management of anemia in chronic kidney disease patients: Baseline findings from Chronic Kidney Disease Japan Cohort Study. Clin Exp Nephrol 15: 248-257, 2011.
4. Imai E, Matsuo S, Makino H, Watanabe T, Akizawa T, Nitta K, et al.: Chronic Kidney Disease Japan Cohort study: Baseline characteristics and factors associated with causative diseases and renal function. Clin Exp Nephrol 14: 558-570, 2010.
5. The Japanese Society of Nephrology: Clinical Practice Guidebook for Diagnosis and Treatment of Chronic Kidney Disease, 2012. Available at: https://cdn.jsn.or.jp/guideline/pdf/CKDguide2012.pdf. Accessed March 10, 2020.
6. Babitt JL, Lin HY: Mechanisms of anemia in CKD. J Am Soc Nephrol 23: 1631-1634, 2012.
7. Haase VH: HIF-prolyl hydroxylases as therapeutic targets in erythropoiesis and iron metabolism. Hemodial Int 21 Suppl 1: S110-S124, 2017.
8. Pergola PE, Spinowitz BS, Hartman CS, Maroni BJ, Haase VH: Vadadustat, a novel oral HIF stabilizer, provides effective anemia treatment in nondialysis-dependent chronic kidney disease. Kidney Int 90: 1115-1122, 2016.
9. Nangaku M, Farag YMK, deGoma E, Luo W, Vargo D, Khawaja Z: Vadadustat, an oral hypoxia-inducible factor prolyl hydroxylase inhibitor, for treatment of anemia of treating chronic kidney disease: Two randomized phase 2 trials in Japanese patients. Nephrol Dial Transplant Accepted: February, 2020.
10. Martin ER, Smith MT, Maroni BJ, Zuraw QC, deGoma EM: Clinical trial of vadadustat in patients with anemia secondary to stage 3 or 4 chronic kidney disease. Am J Nephrol 45: 380-388, 2017.
11. Haase VH, Chertow GM, Block GA, Pergola PE, deGoma EM, Khawaja Z, et al.: Effects of vadadustat on hemoglobin concentrations in patients receiving hemodialysis previously treated with erythropoiesis-stimulating agents. Nephrol Dial Transplant 34: 90-99, 2019.
12. Palmer SC, Navaneethan SD, Craig JC, Johnson DW, Tonelli M, Garg AX, et al.: Meta-analysis: Erythropoiesis-stimulating agents in patients with chronic kidney disease. Ann Intern Med 153: 23-33, 2010.
13. Sanghani NS, Haase VH: Hypoxia-inducible factor activators in renal anemia: Current clinical experience. Adv Chronic Kidney Dis 26: 253-266, 2019.
14. Chen N, Hao C, Liu BC, Lin H, Wang C, Xing C, et al.: Roxadustat treatment for anemia in patients undergoing long-term dialysis. N Engl J Med 381: 1011-1022, 2019.
15. Chen N, Hao C, Peng X, Lin H, Yin A, Hao L, et al.: Roxadustat for anemia in patients with kidney disease not receiving dialysis. N Engl J Med 381: 1001-1010, 2019.

### Example 2: Vadadustat (Compound 1) for the Maintenance Treatment of Anemia in Subjects with Hemodialysis-Dependent Chronic Kidney Disease.

This example describes a phase 3 randomized, double-blinded, active-controlled study to evaluate the efficacy and safety of oral Compound **1** for the maintenance treatment of anemia in subjects with Hemodialysis-Dependent Chronic Kidney Disease (HD-CKD) in approximately 300 subjects. Efficacy and safety of Compound **1** was compared with darbepoetin alfa for the maintenance treatment of anemia in subjects with DD-CKD after conversion from current ESA therapy.

The study population consisted of subjects ≥20 years of age with DD-CKD, diagnosed with CKD, had received either hemodialysis or hemodiafiltration 3 times a week for at least 12 weeks, had been receiving the same ESA therapy (epoetin alfa, epoetin beta, or epoetin kappa [≤9000 IU/week]; DA [≤60 µg/week]; or epoetin beta pegol [≤250 µg/4 weeks]) for at least 8 weeks, had a mean Hb value of ≥9.5 to ≤12.0 g/dL, and had a serum ferritin level of ≥100 ng/mL or transferrin saturation (TSAT) of ≥20%Hb.

### Selection and Withdrawal of Subjects

Subjects are selected for the study based on the following inclusion and exclusion criteria.

### Inclusion Criteria

Subjects deemed eligible for the study included those who:
- Be at least 20 years of age, inclusive;
- Have diagnosis of Chronic Kidney Disease;
- Be receiving hemodialysis or hemodialysis filtration at least three (3) times per week for ≥ 12 weeks before screening;
- Be receiving the same formulation of ESA using the same administration route and the same dose interval (the dose interval should be the one described in the package insert for the ESA formulation) within 8 weeks prior to the first day of the screening period, using the dosages described below:
   Epoetin alfa and epoetin beta and kappa: ≤ 9,000 IU every week
   Darbepoetin alfa: ≤ 60 µg every week
   Epoetin beta pegol: ≤ 250 µg every 4 weeks
- Have a mean of the most recent Hb levels at screening in the range of ≥ 9.5 to ≤ 12.0 g/dL.
- Have a difference of <1.5 g/dL in Hb for the latest two tests in the screening period;
- Have serum ferritin values of ≥100 ng/mL or transferrin saturation of ≥20% during the screening period; and
- Have folic acid and vitamin B12 values above standard minimum values during the screening period.

### Key Exclusion Criteria

Subjects deemed ineligible for inclusion in the study included those who:
- Have anemia due to a cause other than CKD;
- Have active bleeding or blood loss;
- Have received a red blood cell transfusion;
- Have uncontrolled hypertension;
- Have active ocular fundus disease or cannot undergo an ocular fundus examination;
- Have been diagnosed with cardiovascular disease, malignancy, or hemisiderosis;

### Key Efficacy Endpoints

Efficacy endpoints for this study were defined as follows:

### Primary

Average Hb level at weeks 20 and 24 of the treatment period.

### Secondary

Hb level at each treatment visit; and

The proportion of patients within the target Hb range (above: ≥ 12.0 g/dL; within: 10.0-12.0 g/dL; below: < 10.0 g/dL).

### Safety Endpoints

Safety endpoints for this study were defined as follows:
- Adverse events (AEs);
- Adverse drug reactions; and

### Other Efficacy Endpoints

Other endpoints for this study were defined as follows:
- Iron-related parameters: serum ferritin, transferrin saturation (TSAT), total iron-binding capacity (TIBC), hepcidin, serum iron, and monthly dose of iron by any route of administration (oral and intravenous); and
- Red blood cell indices.

### Statistical Analysis

### Primary Efficacy Endpoints

*Noninferiority:* Compound 1 was considered noninferior to Darbepoetin alfa if the 95% confidence interval (CI) lower limit for the difference between Compound 1 and Darbepoetin alfa in the Least Squares (LS) Mean of the average Hb at weeks 20 and 24 was more than or equal to the predefined noninferiority margin of -0.75 g/dL. Difference in LS Mean refers to mixed-model repeated measures (MMRM) with an unstructured covariance matrix within patients.

### Treatment of Subjects

Subjects were randomized 1:1 to either
- Compound **1** with an initial dose of 300 mg/ once daily and adjusted to 150-600 mg/once daily; or
- Darbepoetin alfa (IV) initial dose is as follows:

Dosing information for all subjects is shown in **FIG. 1****.**

For subjects already on darbepoetin, the initial dosing regimen in the study was based on the prior dosing regimen.

For subjects taking other ESAs, the initial dose of darbepoetin was based on the approved local product label.

For all subjects, no additional ESA doses were administered after Screening visit 2 (SV2) and prior to the Randomization visit.

### Dose Adjustment Guidelines

Dosing was initiated at the baseline visit, and the first dose of study medication (Compound **1** or darbepoetin alfa) was administered at the investigative site after other baseline procedures have been completed. For all subjects, no additional ESA doses were administered after SV2 and prior to the Randomization visit. Hemoglobin was monitored via HemoCue^{®} point of care device throughout the study to determine if the dose of study medication (Compound **1** or darbepoetin alfa) needed to be adjusted or suspended.

The aim is to maintain a Hb level of 10.0-12.0 g/dL throughout the study.

The Dose Adjustment Algorithm for Compound **1** and darbepoetin alfa is as follows (see below).

When adjusting therapy, Hb rate of rise, rate of decline, and variability as well as the subject's clinical condition (i.e., recent illness, volume depletion, volume overload, etc.) were considered.

### Dose Adjustment Algorithm:

- Increases in the dose were not allowed to occur more frequently than once every 4 weeks. Decreases in dose were allowed to occur more frequently. Frequent dose adjustments were avoided.
- If the Hb rose rapidly, the dose was reduced.
- If the Hb fell below 10.0 g/dL, the dose was increased.
- If the Hb level exceeded 12.0 g/dL, the dose was reduced.

For subjects randomized to darbepoetin alfa, the initial dose is determined as follows:
- For subjects already on darbepoetin, the initial dosing regimen in the study was based on the prior dosing regimen.
- For subjects taking other ESAs, the initial dose of darbepoetin was based on the approved local product label.

### Dosing Instructions

### Compound 1

All subjects start with an initial dose of 300 mg/day at baseline visit. Dose levels of Compound **1** include 150, 300, 450, and 600 mg. Each subject took his/her first dose of study medication at the investigative site at the baseline visit. Thereafter, study medication was taken once daily on an outpatient basis. Subjects took Compound **1** with or without food. The dose was taken at approximately the same time each day. The subject was instructed to take any oral iron supplements at least 2 hours before or 2 hours after the dose of Compound 1.

### Darbepoetin alfa

Darbepoetin alfa was administered, stored, and dispensed according to the approved local product label.

### Iron Supplementation

Investigators prescribed iron supplementation as needed during the study to maintain ferritin ≥100 ng/mL or TSAT ≥20%. Important: Because of the potential for oral iron to reduce the bioavailability of Compound 1, the study medication was not administered concurrently with an oral iron supplement (including multivitamins containing iron). The subject was instructed to take any oral iron supplements at least 2 hours before or 2 hours after the dose of Compound 1.

### Results

Patient characteristics at baseline are shown in **Table 5.**

**Table 5. Patient Characteristics at Baseline.**

| **Characteristic** | | **VDT (N** = **162)** | | **DA (N = 161)** | |
|---|---|---|---|---|---|
| **Sex (male), n (%)** | | 104 (64.2) | | 109 (67.7) | |
| **Age (years)** | | 66.0 ± 11.3 | | 64.9 ± 11.7 | |
| **Body weight (kg) (dry weight)** | | 58.1 ± 11.9 | | 58.8 ± 13.8 | |
| **BMI (kg/m²)** | | 22.4 ± 3.4 | | 22.4 ± 4.5 | |
| **Hb (g/dL)** | | 10.73 ± 0.7 | | 10.73 ± 0.7 | |
| **Duration of dialysis (years)** | | 7.4 ± 6.7 | | 7.6 ± 7.6 | |
| **Serum ferritin (ng/mL)** | | 144.5 ± 139.6 | | 140.0 ± 95.3 | |
| **TSAT (%)** | | 28.6 ± 10.6 | | 26.9 ± 9.4 | |

| **Prior ESA** | | **n (%)** | **Weekly dose** | **n (%)** | **Weekly dose** |
|---|---|---|---|---|---|
| | **Epoetin (IU)** | 49 (30.2) | 3704 ± 2118 | 53 (32.9) | 4783 ± 3183 |
| | **Darbepoetin alfa (µg)** | 97 (59.9) | 17.2 ± 12.2 | 90 (55.9) | 18.7 ± 14.1 |
| | **Epoetin beta pegol (µg)** | 16 (9.9) | 18.8 ± 12.1 | 18 (11.2) | 22.9 ± 17.4 |
| **Comorbidities, n (%)** | | | | | |
| | **Hypertension** | 152 (93.8) | | 147 (91.3) | |
| | **Diabetes mellitus** | 35 (21.6) | | 49 (30.4) | |
| | **Dyslipidemia** | 59 (36.4) | | 79 (49.1) | |

| | | | | | |
|---|---|---|---|---|---|
| Data are mean ± SD (standard deviation) unless otherwise noted. | | | | | |

### Results

Compound **1** was shown to be effective in maintaining Hb levels within the target Hb range, and no new safety concerns were identified. The findings support the use of Compound **1** for treating anemia in DD-CKD patients who convert from ESA therapy.

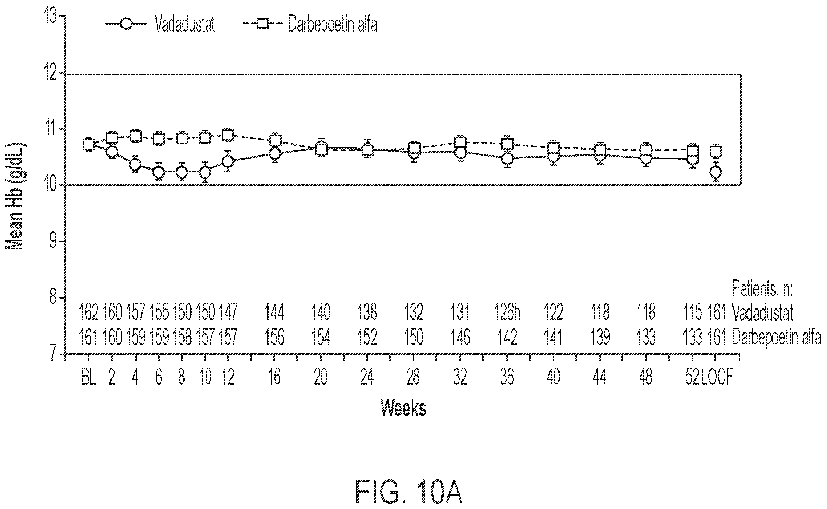

Mean average Hb levels were maintained from baseline to week 52 in both groups (FIG10 A). Indeed, the primary efficacy endpoint was as measured by average Hb at weeks 20 and 24. See (**Table 6**). The 95% CI for both VDT and DA groups at weeks 20-24 was within the target Hb range of 10.0-12.0 g/dL (10.61 and 10.65 g/dL for Compound 1 and darbepoetin alfa, respectively). In the FAS, the 95% CI lower limit was above the predefined noninferiority margin of -0.75 g/dL, confirming the noninferiority of VDT to DA. That is, at week 24 the primary endpoint was met: The difference in mean Hb was -0.05 g/dL (95% CI -0.26, 0.17 g/dL), achieving the pre-specified non-inferiority criterion of -0.75 g/dL.

**Table 6. Mean Change in average Hb at weeks 20-24 in HD-CKD Patients**

| **Treatment group** | **N** | **Average Hb, weeks 20 and 24** | |
|---|---|---|---|
| | | **LSMean*** | **95% CI** |
| VDT | 160 | 10.61 | 10.45, 10.76 |
| DA | 160 | 10.65 | 10.50, 10.80 |
| Difference (VDT-DA) | - | -0.05 | -0.26, 0.17 |

As shown in Table 6, the mean Hb level at week 20 and week 24 at 10.61 g/dL (95% CI 10.45, 10.76 g/dL) for Compound **1**-treated subjects compared to 10.65 g/dL (95% CI 10.50, 10.80 g/dL) for darbepoetin alfa-treated subjects.

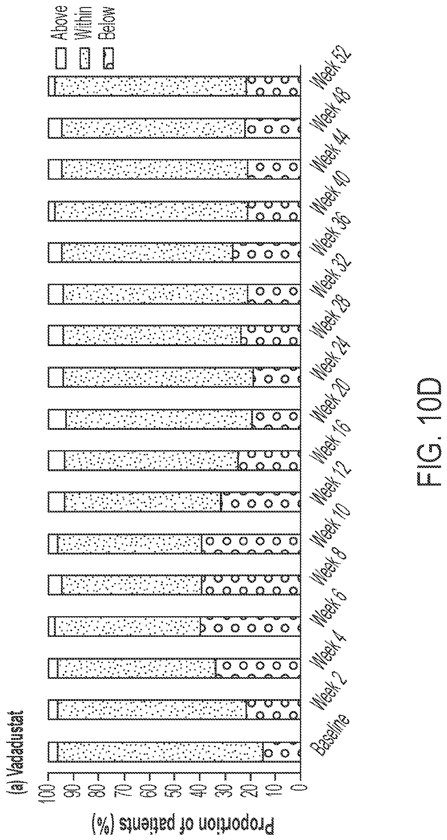

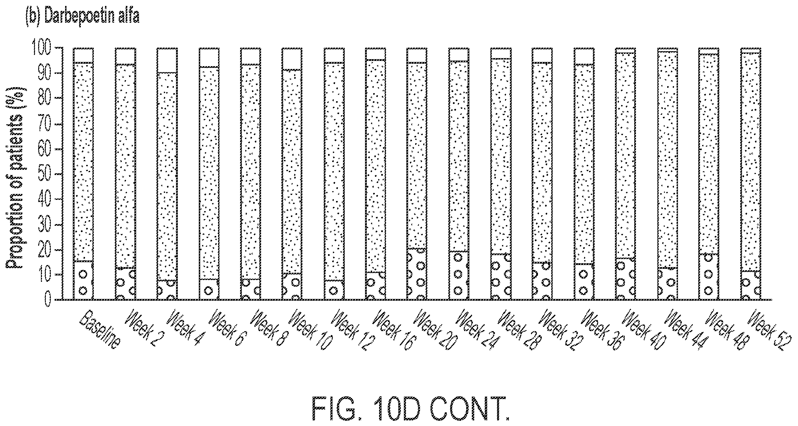

The proportion of patients who exhibited Hb levels within the target range of 10.0-12.0 g/dL at baseline and weeks 24 and 52 was 81.5%, 75.4%, and 75.7% in the Compound **1** group and 78.9%, 75.7%, and 86.5% in the DA group, respectively (**FIG. 10D**). The proportion of patients who exhibited an Hb excursion of ≥12 and ≥13 g/dL was 25.3% and 3.7%, respectively, in the Compound **1** group and 29.8% and 3.1%, respectively, in the DA group. No patients in either group exhibited a rapid rise in Hb levels (>2 g/dL/4 weeks) during the 52-week treatment period.

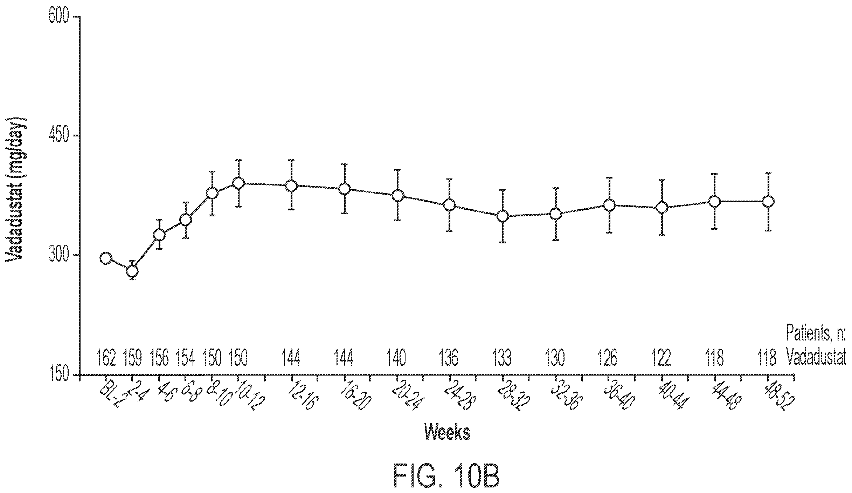

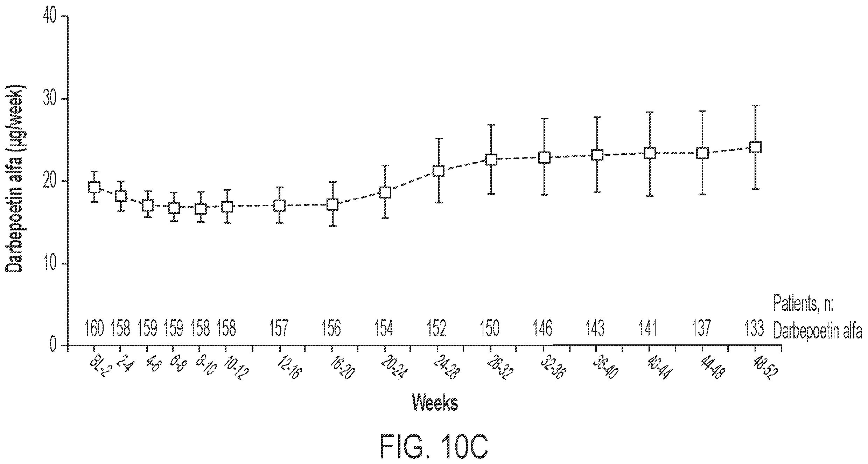

**FIG. 10A** shows the mean Hb over time in HD-CKD patients over 52 weeks who received therapy with Compound **1** (VDT) or darbepoetin alfa (DA). **FIG. 10B** shows the average dose of Compound **1** (VDT) for HD-CKD patients through week 52 of the study. Compound **1** was started at 300 mg/day, and the mean daily doses (95% CI) of Compound 1 was 375.34 (343.45-407.23) mg at weeks 20-24 and 367.65 (331.91-403.39) mg at weeks 48-52. **FIG. 10C** shows the average dose of darbepoetin alfa (DA)for HD-CKD patients through week 52 of the study. The mean weekly doses (95% CI) of DA were 19.31 (17.43-21.19) µg at baseline and week 2, 18.67 (15.43-21.91) µg at weeks 20-24, and 24.15 (19.12-29.18) µg at weeks 48-52.

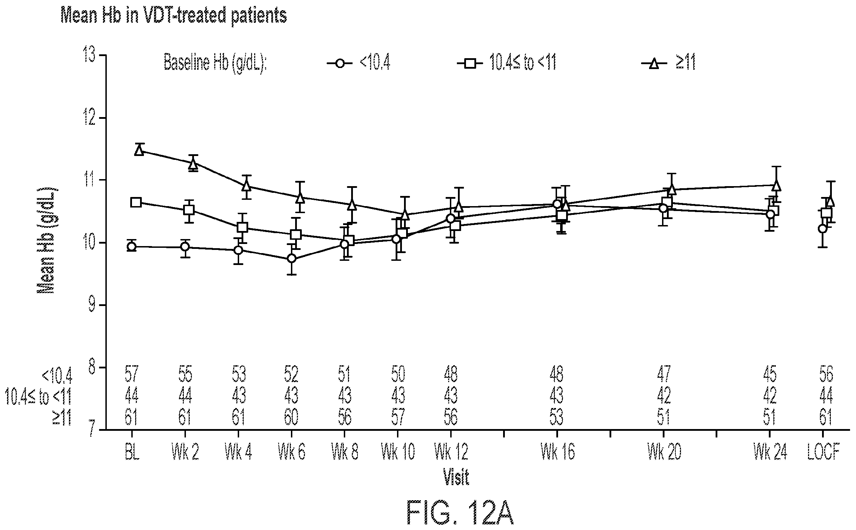

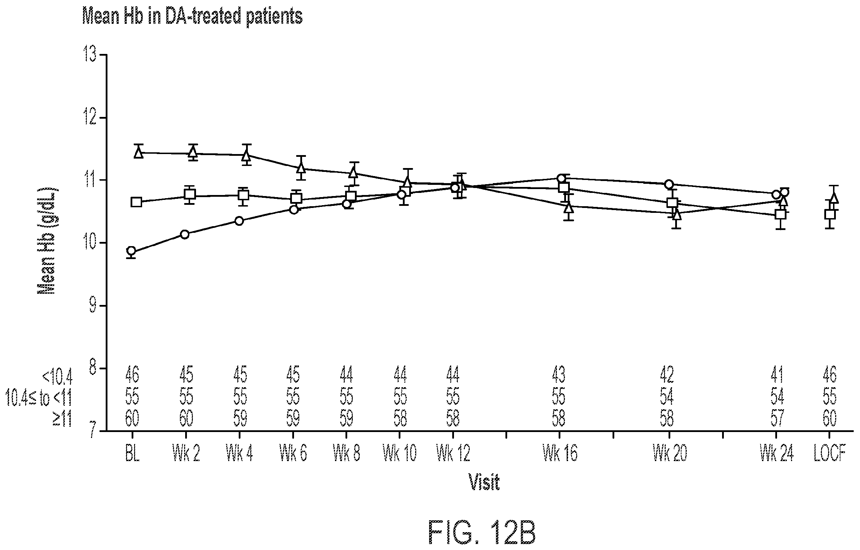

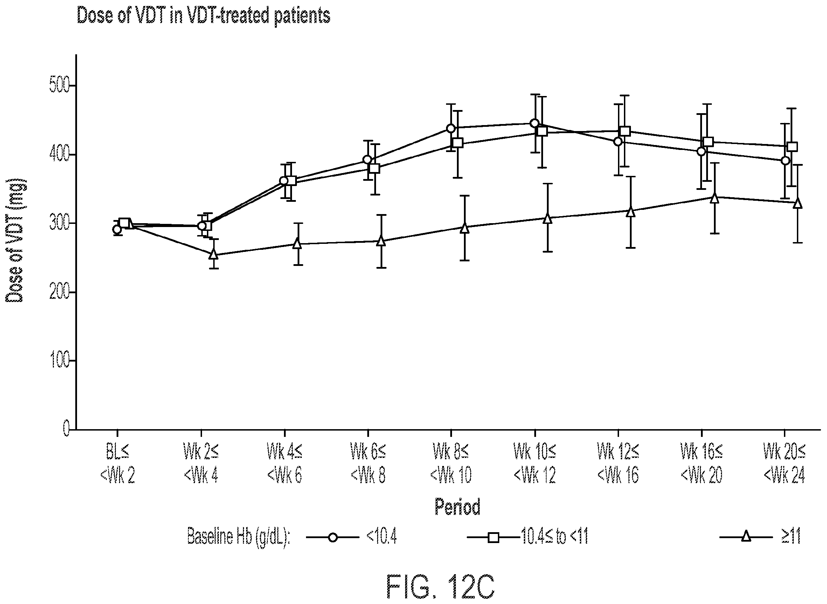

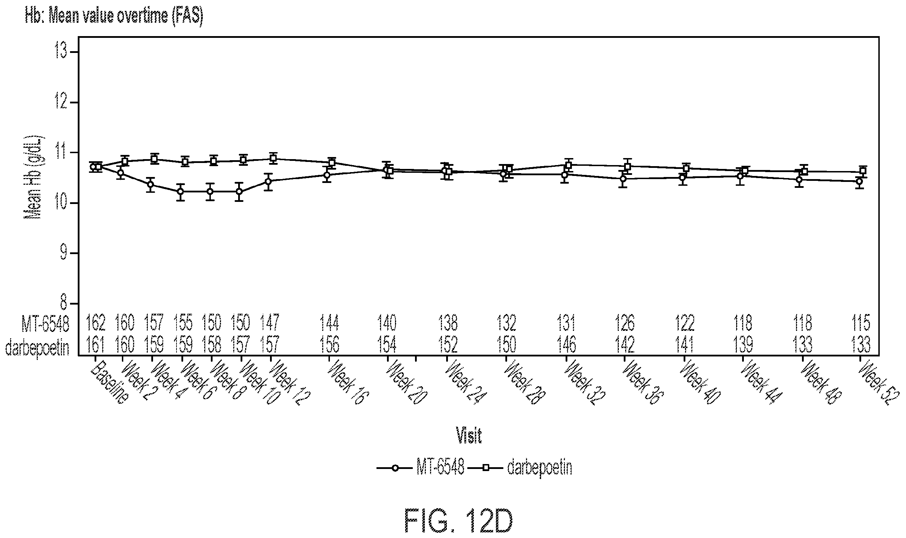

**FIG. 12A** shows the mean Hb by baseline Hb in HD-CKD patients treated with Compound **1** (VDT), **FIG. 12B** shows the mean Hb by baseline Hb in patients treated with HD-CKD patients treated with darbepoetin alfa (DA), and **FIG. 12C** shows the mean dose of Compound **1** (VDT) in HD-CKD patients receiving this therapy. **FIG. 12D** shows the mean Hb value in HD-CKD patients over the 52-week study. Specifically, the mean Hb level at week 52 was 10.39 g/dL (95% CI 10.24, 10.54 g/dL) for vadadustat-treated subjects compared to 10.62 g/dL (95% CI 10.48, 10.76 g/dL) for darbepoetin alfa-treated subjects (**FIG. 12D**).

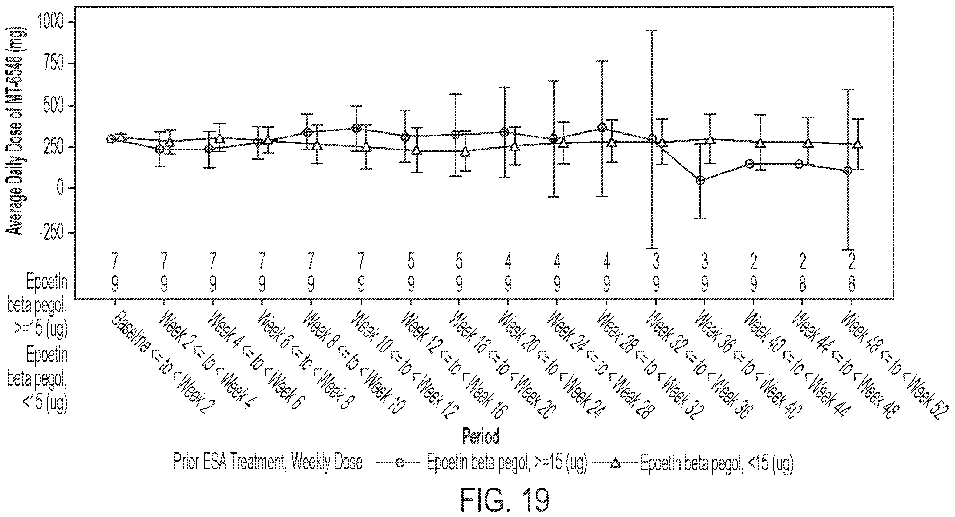

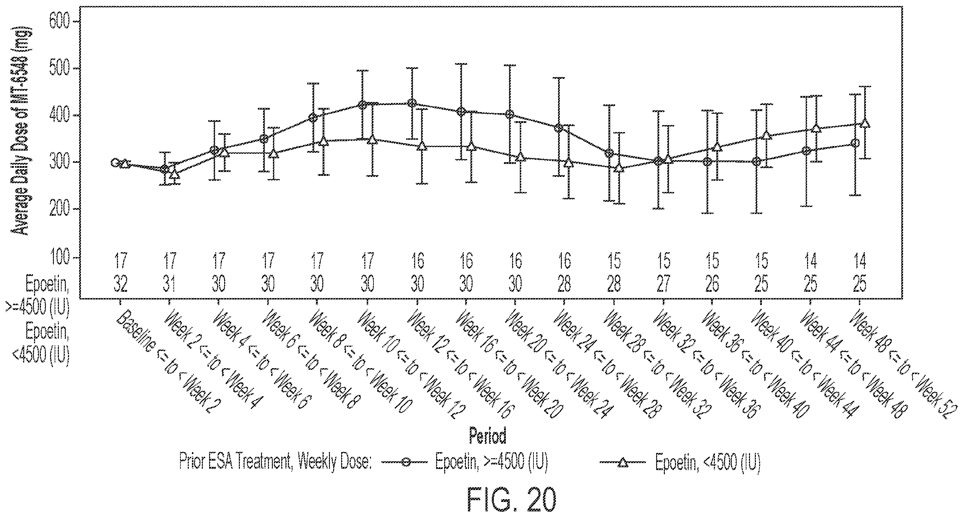

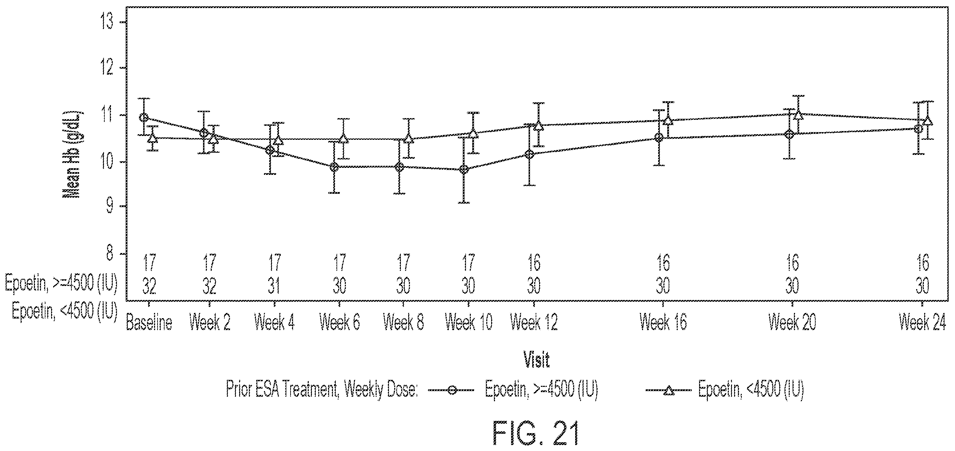

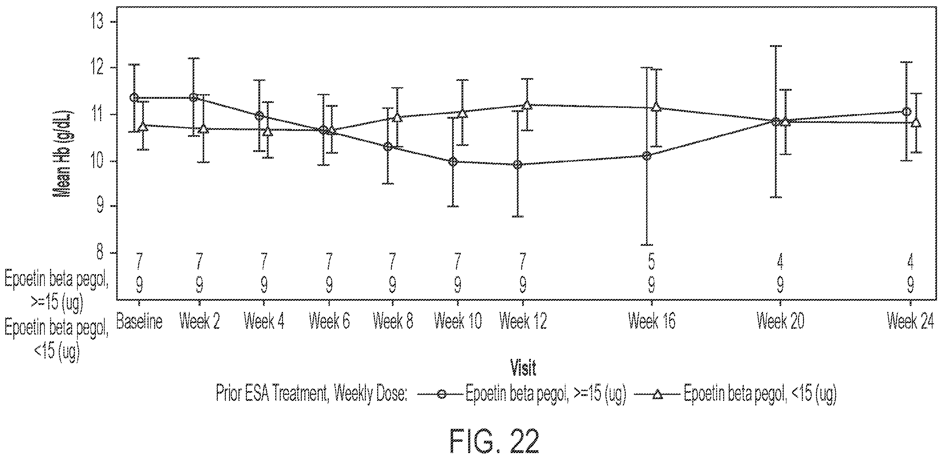

**FIG. 13A** shows the mean Hb in HD-CKD patients receiving Compound **1** (VDT) based on prior treatment with epoetin, darbepoetin alfa (DA), or epoetin beta pegol (EBP). **FIG. 13B** shows the average dose of Compound **1** (VDT) for HD-CKD patients based on prior treatment with epoetin, darbepoetin alfa (DA), or epoetin beta pegol (EBP). **FIG. 13C** shows the mean Hb in HD-CKD patients receiving Compound **1** (VDT) based on the pre-conversion dose of darbepoetin alfa (DA). **FIG. 13D** shows the average dose of Compound **1** (VDT) in HD-CKD patients based on the pre-conversion dose of darbepoetin alfa (DA) up to 24 weeks. **FIG. 13E** shows the average dose of Compound **1** (VDT, MT-6548) in HD-CKD patients based on the pre-conversion dose of darbepoetin alfa (DA) up to 52 weeks. **FIG. 19** shows the mean average dose of Compound **1** (MT-6548) in HD-CKD patients based on the pre-conversion dose of epoetin beta pegol. **FIG. 20** shows the mean average dose of Compound **1** (VDT) in HD-CKD patients based on the pre-conversion dose of epoetin. **FIG. 21** shows the mean Hb in HD-CKD patients receiving Compound **1** (VDT) based on the pre-conversion dose of epoetin. **FIG. 22** shows the mean Hb in HD-CKD patients receiving Compound **1** (VDT) based on the pre-conversion dose of epoetin beta pegol.

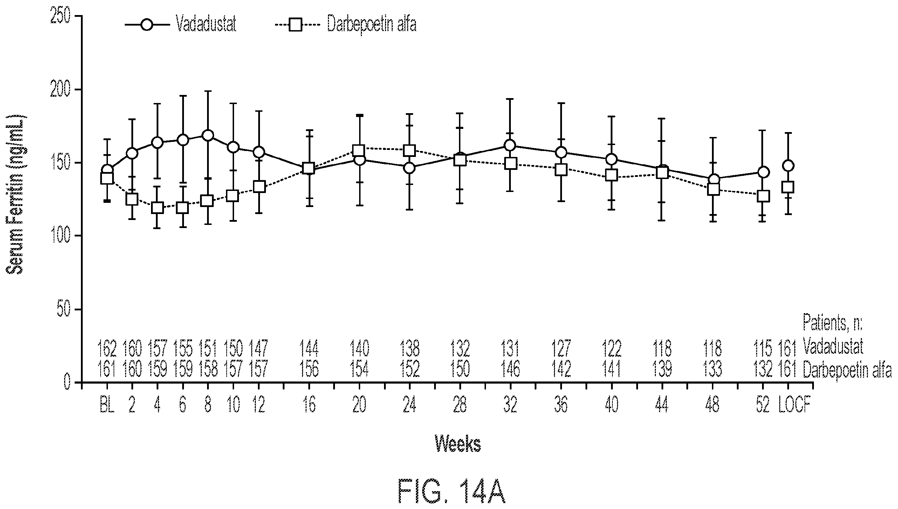

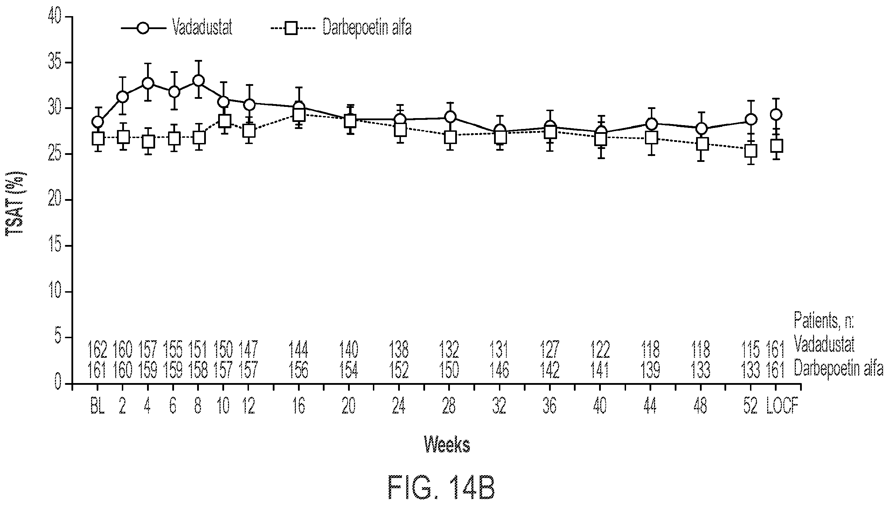

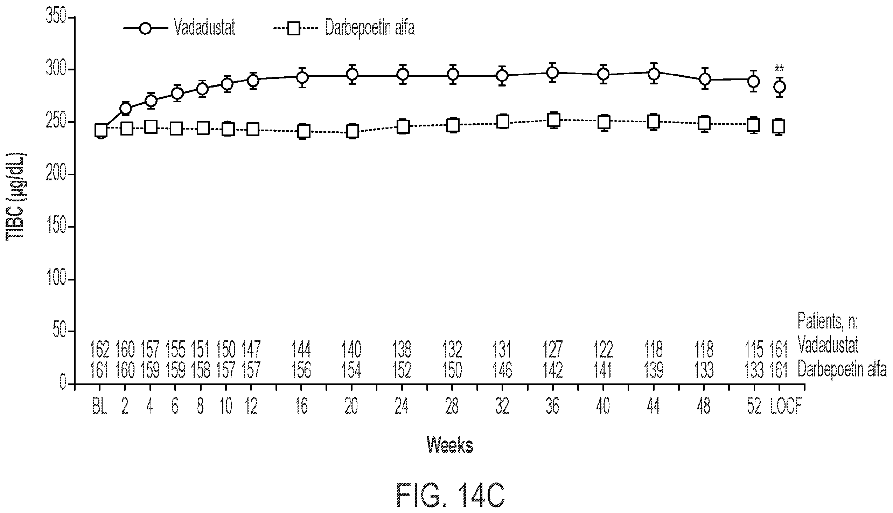

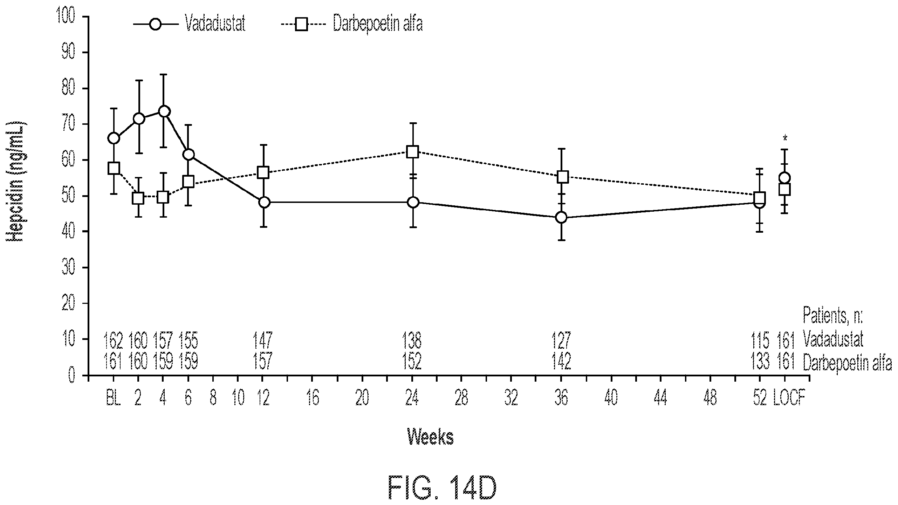

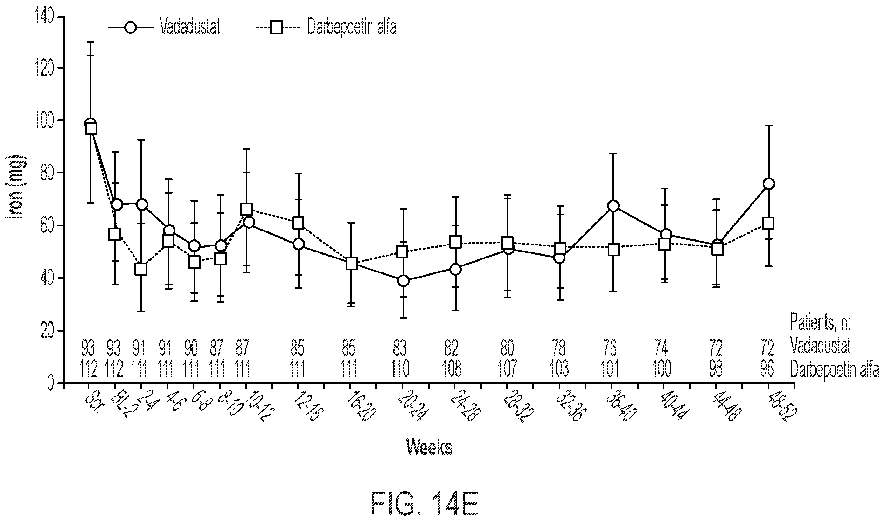

**FIG. 14** compares various iron related parameters for the overall HD-CKD population receiving therapy with Compound **1** and those receiving therapy with darbepoetin alfa (DA), including differences in serum ferritin (ng/mL), TSAT %, TIBC (µg/dL), hepcidin (ng/mL), serum iron (µg/dL), and the monthly dose of iron by any route (mg). There were no significant differences in serum ferritin and TSAT at week 52 (LOCF) compared to baseline in both groups (**FIGS. 14A****,** **14B**). The mean total iron-binding capacity (TIBC) was significantly increased from baseline to week 52 (LOCF) in the Compound 1 group, whereas it remained almost stable in the DA group (**FIG. 14C**). The mean hepcidin level significantly decreased from baseline at week 52 (LOCF) in the Compound 1 group, whereas it remained unchanged in the DA group (**FIG. 14D**). No significant differences were noted in the mean monthly dose of intravenous iron from the screening period to weeks 48-52 in both groups (**FIG.14E**). The proportion of patients receiving intravenous iron during the screening period, and weeks 48-52 was 26.5%, and 30.9%, respectively, in the Compound 1 group and 30.4%, and 33.3%, respectively, in the DA group. The proportion of patients receiving oral iron during the screening period, and weeks 48-52 was 4.3%, and 3.3%, respectively, in the VDT group and 1.2%, and 2.2%, respectively, in the DA group.

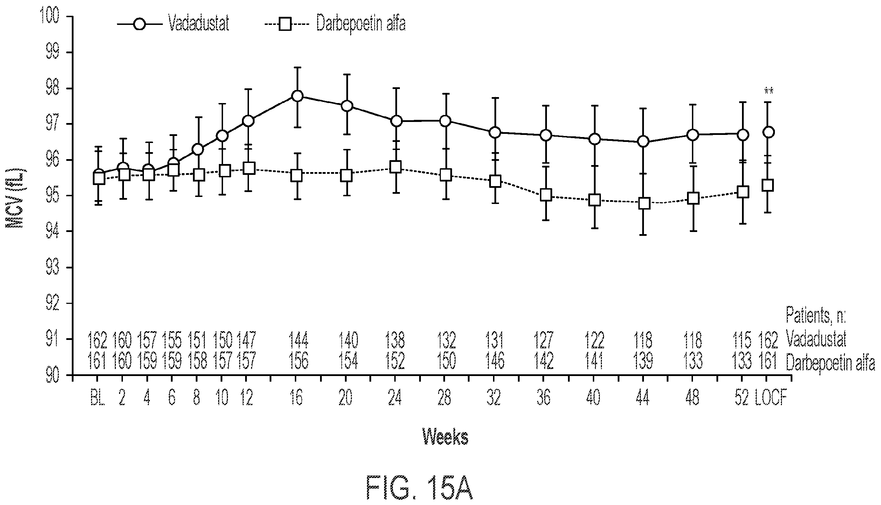

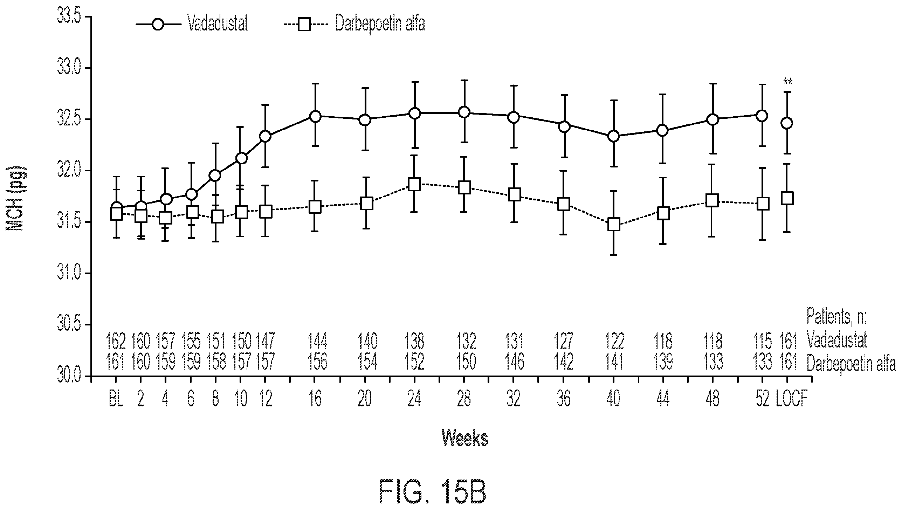

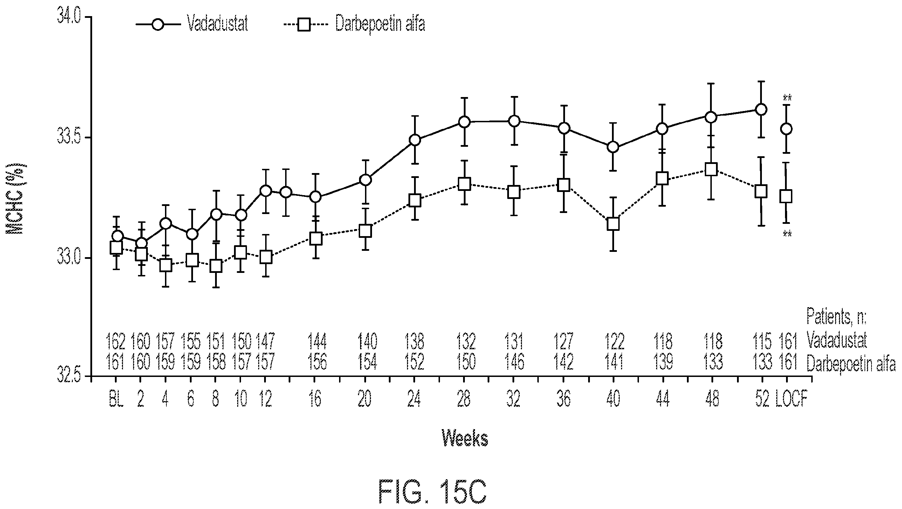

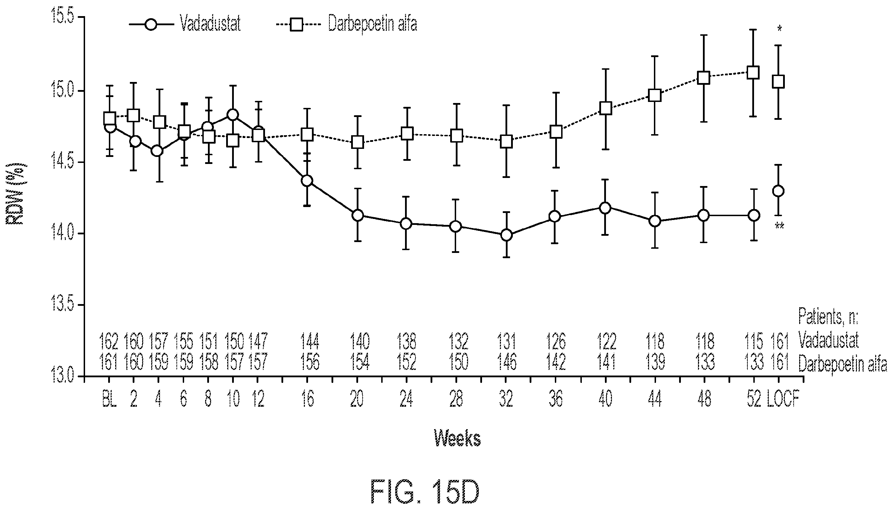

**FIG. 15** compares red blood cell indices MCH (mean corpuscular hemoglobin), MCHC (mean corpuscular hemoglobin concentration), MCV (mean corpuscular volume); and RDW (red cell distribution width) for Compound **1** and DA patients up to 52 weeks. The changes in red blood cell parameters are shown in **FIGS. 15A-D.** The mean MCV and MCH levels significantly increased from baseline at week 52 (LOCF) only in the Compound 1 group (**FIG. 15A****,** **15B**), whereas the mean MCHC level significantly increased from baseline at week 52 in both groups (**FIG. 15C**). For the mean RDW significantly decreased from baseline at week 52 (LOCF) in the Compound 1 group, whereas it significantly increased at week 52 in the DA group (**FIG. 15D**).

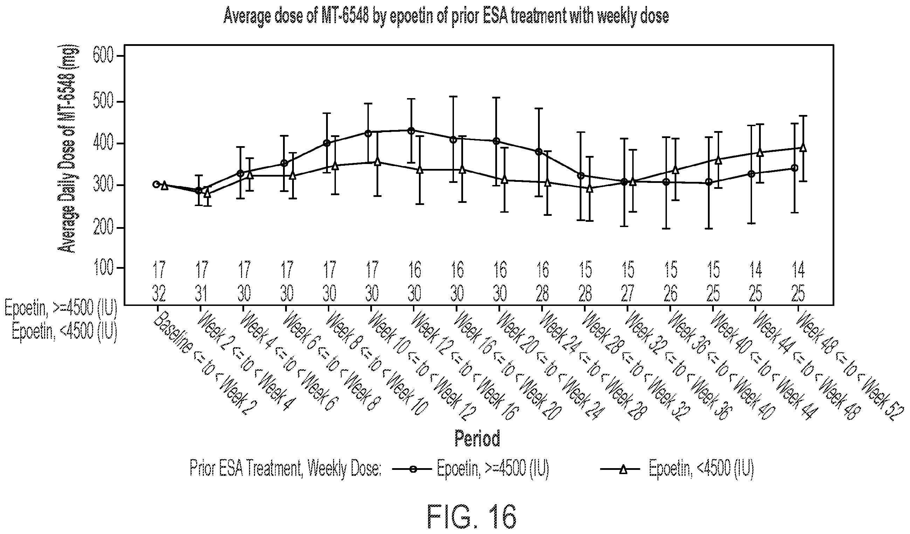

**FIG. 16** shows the average dose of Compound **1** in HD-CKD conversion subgroups based on the weekly dose of epoetin previously received by the patient.

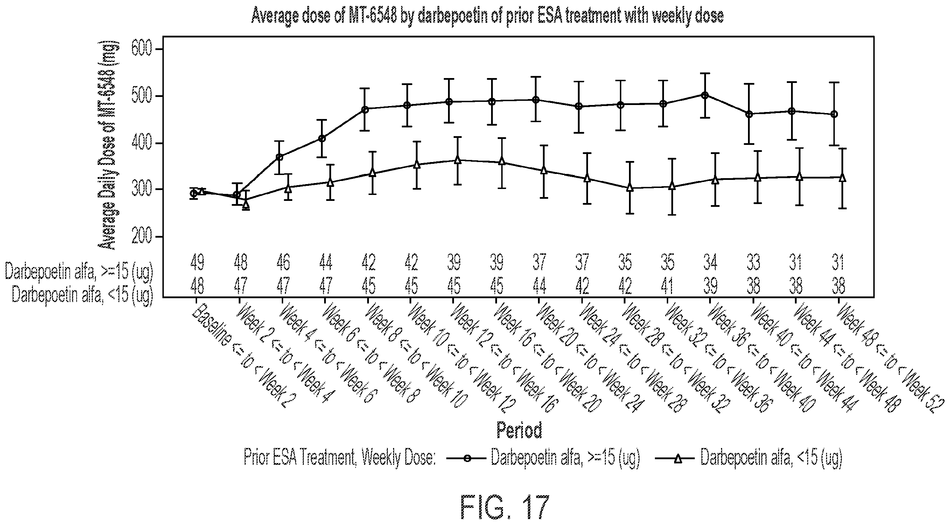

**FIG. 17** shows the average dose of Compound **1** in HD-CKD conversion subgroups based on the weekly dose of darbepoetin alfa (DA) previously received by the patient. Over the course of the 52-week study, patients who received a weekly dose of DA ≥ 15 µg received higher average doses of Compound **1** (*e.g*., after week 2 of the study).

These data show that Compound **1** is effective for treating anemia in conversion of HD-CKD patients who previously received ESA therapy. Moreover, higher doses of Compound **1** may be particularly beneficial for HD-CKD conversion patients who have received higher doses of darbepoetin alfa (DA) or an epoetin (*e.g*., epoetin alfa, epoetin beta, or epoetin gamma): for example, such patients can benefit from, e.g., doses of 450 or 600 mg of Compound **1.** Additionally, therapy using Compound **1** has shown to be effective (*e.g*., in maintaining target Hb levels) and durable over the course of the 52-week study period.

*Safety Assessment.* Safety results at 52 weeks are provided in **Table 7.** During the treatment period up to 52 weeks, adverse events of thromboembolism such as cerebral infarction, myocardial infarction, and pulmonary embolism were similar between the Compound 1 groups and the control group (Darbepoetin alfa group), and no adverse drug reactions of thromboembolism were observed. Additionally, no meaningful imbalance between groups was observed in the proportion of patients who reported AEs of special interest (cardiovascular event, cardiac failure, retinal disorder, malignancy, hyperkalemia, pulmonary hypertension).

**Table 7. Adverse Events**

| **Overview, n (%)** | **VDT (N = 162)** | **DA (N = 161)** |
|---|---|---|
| Subjects with ≥1 AE | 154 (95.1) | 158 (98.1) |
| Adverse drug reaction | 18 (11.1) | 6 (3.7) |
| Serious AEs | 41 (25.3) | 44 (27.3) |
| Serious adverse drug reaction | 0 (0.0) | 0 (0.0) |
| Discontinuation due to AEs | 16 (9.9) | 14 (8.7) |
| Dose reduction or interruption of study drug due to AEs | 13 (8.0) | 4 (2.5) |
| Deaths due to AEs | 2 (1.2) | 1 (0.6) |

| **Most common AEs with VDT, n (%)** | **VDT (N = 162)** | **DA (N = 161)** |
|---|---|---|
| Nasopharyngitis | 74 (45.7) | 73 (45.3) |
| Diarrhea | 25 (15.4) | 24 (14.9) |
| Shunt stenosis | 23 (14.2) | 26 (16.1) |

| **AEs of special interest, n (%)** | **VDT (N = 162)** | **DA (N = 161)** |
|---|---|---|
| Cardiovascular event, cardiac failure | 13 (8.0) | 15 (9.3) |
| Retinal disorder | 21 (13.0) | 16 (9.9) |
| Malignancy | 7 (4.3) | 9 (5.6) |
| Hyperkalemia | 1 (0.6) | 1 (0.6) |
| Pulmonary hypertension | 0 (0.0) | 0 (0.0) |

As shown in Table 7, the incidence of AEs was 95.1% in the Compound **1**-treated group compared to 98.1% in the darbepoetin alfa-treated group. The top three most common AEs reported in vadadustat-treated subjects were nasopharyngitis (45.7%), diarrhea (15.4%), and shunt stenosis (14.2%). The incidence of serious AEs was 25.3% in the Compound **1**-treated group compared to 27.3% in the darbepoetin alfa-treated group; no serious AE was considered related to study drug. There were two deaths reported in the Compound **1**-treated group and one death in the darbepoetin alfa-treated group and all three were assessed as not related to either Compound **1** or darbepoetin alpha.

### Subgroup Analysis

Subgroup analysis based on the patient background (e.g., etiology of CKD, comorbidities, duration of anemia, duration of dialysis, prior ESA therapy, and baseline C-reactive protein levels) revealed that the LSMean Hb levels at week 52 were maintained within the target range of 10.0-12.0 g/dL irrespective of the patient backgrounds by Compound 1 dose adjustment (150-600 mg/day) (**Table 8**)**.**

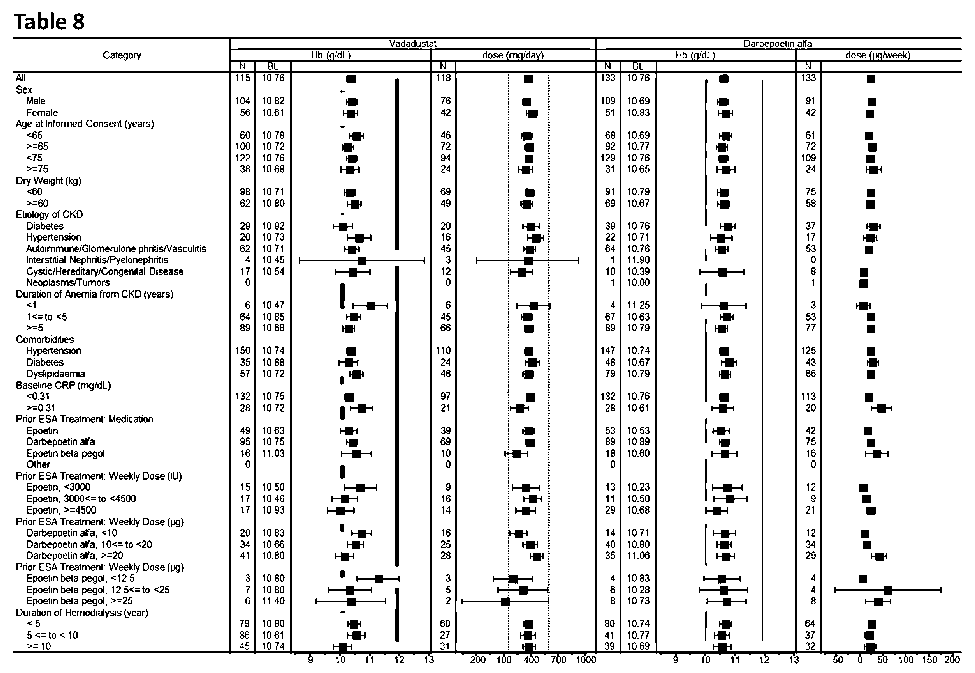

### Discussion

This 52-week, phase 3 study confirmed the effectiveness of Compound 1 (VDT) in maintaining Hb levels within the target range in ESA-converted patients with anemia on hemodialysis and demonstrated the noninferiority of the oral VDT to DA for the treatment of anemia in these patients. In the VDT group, Hb levels decreased transiently in the early treatment period after switching from ESA. This may be due to the fact that the initial VDT dose was set to avoid Hb level overshoot in ESA-converted patients and to the dose adjustment algorithm requiring that Hb levels should be <10.0 g/dL to increase the VDT dose with an interval of ≥4 weeks. However, when the VDT dose was adjusted according to the dose adjustment algorithm, approximately 80% of the patients could be maintained within the target Hb range at weeks 24 and 52, similar to that at baseline. The extent was similar to that observed with DA.

The effects of Compound 1 (VDT) on iron parameters including increased TIBC and decreased serum hepcidin levels in patients receiving hemodialysis in this study, which was not seen in the DA group, were consistent with those observed in the previous study of VDT in anemia patients undergoing hemodialysis (1).

The most common AEs in the Compound 1 (VDT) group were nasopharyngitis, diarrhea, and shunt stenosis, which were similar to those observed in the DA group. The AEs of special interest, including cardiovascular event/cardiac failure, retinal disorder, malignancy, hyperkalemia, and thrombotic events, also occurred similarly in the VDT and DA groups. Retinal hemorrhage was reported more often in the VDT group than in the DA group (9.9% vs. 6.2%, respectively); however, all these events were mild and the causality with the study drug was not considered reasonable. Furthermore, no significant changes in blood VEGF levels were observed in patients with retinal hemorrhage. Ophthalmoscopy of these patients did not show difference in the rate of progression compared with the screening period. Hyperkalemia and arteriovenous fistula thrombosis occurred more frequently with other HIF-PHI than with the control (epoetin alfa) (2); however, pathophysiological mechanism by which HIF-PHIs may cause these events is unclear, and no higher risk of these has been noted in our study. Nonetheless, the AEs of special interest need to be further investigated across the HIF-PHI class.

The factors, including patient backgrounds that may affect the efficacy of HIF-PHIs, have not been understood. The present study showed that adjusting the Compound 1 (VDT) with a dose of 150-600 mg/day could maintain Hb levels within the target range, irrespective of patient background as shown in **Table 8.**

In conclusion, in a Phase 3 study in patients with anemia on hemodialysis who were previously receiving ESAs, oral Compound 1 (VDT) was non-inferior to DA in maintaining Hb levels at weeks 20 and 24. The mean Hb level was maintained within the target range for up to 52 weeks in both groups. Compound 1 (VDT) was well tolerated, and no new safety concerns were identified. These findings suggest that VDT is useful for the treatment of anemia in patients on hemodialysis converting from ESA therapy.

### References

1. Nangaku M, Youssef F, deGoma E, et al.: Vadadustat, an oral hypoxia-inducible factor prolyl hydroxylase inhibitor, for the treatment of anemia of chronic kidney disease: Two randomized phase 2 trial in Japanese patients. Nephrol Dial Transplant, 2020, in press.
2. Chen N, Hao C, Liu BC, Lin H, Wang C, Xing C, Liang X, Jiang G, Liu Z, Li X, Zuo L, Luo L, Wang J, Zhao MH, Liu Z, Cai GY, Hao L, Leong R, Wang C, Liu C, Neff T, Szczech L, Yu KHP: Roxadustat Treatment for Anemia in Patients Undergoing Long-Term Dialysis. N Engl J Med 381: 1011-1022, 2019.

### Example 3: Phase 2 Clinical Trial to Evaluation Compound 1 (Vadadustat) in Patients Receiving Hemodialysis Previously Treated with Erythropoiesis-Stimulation Agents (ESA)

In this open-label Phase 2 trial, Compound 1 was evaluated in 94 subjects receiving hemodialysis, previously maintained on epoetin alfa. Subjects were sequentially assigned to one of three Compound 1 dose cohorts by starting dose: 300 mg once daily (QD), 450 mg QD or 450 mg thrice weekly (TIW). The primary endpoint was mean hemoglobin (Hb) change from pre-baseline average to midtrial (Weeks 7-8) and end-of-trial (Weeks 15-16) and was analyzed using available data (no imputation).

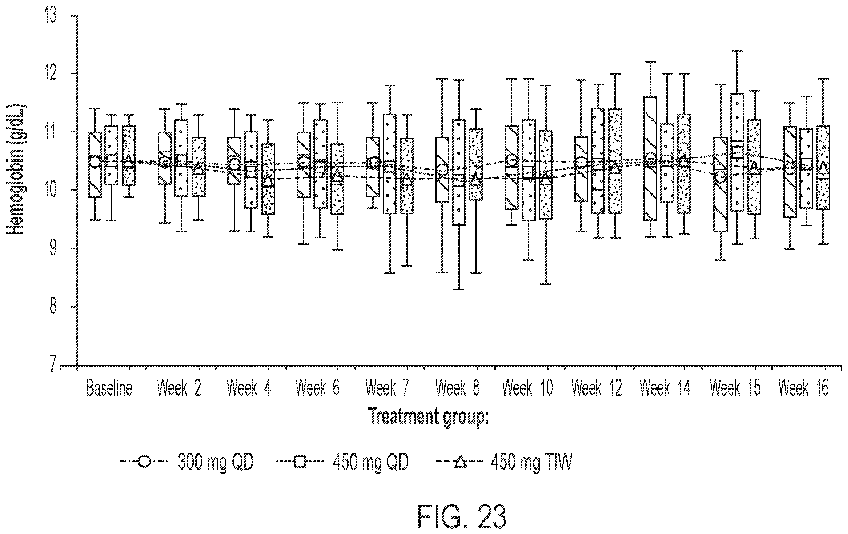

*Results.* Overall, 80, 73 and 68% of subjects in the 300 mg QD, 450 mg QD, and 450 mg TIW dose cohorts respectively, completed the study. For all dose cohorts no statistically significant mean change in Hb from pre-baseline average was observed (**FIG. 23**), and mean Hb concentrations analyzed using available data remained stable at mid- and end-of-trial. There was one subject with an Hb excursion>13 g/dL. Overall, 83% of subjects experienced an adverse event (AE); the proportion of subjects who experienced at least one AE was similar among the three dose cohorts. The most frequently reported AEs were nausea (11.7%), diarrhea (10.6%) and vomiting (9.6%). No deaths occurred during the study. No serious AEs were attributed to vadadustat.

*Conclusions.* Compound maintained mean Hb concentrations in subjects on hemodialysis previously receiving epoetin. These data support further investigation of vadadustat to assess its long-term safety and efficacy in subjects on hemodialysis.

### Example 4: Phase 3 Clinical Trials to Evaluate Compound 1 (Vadadustat or VDT or VADA) for the Treatment of Anemia in Patients with Dialysis-Dependent Chronic Kidney Disease (DD-CKD)

Two randomized Phase 3, open-label, sponsor-blind, active-controlled non-inferiority (NI) trials were conducted in anemic DD-CKD patients with incident dialysis and limited ESA exposure (Correction/Conversion trial) and with prevalent dialysis and established ESA treatment (Conversion trial). The primary safety endpoint was prespecified as the time to first major adverse cardiovascular event (MACE) (all-cause mortality, non-fatal MI, or non-fatal stroke) pooled across the two trials. Primary and key secondary efficacy endpoints were mean change in Hb from baseline [BL] to wks 24-36 and to wks 40-52 between two treatment arms, respectively.

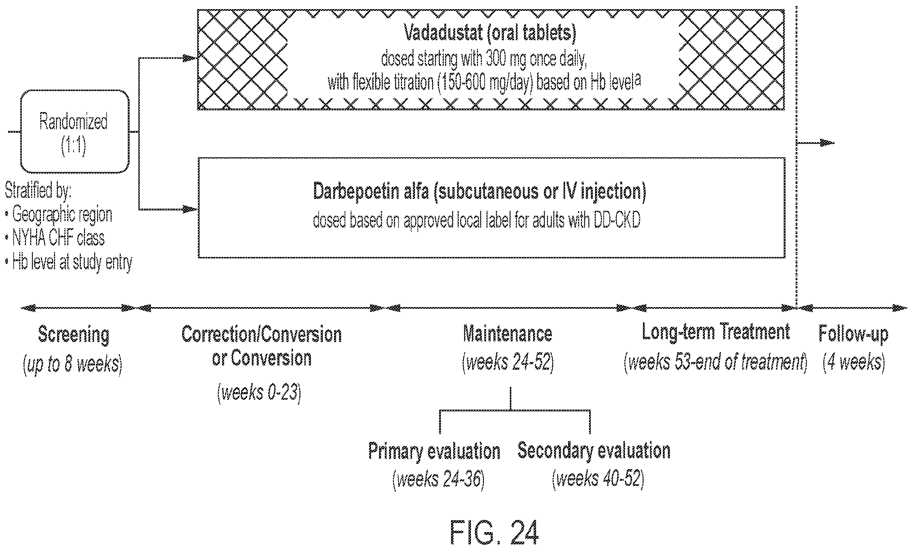

### Study Design Overview for Incident DD-CKD/Correction/Conversion (Corr/Conv) Trial (FIG. 24):

Phase 3, randomized, open-label, active-controlled study of the efficacy and safety of vadadustat versus darbepoetin alfa for the maintenance treatment of anemia after the correction of hemoglobin (Hb) or conversion from current ESA therapy in subjects who have recently initiated dialysis treatment for DD-CKD. Following a Screening period of up to 8 weeks (56 days), subjects who met all inclusion and none of the exclusion criteria were randomized 1:1 to vadadustat or darbepoetin alfa.

Randomization was stratified by:
- Geographic region (United States [US] versus European Union [EU] versus Rest of World [ROW]).
- New York Heart Association congestive heart failure (CHF) Class 0 (no CHF) or I versus II or III.
- Study entry hemoglobin (Hb) level (<9.5 or ≥9.5 g/dL).

Following randomization, there were 4 periods during the study:
- Correction/Conversion Period (Weeks 0 to 23): initial period on study medication.
- Maintenance Period (Weeks 24 to 52): period on study medication during which efficacy was assessed (primary evaluation period: Weeks 24 to 36; secondary evaluation period: Weeks 40 to 52).
- Long-term Treatment Period (Weeks 53 to End of Treatment [EOT]): continued study medication to assess long-term safety.
- Follow-up (EOT + 4 weeks): post-treatment visit (either in person or via telephone) for safety.

*Inclusion Criteria*: Subjects met the following criteria:
- ≥ 18 years of age.
- Initiated chronic maintenance dialysis (either peritoneal or hemodialysis) for end-stage kidney disease within 16 weeks prior to Screening.
- Mean screening hemoglobin (Hb) between 8.0 and 11.0 g/dL (inclusive), as determined by the average of 2 Hb values measured by the central laboratory during Screening.
- Serum ferritin ≥ 100 ng/mL and transferrin saturation (TSAT) ≥20% at Screening.
- Folate and vitamin B12 measurements ≥ lower limit of normal at Screening.
- Understands the procedures and requirements of the study and provides written informed consent and authorization for protected health information disclosure.

*Exclusion Criteria*: Subjects were excluded if they met any of the following criteria:
- Anemia due to a cause other than CKD or subjects with active bleeding or recent blood loss.
- Subjects with sickle cell disease, myelodysplastic syndromes, bone marrow fibrosis, hematologic malignancy, myeloma, hemolytic anemia, thalassemia, or pure red cell aplasia.
- Red blood cell (RBC) transfusion within 8 weeks prior to randomization.
- Anticipated to recover adequate kidney function to no longer require dialysis.
- Aspartate aminotransferase (AST)/serum glutamic oxaloacetic transaminase (SGOT), alanine aminotransferase (ALT)/serum glutamic pyruvic transaminase (SGPT), or total bilirubin >2.0 x upper limit of normal (ULN) at or during Screening. Subjects with a history of Gilbert's syndrome are not excluded.
- Uncontrolled hypertension (defined as confirmed predialysis systolic blood pressure [BP] >190 mmHg or diastolic BP >110 mmHg at rest) at or during Screening.
- Severe heart failure at or during Screening (New York Heart Association Class IV).
- Acute coronary syndrome (hospitalization for unstable angina, myocardial infarction); surgical or percutaneous intervention for coronary, cerebrovascular, or peripheral artery disease (aortic or lower extremity); surgical or percutaneous valvular replacement or repair; sustained ventricular tachycardia; hospitalization for CHF; or stroke within 12 weeks prior to or during Screening.
- History of active malignancy within 2 years prior to or during Screening, except for treated basal cell carcinoma of skin, curatively resected squamous cell carcinoma of skin, or cervical carcinoma in situ.
- History of deep vein thrombosis (DVT) or pulmonary embolism (PE) within 12 weeks prior to randomization.
- History of hemosiderosis or hemochromatosis.
- History of prior organ transplantation or scheduled organ transplant (subjects on the kidney transplant wait-list or with a history of failed kidney transplant are not excluded), or prior hematopoietic stem cell or bone marrow transplant (corneal transplants and stem cell therapy for knee arthritis are not excluded).
- Hypersensitivity to vadadustat, darbepoetin alfa, or any of their excipients.
- Use of an investigational medication or participation in an investigational study within 30 days or 5 half-lives of the investigational medication (whichever is longer), prior to or during Screening.
- Previous participation in this study or previous participation in a study with another hypoxia-inducible factor prolyl-hydroxylase inhibitor (HIF-PHI) other than vadadustat.
- Females who are pregnant or breastfeeding. Women of childbearing potential who are unable or unwilling to use an acceptable method of contraception.
- Non-vasectomized male subjects who are unable or unwilling to use an acceptable method of contraception.
- Any other reason, which in the opinion of the Investigator, would make the subject not suitable for participation in the study.
- Subjects meeting the following ESA within 8 weeks prior to or during Screening:
   ∘ epoetin >7700 units/dose three times per week or >23,000 units per week;
   ∘ darbepoetin alfa:>100 mcg/week;
   ∘ methoxy polyethylene glycol-epoetin beta: >100 mcg every other week or >200 mcg/month.
   *Study Design Overview for Prevalent DD-CKD*/*Conversion (Conv) Trial (**FIG. 24**):*

Phase 3, randomized, open-label, active-controlled study of vadadustat vs darbepoetin alfa for the maintenance treatment of anemia after conversion from ESA therapy.

Following a Screening period of up to 8 weeks (56 days), subjects who met all inclusion and none of the exclusion criteria were randomized 1:1 to vadadustat or darbepoetin alfa.

Randomization was stratified by:
- Geographic region (United States [US] versus European Union [EU] versus Rest of World [ROW])
- New York Heart Association congestive heart failure (CHF) Class 0 (no CHF) or I versus II or III.
- Study entry Hb (<10.0 versus ≥10.0 g/dL).

Following randomization, there were 3 periods during the study:
- Conversion and Maintenance Period (Weeks 0 to 52): conversion to study medication for maintaining Hb (Weeks 0-23), primary efficacy evaluation (Weeks 24-36), and secondary efficacy evaluation (Weeks 40-52).
- Long-term Treatment Period (Weeks 53 to End of Treatment [EOT]): continued study medication to assess long-term safety.
- Follow-up (EOT + 4 weeks): post-treatment visit (either in person or via telephone) for safety.

*Inclusion Criteria:*
- ≥18 years of age.
- Receiving chronic maintenance dialysis (either peritoneal or hemodialysis) for end-stage kidney disease for at least 12 weeks prior to Screening.
- Currently maintained on ESA therapy, with a dose received within 6 weeks prior to or during Screening.
- Mean screening Hb between 8.0 and 11.0 g/dL (inclusive) in the US and between 9.0 and 12.0 g/dL (inclusive) outside of the US, as determined by the average of 2 Hb values measured by the central laboratory during Screening.
- Serum ferritin ≥100 ng/mL and transferrin saturation (TSAT) ≥20% during Screening.
- Folate and vitamin B12 measurements ≥ lower limit of normal during Screening.
- Understands the procedures and requirements of the study and provides written informed consent and authorization for protected health information disclosure.

*Exclusion Criteria:*
- Anemia due to a cause other than CKD or subjects with active bleeding or recent blood loss.
- History of sickle cell disease, myelodysplastic syndromes, bone marrow fibrosis, hematologic malignancy, myeloma, hemolytic anemia, thalassemia, or pure red cell aplasia.
- Red blood cell (RBC) transfusion within 8 weeks prior to randomization.
- Anticipated to recover adequate kidney function to no longer require dialysis.
- Aspartate aminotransferase (AST)/serum glutamic oxaloacetic transaminase (SGOT), alanine aminotransferase (ALT)/serum glutamic pyruvic transaminase (SGPT), or total bilirubin >2.0 x upper limit of normal (ULN) during Screening. Subjects with a history of Gilbert's syndrome are not excluded.
- Uncontrolled hypertension (defined as confirmed predialysis systolic blood pressure [BP] >190 mmHg or diastolic BP >110 mmHg at rest) during Screening.
- Severe heart failure (HF) during Screening (New York Heart Association Class IV).
- Acute coronary syndrome (hospitalization for unstable angina or myocardial infarction), surgical or percutaneous intervention for coronary, cerebrovascular or peripheral artery disease (aortic or lower extremity), surgical or percutaneous valvular replacement or repair, sustained ventricular tachycardia, hospitalization for HF, or stroke within 12 weeks prior to or during Screening.
- History of active malignancy within 2 years prior to or during Screening, except for treated basal cell carcinoma of skin, curatively resected squamous cell carcinoma of skin, or cervical carcinoma in situ.
- History of deep vein thrombosis (DVT) or pulmonary embolism (PE) within 12 weeks prior to randomization.
- History of hemosiderosis or hemochromatosis.
- History of prior organ transplantation or scheduled organ transplant (subjects on the kidney transplant wait-list or with a history of failed kidney transplant are not excluded), or prior hematopoietic stem cell or bone marrow transplant (corneal transplants and stem cell therapy for knee arthritis are not excluded).
- Hypersensitivity to vadadustat, darbepoetin alfa, or any of their excipients.
- Use of an investigational medication or participation in an investigational study within 30 days or 5 half-lives of the investigational medication (whichever is longer), prior to Screening.
- Previous participation in this study or previous participation in a study with another hypoxia-inducible factor prolyl-hydroxylase inhibitor (HIF-PHI) other than vadadustat.
- Females who are pregnant or breastfeeding. Women of childbearing potential who are unable or unwilling to use an acceptable method of contraception.
- Non-vasectomized male subjects who are unable or unwilling to use an acceptable method of contraception.
- Any other reason, which in the opinion of the Investigator, would make the subject not suitable for participation in the study.

### Efficacy Endpoints

*Primary Efficacy Endpoints:*
- Mean change in Hb between Baseline (mean pretreatment Hb) and the primary evaluation period (mean Hb from Weeks 24 to 36).

*Primary Efficacy Endpoint Analysis:* The primary analysis used an analysis of covariance (ANCOVA) with multiple imputations, stratified by the randomization strata and using Baseline Hb as the covariate. A 2-sided, 95% confidence interval (CI) was calculated for the difference between the vadadustat group and control group. Noninferiority of vadadustat was established if the lower limit of this CI is ≥ -0.75 g/dL.

*Key Secondary Efficacy Endpoints:*
- Mean change in Hb value between Baseline (mean pretreatment Hb) and the secondary evaluation period (Weeks 40 to 52).
- Proportion of subjects with Hb value within the target range during the primary evaluation period (Weeks 24 to 36).
- Proportion of subjects with Hb values within the target range during the secondary evaluation period (Weeks 40 to 52).

*Other Secondary Efficacy Endpoints*:
- Proportion of time with Hb values within the target range during the primary evaluation period (Weeks 24 to 36).
- Proportion of time with Hb values within the target range during the secondary evaluation period (Weeks 40 to 52).
- Proportion of subjects with Hb increase of >1.0 g/dL from Baseline.
- Time to achieve Hb increase of >1.0 g/dL from Baseline.
- Mean change in Hb between Baseline (mean pretreatment Hb) and the primary evaluation period (mean Hb from Weeks 24-36) stratified by pre-Baseline ESA exposure.
- Proportion of subjects receiving IV iron therapy from Baseline to Week 52.
- Mean monthly dose of IV elemental iron administered from Baseline to Week 52 in subjects who have received IV iron.
- Proportion of subjects receiving RBC transfusion(s) from Baseline to Week 52.
- ESA rescue.
- Dose adjustments from Baseline to Week 52.

### Safety Endpoints:

- MACE, defined as all-cause mortality, non-fatal myocardial infarction (MI), or non-fatal stroke. Individual components of MACE:
   ∘ All-cause mortality
   ∘ Non-fatal myocardial infarction
   ∘ Non-fatal stroke.
- Thromboembolic events: arterial thrombosis, DVT, PE, or vascular access thrombosis.
- Hospitalization for heart failure (HF).
- Expanded MACE defined as all-cause mortality, non-fatal myocardial infarction, non-fatal stroke, hospitalization for HF, or thromboembolic event.
- Fatal/non-fatal MI.
- Fatal/non-fatal stroke.
- Sudden death.
- Cardiovascular death.
- Non-cardiovascular death.
- Hospitalization.
- Hb >12.0 g/dL, >13.0 g/dL, or >14.0 g/dL.
- Hb < 8.0 g/dL.
- Hb increase >1.0 g/dL within any 2-week interval or >2.0 g/dL within any 4-week interval.
- Adverse events (AEs) and serious AEs (SAEs).
- Vital sign measurements and clinical laboratory values.
- Time to achieve stable Hb values within target range.
- Proportion of subjects with Hb values in the target range without evidence of iron overload.

### Dosing Regimens

Subjects were randomized 1:1 to either:
- Compound 1 (vadadustat) starting dose: 2 tablets once daily (300 mg/day). Darbepoetin alfa IV/SC starting dose: based on the current Package Insert (PI) for investigational sites in the US, and the European Summary of Product Characteristics (SmPC) for all other investigational sites (non-US) for adult patients with CKD on dialysis.
   ∘ For subjects already on darbepoetin alfa, the initial dosing regimen in the study was based on the prior dosing regimen.

For all subjects, it was recommended that no additional ESA doses be administered after Screening visit 2 (SV2) and prior to the Randomization visit.

The starting dose and the proposed dosing algorithm in these studies were designed to increase to or maintain Hb in a predictable and controlled manner while minimizing abrupt increases or excessive rises in Hb levels. Based on plasma concentrations and PD measures from previously conducted clinical studies with vadadustat, a population PK/PD model was developed. Using this model and the dosing proposed dosing algorithm, simulations were carried out to evaluate the effects of different starting doses and the resulting Hb responses to support the dosing rationale. Results of the simulations indicated that a starting dose regimen of 300 mg once daily along with the proposed dosing algorithm were optimal to increase to or maintain Hb levels of 10.0 to 11.0 g/dL in the US and 10.0 to 12.0 g/dL outside of the US while minimizing excessive rises.

*Dose Adjustment Guidelines:* Dosing was initiated at the Baseline visit, and the first dose of study medication (Compound 1 or darbepoetin alfa) was administered at the investigative site after other Baseline procedures were completed. The Investigator may elect to postpone the initial dose of study medication until a subsequent visit based on the subject's Hb level or Hb trajectory assessed at the Baseline visit, or based on timing of the last ESA dose given during screening.

Hemoglobin was monitored via HemoCue^{®} point of care device throughout the study to determine if the dose of study medication (vadadustat or darbepoetin alfa) should be adjusted, interrupted or maintained.

*Year 1-4 Treatment Period Visits:* From Weeks 0 to 12, Hb was measured via HemoCue every 2 weeks for monitoring for dose adjustment. From Week 12 to Week 52, Hb via HemoCue was monitored every 4 weeks, unless more frequent monitoring was clinically indicated or warranted based on dosing changes. From Week 53 through the end of the study, Hb was continued to be monitored via HemoCue to determine if the dose of study medication should be adjusted, interrupted, or maintained. Hemoglobin was assessed with a complete blood count (CBC) through the central laboratory for efficacy and safety evaluations; however, dose adjustments should be based on the local HemoCue Hb value. If the Investigator has an immediate clinical concern about a subject's HemoCue value, the Investigator may use clinical judgement and repeat the HemoCue Hb, use local lab values or wait for central lab results. The test method utilized to inform the treatment decision must be recorded in the appropriate CRF and the subject's source.

*Year 2-4 Monthly Hb Monitoring:* Additionally, after Week 52, the Hb drawn as part of the local standard of care labs must be monitored monthly for dosing oversight. Per the vadadustat or darbepoetin alfa dosing algorithms, if the Hb value suggests a dose adjustment is needed, an unscheduled visit must be performed. If monthly standard of care labs is not available, a study unscheduled visit must be performed. This visit will include, at minimum, the Hb measurement via HemoCue, dose adjustment assessment, and adverse events assessment. The monthly Hb monitoring method is flexible between study visits after Week 52 to minimize unnecessary travel or redundant blood sampling for the subject.

The aim was to increase to or maintain a Hb level of 10.0 to 11.0 g/dL in the US and 10.0 to 12.0 g/dL outside the US throughout the study.

Dose adjustments were guided by Hb concentration and the Dose Adjustment Algorithms. The Dose Adjustment Algorithm for darbepoetin alfa follows the Package Insert (PI) for investigational sites in the US, and the European Summary of Product Characteristics (SmPC) for all other investigational sites (non-US) for adult patients with CKD on dialysis.

This protocol provides guidance for the treatment of subjects with anemia associated with CKD in order to achieve and maintain Hb levels within the target Hb range. Dose adjustment should be based on the Investigator's clinical discretion, incorporating the protocol guidance and considering the subject's clinical condition, Hb rate of rise, Hb rate of decline, Hb variability and ESA responsiveness.

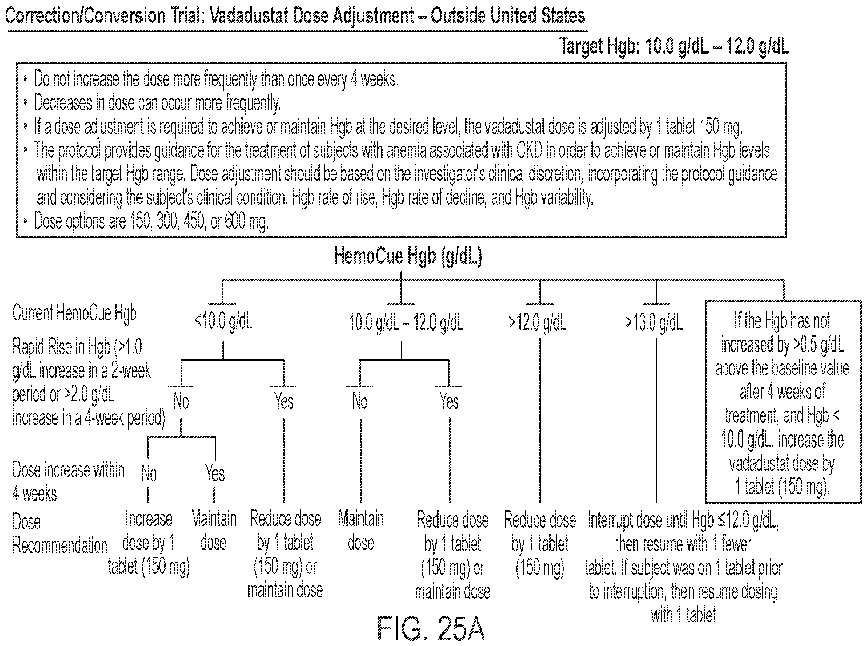

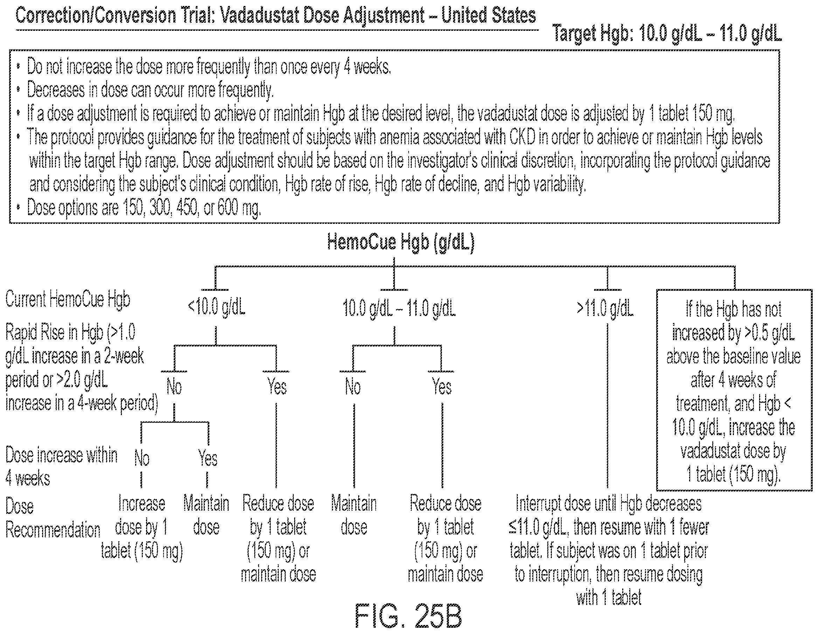

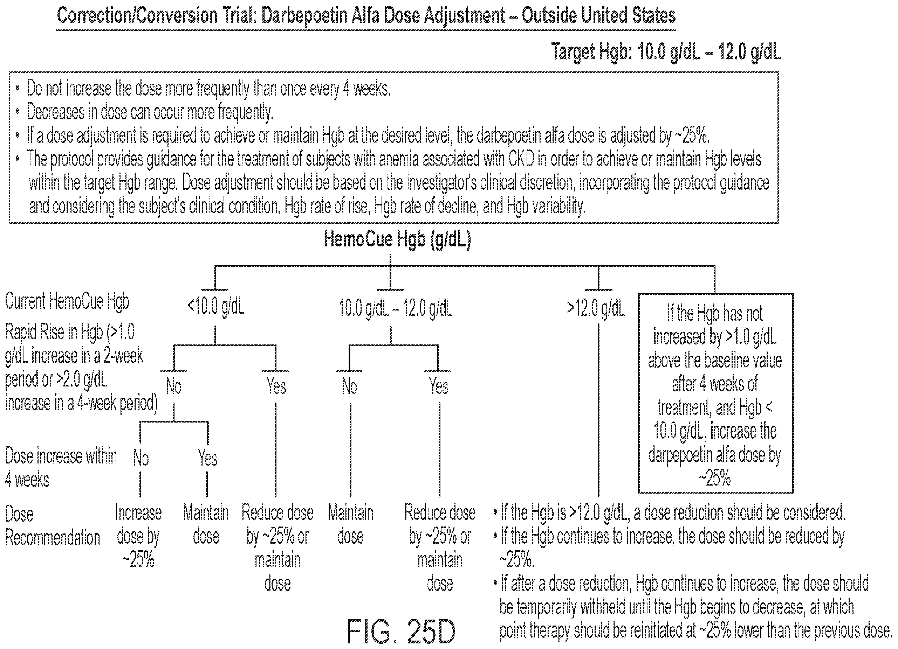

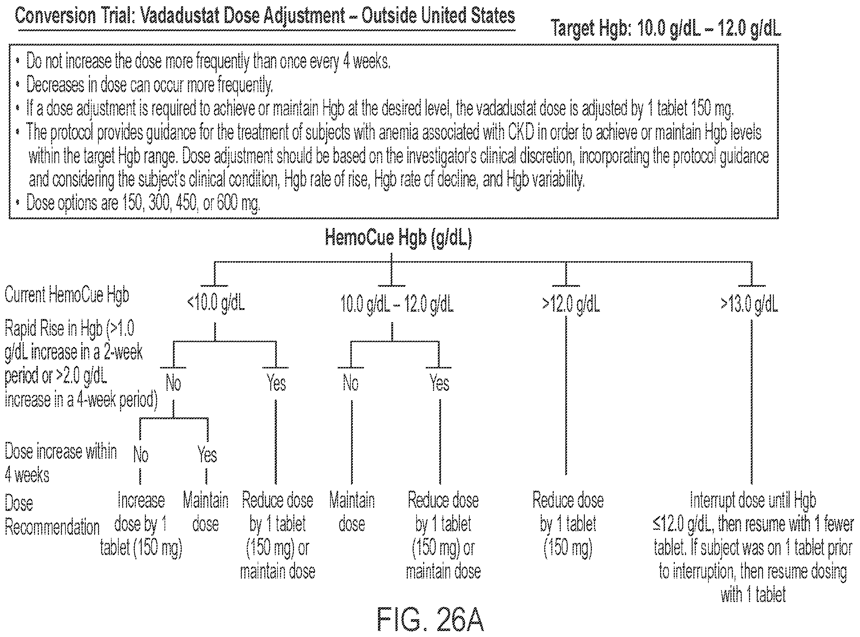

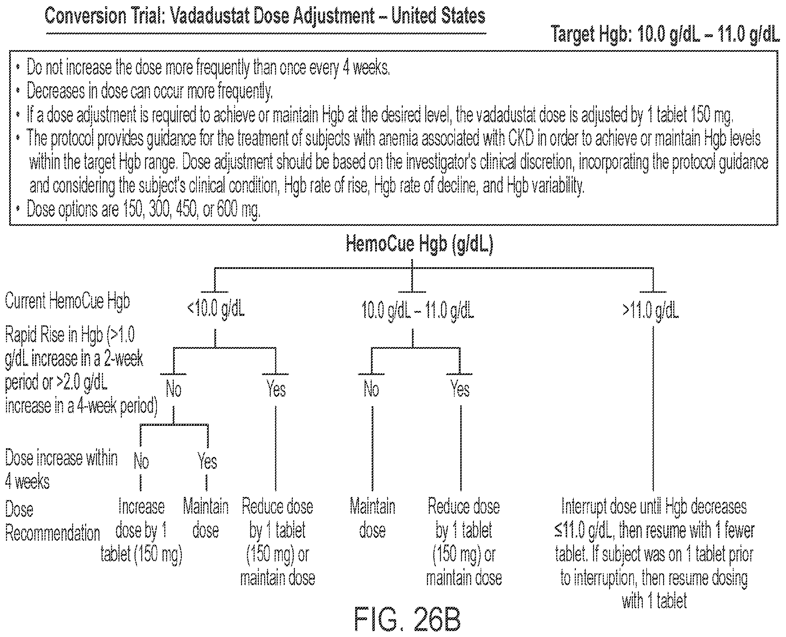

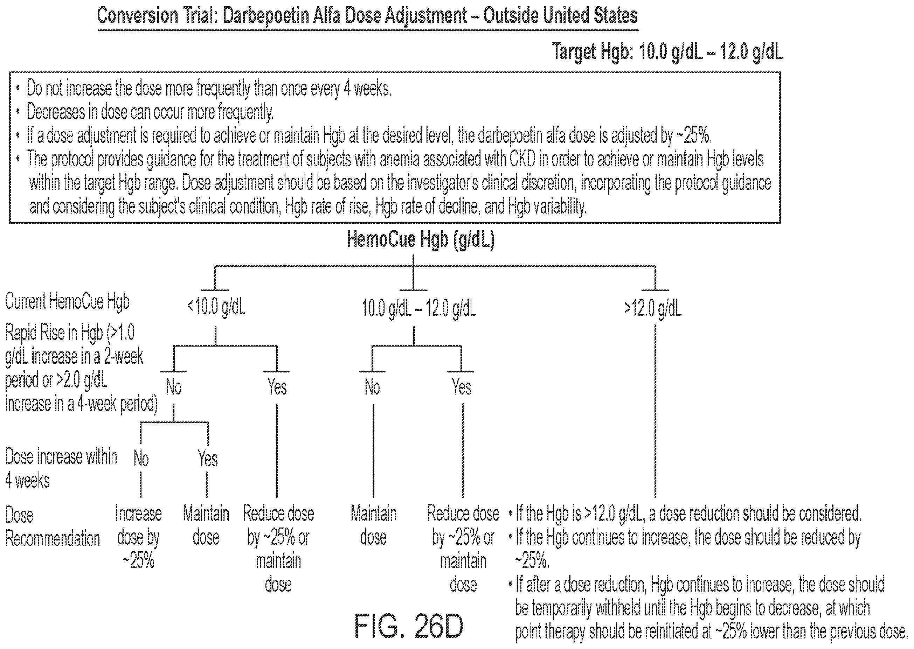

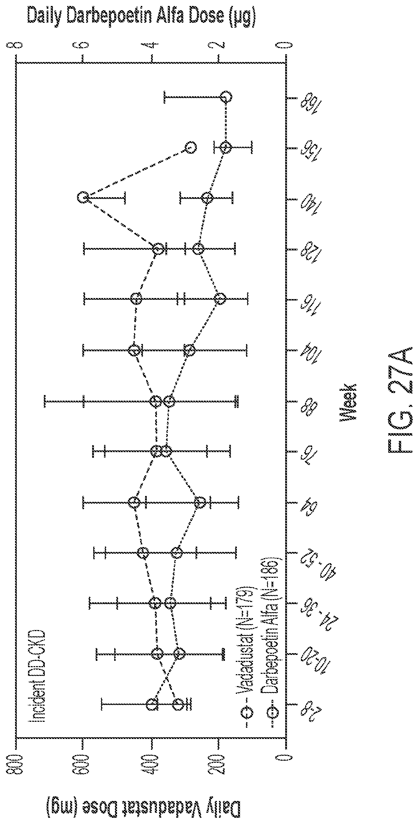

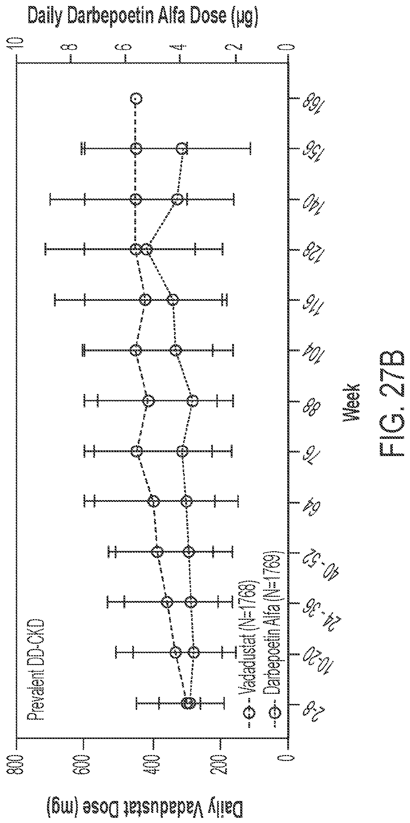

*Compound 1 Dosing:* Compound 1 (vadadustat) was dosed according to the Dose Adjustment Algorithms in **FIGS. 25A-25B****, 26A-26B.** All subjects started with 2 tablets daily (300 mg/day). Dose levels of vadadustat included 150, 300, 450, and 600 mg (available tablet strength is 150 mg) (**FIG. 27A-27B****).** Each subject took his/her first dose of vadadustat at the investigative site at the Baseline visit. Thereafter, vadadustat were taken once daily on an outpatient basis. Vadadustat was taken with or without food. The full dose should be taken at approximately the same time each day. The subjects were instructed to take any oral iron supplements (including multivitamins containing iron), iron containing phosphate binders, or any medications containing iron at least 2 hours before or 2 hours after the dose of vadadustat.

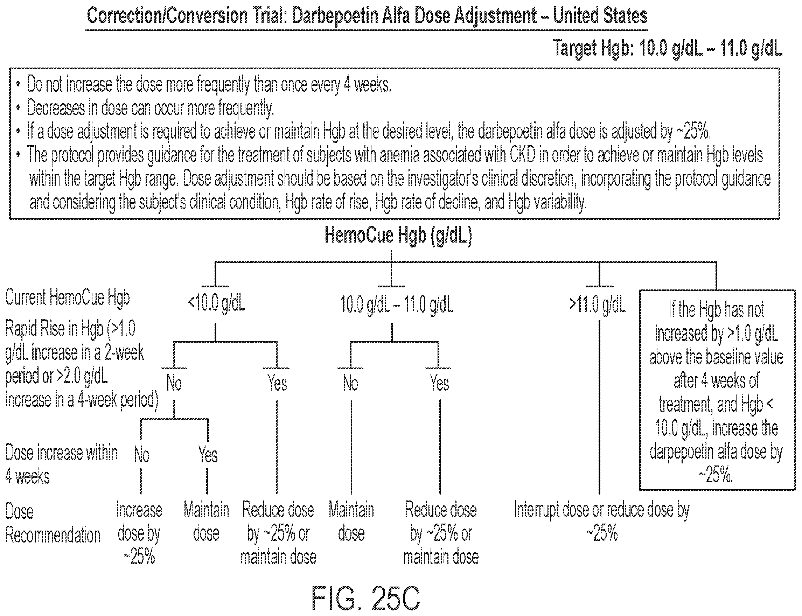

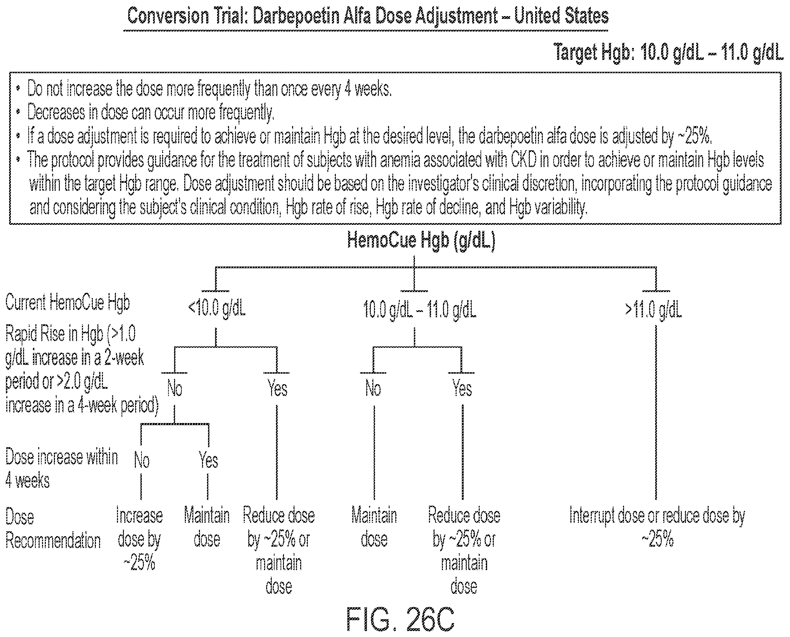

*Darbepoetin Alfa Dosing:* Darbepoetin alfa (DA) was dosed according to the Dose Adjustment Algorithms in **FIGS. 25C-25D****, 26C-26D.** Subsequent administration of darbepoetin alfa may occur at the clinic/investigative site or may be self-administered at home per regional standard-of-care and/or based on dialysis modality (hemodialysis or peritoneal dialysis). Darbepoetin alfa dosing is independent of the visit schedule, and the dosing schedule may shift per local standard-of-care, the patient's dialysis schedule, and per Investigator discretion. Darbepoetin alfa was administered, stored, and dispensed according to the Dosing Algorithms, and Package Insert (PI) for investigational sites in the US, and the European Summary of Product Characteristics (SmPC) for all other investigational sites (non-US) for adult patients with CKD on dialysis.

*Iron Supplementation:* Iron supplementation (IV, oral, or intradialytic) was prescribed during the study to maintain ferritin ≥100 ng/mL or TSAT ≥20%. Subjects already receiving oral iron supplementation as part of their treatment plan continued their current treatment regimen. For subjects who were on peritoneal dialysis, oral iron supplementation was allowed as per local guidelines and routine practice. Because of the potential for oral iron to reduce the bioavailability of vadadustat, the study medication was not to be administered concurrently with an oral iron supplement (including multivitamins containing iron), iron containing phosphate binders, or any medications containing iron. Subjects were instructed to take these medications at least 2 hours before or 2 hours after the dose of vadadustat.

### Results

3923 patients were randomized 1:1 to Compound 1 (VADA) or darbepoetin alpha (DA) (Corr/Conv, N=369; Conv, N=3554). See **Table 9** for baseline demographics.

**Table 9**

| | | **Incident DD-CKD** | | **Prevalent DD-CKD** | |
|---|---|---|---|---|---|
| | | Vadadustat | Darbepoetin alfa | Vadadustat | Darbepoetin alfa |
| **Characteristic** | | (N=181) | (N=188) | (N=1777) | (N=1777) |
| Age, y, mean (SD) | | 56.5 (14.8) | 55.6 (14.6) | 57.9 (13.9) | 58.4 (13.8) |
| Male, no. (%) | | 107 (59.1) | 113 (60.1) | 990 (55.7) | 1004 (56.5) |
| Time since dialysis initiated, mean (SD), y | | 0.14 (0.09) | 0.15 (0.28) | 4.00 (4.02) | 3.94 (4.01) |

| **Disease history, n (%)** | | | | | |
|---|---|---|---|---|---|
| | Diabetes mellitus | 105 (58.0) | 96 (51.1) | 971 (54.6) | 998 (56.2) |
| | Cardiovascular disease | 69 (38.1) | 73 (38.8) | 868 (48.8) | 932 (52.4) |

| **Hemoglobin** | | | | | |
|---|---|---|---|---|---|
| | Mean (SD), g/dL | 9.4 (1.1) | 9.2 (1.1) | 10.6 (0.9) | 10.2 (0.8) |

| **Distribution, no. (%)** | | | | | |
|---|---|---|---|---|---|
| | <9.5 g/dL | 94 (51.9) | 99 (52.7) | N/A | N/A |
| | ≥9.5 g/dL | 87 (48.1) | 89 (47.3) | N/A | N/A |
| | <10.0 g/L | N/A | N/A | 620 (34.9) | 619 (34.8) |
| | ≥10.0 g/dL | N/A | N/A | 1157 (65.1) | 1158 (65.2) |

| **Baseline iron use, no. (%)** | | | | | |
|---|---|---|---|---|---|
| Patients not receiving any iron | | 52 (28.7) | 56 (29.8) | 660 (37.1) | 721 (40.6) |
| Patients receiving IV iron only | | 92 (50.8) | 110 (58.5) | 911 (51.3) | 853 (48.0) |

### Efficacy Endpoints.

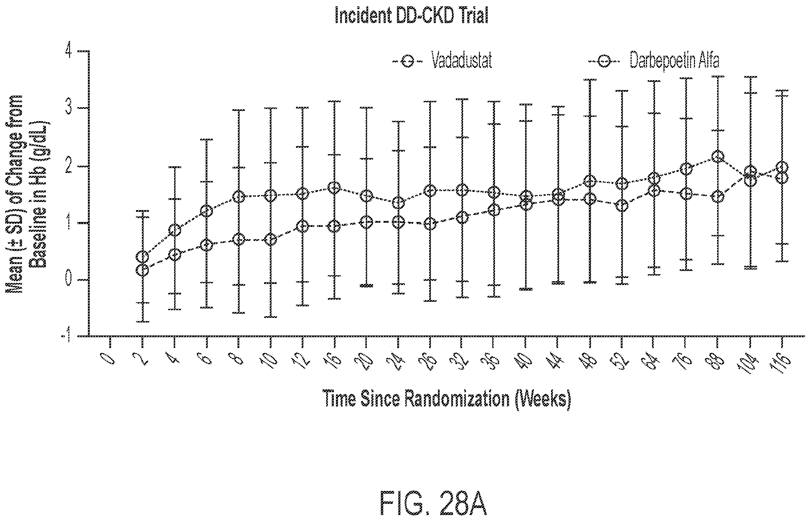

*Incident DD-CKD Trial (Corr*/*Conv).* **Figures 28A-28B** show the change from baseline in hemoglobin in both treatment groups. During weeks 24 to 36, mean (SEM) changes from baseline in hemoglobin were 1.26 (0.11) g/dL and 1.58 (0.11) g/dL in the vadadustat and darbepoetin alfa groups, respectively, with a corresponding mean difference between groups of -0.31 (0.11) g/dL (95% CI, -0.53 to -0.10). During weeks 40 to 52, mean (SEM) changes from baseline in hemoglobin were 1.42 (0.132) g/dL and 1.50 (0.136) g/dL in the vadadustat and darbepoetin alfa groups, respectively, with a corresponding mean difference between groups of -0.07 (0.13) g/dL (95% CI, -0.34 to 0.19).

The proportions of patients with an average hemoglobin value within the geography-specific target range during weeks 24-36 and 40-52 were 43.6% vs 56.9% and 39.8% vs 41.0% in the vadadustat and darbepoetin alfa group, respectively.

The proportion of patients requiring RBC transfusions during weeks 24-36 and 40-52 were 1.3% vs 1.8% and 2.4% vs 0.7% in the vadadustat and darbepoetin alfa group, respectively.

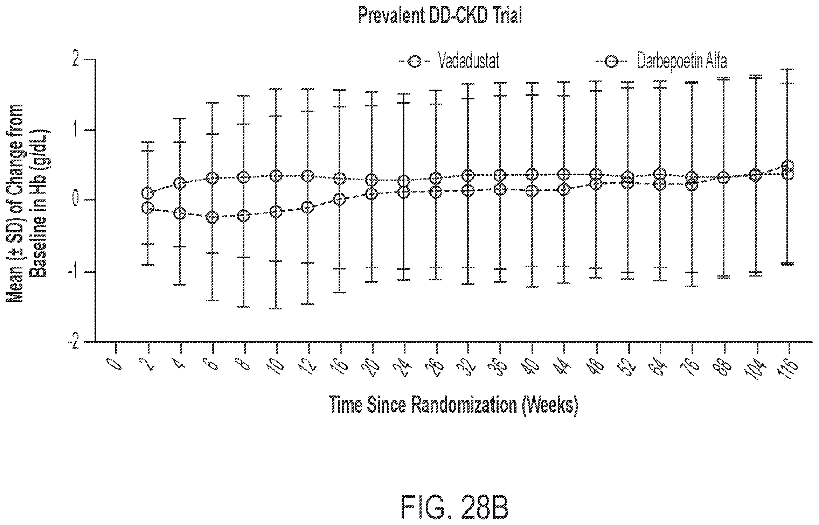

*Prevalent DD-CKD* Trial *(Conv).* As shown in **Figure 28B** the mean (SEM) changes from baseline in hemoglobin during weeks 24 to 36, were 0.19 (0.032) g/dL and 0.36 (0.032) g/dL in the vadadustat and darbepoetin alfa groups, respectively, with a corresponding mean difference between groups of -0.17 (0.033) g/dL (95% CI, -0.23 to -0.10). During weeks 40 to 52, mean changes from baseline in hemoglobin were 0.23 (0.035) g/dL and 0.41 (0.033) g/dL in the vadadustat and darbepoetin alfa groups, respectively, with a corresponding mean difference between groups of - 0.18 (0.035) g/dL (95% CI, -0.25 to -0.12). The proportion of patients with an average hemoglobin value within the geography-specific target range during weeks 24-36 and 40-52 were 49.2% versus 53.2% and 44.3% versus 50.9% in the vadadustat and darbepoetin alfa group, respectively.

The proportion of patients requiring RBC transfusions during weeks 24-36 and 40-52 were 2.0% vs 1.9% and 3.7% vs 3.1% in the vadadustat and darbepoetin alfa group, respectively.

Iron Parameters. The mean concentrations of hepcidin and ferritin and TSAT values were similar in the vadadustat and darbepoetin alfa groups during the primary and the secondary evaluation periods in the incident CC-CKD and the prevalent DD-CKD trials (**Table 10**)**.**

**Table 10: Iron-Related Parameters at Baseline, Averages over Weeks 24-26, and Weeks 40-52 in Randomized Population**

| | Hepcidin (ng/mL) | | Ferritin (ng/mL) | | TSAT (%) | |
|---|---|---|---|---|---|---|
| | Vadadustat | Darbepoetin alfa | Vadadustat | Darbepoetin alfa | Vadadustat | Darbepoetin alfa |
| **Incident trial** | N=181 | N=188 | N=181 | N=188 | N=181 | N=188 |
| Baseline, mean (SD) | 122.43 (109.48) | 126.88 (111.18) | 469.72 (316.92) | 527.76 (401.10) | 31.32 (9.45) | 34.21 (12.68) |
| Weeks 24-36, mean (SD) | 96.05 (95.19) | 105.88 (89.65) | 495.73 (398.49) | 529.63 (410.72) | 31.65 (10.61) | 36.26 (13.82) |
| Weeks 40-52, mean (SD) | 95.73 (72.10) | 101.14 (95.62) | 555.47 (453.16) | 559.42 (458.51) | 33.11 (12.00) | 35.62 (13.78) |

| **Prevalent trial** | N=1777 | N=1777 | N=1777 | N=1777 | N=1777 | N=1777 |
|---|---|---|---|---|---|---|
| Baseline, mean (SD) | 193.86 (140.12) | 190.38 (135.87) | 846.78 (562.65) | 840.65 (538.49) | 38.06 (13.45) | 37.63 (13.17) |
| Weeks 24-36, mean (SD) | 146.95 (123.57) | 165.52 (128.21) | 801.93 (605.91) | 838.18 (580.75) | 34.77 (12.55) | 36.42 (14.25) |
| Weeks 40-52, mean (SD) | 137.41 (119.87) | 158.22 (123.37) | 787.27 (550.21) | 828.91 (565.77) | 34.11 (12.44) | 36.62 (14.31) |

### Discussion

These two global phase III clinical trials aimed at evaluating the safety and efficacy of once-daily oral vadadustat compared to darbepoetin alfa for the maintenance treatment of patients with kidney failure and anemia met their prespecified, regulatory agency-approved noninferiority margin for cardiovascular safety and hematologic efficacy. Vadadustat was noninferior to darbepoetin alfa in maintaining hemoglobin concentrations among anemic patients who were new to or established on dialysis during the primary evaluation period (weeks 24-36). Similar efficacy was sustained throughout the secondary evaluation period (weeks 40-52) and during the entire course of the studies. These findings were consistent and robust across all prespecified subgroup analyses.

Darbepoetin alfa is an injectable hyperglycosylated recombinant erythropoietin molecule, developed for sustained activation of the erythropoietin receptor,^{1,2} whereas vadadustat is an orally active, small-molecule HIF prolyl-hydroxylase inhibitor developed to stabilize HIF and thereby mimic a state of cellular hypoxia.³ Although anemia in patients with DD-CKD is multifactorial, the success of recombinant human erythropoietin in treating the anemia of kidney failure has provided strong evidence that inadequately low production of endogenous erythropoietin by the diseased kidneys and lack of a compensatory increase of hepatic erythropoietin production are major causes.⁴ Although the mechanisms dampening endogenous erythropoietin production in CKD have not been resolved, the current studies indicate sustained de-inhibition by targeting the HIF pathway, thereby extending previous phase II study results of vadadustat and other HIF prolyl-hydroxylase inhibitors.⁵⁻⁸

After study initiation in the prevalent DD-CKD trial, we observed a temporary decline and less rapid increase of hemoglobin concentrations in patients randomized to vadadustat compared with darbepoetin alfa, which we interpret as a consequence of the study design. All patients randomized to vadadustat were started on the same vadadustat dose (i.e., 300 mg/d), whereas those randomized to darbepoetin alfa either continued darbepoetin alfa on a dose that had previously been adjusted to individual needs or were switched from another ESA using accepted conversion factors for the previously established dose. The virtual overlay of hemoglobin concentration curves beyond 24 weeks thus demonstrates not only the efficacy of vadadustat but also the ability to achieve a target hemoglobin range without overshooting through dose titration of vadadustat. Mean maintenance doses for vadadustat during the primary and secondary efficacy evaluation periods suggest that dose requirements in most patients are above the starting dose used in the current trials.

In conclusion, vadadustat was not inferior to darbepoetin alfa in correction/maintenance of hemoglobin concentrations in patients with DD-CKD and anemia.

### References

1. Prescribing information. ARANESP® (darbepoetin alfa) injection, for intravenous or subcutaneous use. Amgen, 2019.
2. Macdougall IC, Padhi D, Jang G. Pharmacology of darbepoetin alfa. Nephrol Dial Transplant. 2007;22 Suppl 4:iv2-iv9.
3. Pergola PE, Spinowitz BS, Hartman CS, Maroni BJ, Haase VH. Vadadustat, a novel oral HIF stabilizer, provides effective anemia treatment in nondialysis-dependent chronic kidney disease. Kidney Int. 2016;90(5):1115-1122.
4. Eckardt KU. After 15 years of success--perspectives of erythropoietin therapy. Nephrol Dial Transplant. 2001;16(9):1745-1749.
5. Hartman C, Smith MT, Flinn C, et al. AKB-6548, a new hypoxia-inducible factor prolyl hydroxylase inhibitor increases hemoglobin while decreasing ferritin in a 28-day, phase 2a dose escalation study in stage 3 and 4 chronic kidney disease patients with anemia. Journal of the American Society of Nephrology: JASN. 2011;22:435A.
6. Nangaku M, Farag Y, de Goma E, Luo W, Vargo D, Khawaja Z. Vadadustat, an oral hypoxia-inducible factor prolyl hydroxylase inhibitor, for treatment of anemia of chronic kidney disease: two randomized phase 2 trials in Japanese patients. Nephrol Dial Transplant. 2020;(accepted).
7. Nangaku M, Khawaja Z, Luo W, Garafola S, DeGoma E, Komatsu Y. Randomized, placebo-controlled phase 2 trials of vadadustat, an oral hypoxia-inducible factor prolyl hydroxylase inhibitor (HIF-PHI), to treat anemia of chronic kidney disease (CKD) [Abstract]. Journal of the American Society of Nephrology : JASN. 2018;29:171.
8. Haase VH. HIF-prolyl hydroxylases as therapeutic targets in erythropoiesis and iron metabolism. Hemodialysis international International Symposium on Home Hemodialysis. 2017;21 Suppl 1(Suppl 1):5110-s124.

### Example 5: Vadadustat (Compound 1) for the Maintenance Treatment of Anemia in Subjects with Nondialysis-Dependent Chronic Kidney Disease (NDD-CKD)

Two global, Phase 3, randomized, open-label, active-controlled, sponsor-blind clinical trials were conducted to evaluate safety and efficacy of vadadustat (Compound 1) vs the ESA darbepoetin alfa in adult patients with NDD-CKD and anemia. Patients recruited into the ESA-untreated NDD-CKD trial (N=1761) had hemoglobin <10 g/dL and were not previously being treated with an ESA. Patients recruited into the ESA-treated NDD-CKD trial (N=1750) had hemoglobin ranges of 8-11 g/dL (US) or 9-12 g/dL (non-US) and were actively treated with an ESA for anemia associated with CKD. Trial periods in both trials included 1) correction/conversion (weeks 0-23); 2) maintenance (weeks 24-52); 3) long-term treatment (week 53 to end of treatment); and 4) safety follow-up (end-of-treatment to 4 weeks later). The primary safety endpoint was time to first adjudicated major adverse cardiovascular event, defined as all-cause mortality, non-fatal myocardial infarction or non-fatal stroke pooled across both trials. The primary efficacy endpoint in each trial was mean change in hemoglobin from baseline to primary evaluation period (weeks 24-36) comparing vadadustat vs darbepoetin alfa treatment arms. Demographics and baseline characteristics were similar among patients in both trials, and typical for a large proportion of the NDD-CKD population. These trials helped define the safety and efficacy of vadadustat, an oral hypoxia-inducible factor prolyl hydroxylase inhibitor for management of anemia associated with NDD-CKD.

### Methods

Two randomized, Phase 3, open-label, active-controlled clinical trials were conducted to evaluate the safety and efficacy of oral vadadustat versus injectable darbepoetin alfa: Correction (previously ESA-untreated) and Conversion (previously ESA-treated). Target enrollment for each was approximately 1850 patients recruited at approximately 390 and 480 sites in North America, Latin America, Europe, and the Asia Pacific for the ESA-untreated NDD-CKD trial and the ESA-treated NDD-CKD trial, respectively.

*Trial Populations.* Each trial recruited adults (>18 years of age) with NDD-CKD (estimated glomerular filtration rate ≤60 mL/min/1.73 m²). Patients recruited to the ESA-untreated NDD-CKD trial had a screening hemoglobin <10 g/dL, and patients in the ESA-treated NDD-CKD trial had a screening hemoglobin between 8.0 and 11 g/dL (US) or between 9.0 and 12 g/dL (non-US). In addition to the hemoglobin entry criteria, all patients were required to have serum ferritin ≥100 ng/mL and transferrin saturation ≥20% at screening in order to be randomized. In the ESA-untreated NDD-CKD trial, ESA use was permitted during screening, as per standard of care; however, patients were excluded if they received any ESA within 8 weeks of randomization. In the ESA-treated NDD-CKD trial, patients were actively maintained on ESA therapy, with at least one dose received within 6 weeks before or during screening. Patients were excluded from either trial if anemia was due to conditions other than CKD, including active bleeding or recent blood loss, or if they had uncontrolled hypertension or recent cardiovascular events. Comprehensive inclusion and exclusion criteria for each trial are shown in **Table 11.**

**Table 11: Eligibility Criteria**

| **Correction - ESA-untreated NDD-CKD** | **Conversion- ESA-treated NDD-CKD** |
|---|---|
| | **N = 1750** |
| **N = 1761** | |
| Adults (≥18 years old) with diagnosis of CKD with eGFR ≤60 mL/min/1.73 m² by 2009 CKD-EPI criteria, not expected to start dialysis <6 months before screening | Adults (≥18 years old) with diagnosis of CKD with eGFR ≤60 mL/min/1.73 m² by 2009 CKD-EPI criteria, not expected to start dialysis <6 months before screening |
| Ferritin ≥100 ng/mL and transferrin saturation ≥20% | |
| Folate and vitamin B₁₂ measurements ≥ lower limit of normal during screening | |
| Understands the procedures and requirements of the trial and provides written informed consent and authorization for protected health information disclosure | |
| **Hb Eligibility** | |
| Hb <10 g/dL | US: Hb 8-11 g/dL (inclusive) |
| | Non-US: Hb 9-12 g/dL (inclusive) |
| **ESA treatment** | |
| No ESA (eg, recombinant human EPO [rhEPO], darbepoetin alfa, or methoxy polyethylene glycol-epoetin beta) within 8 weeks before randomization | Currently maintained on ESA therapy, with a dose received within 6 weeks before screening |
| **RBC transfusions** | |
| No RBC transfusions within 8 weeks before randomization | |
| **Exclusion of patients with any of the following:** | |
| • Anemia attributable to a cause other than CKD or patients with active bleeding or recent blood loss | |
| • History of sickle cell disease, myelodysplastic syndromes, bone marrow fibrosis, hematologic malignancy, myeloma, hemolytic anemia, thalassemia, or pure red cell aplasia | |
| • AST/SGOT, ALT/SGPT, or total bilirubin >2.0 × ULN during screening; patients with a history of Gilbert syndrome are not excluded | |
| • Uncontrolled hypertension (defined as confirmed predialysis systolic BP >190 mm Hg or diastolic BP >110 mm Hg at rest) during screening | |
| • Severe HF during screening (New York Heart Association class IV) | |
| • Acute coronary syndrome (hospitalization for unstable angina or MI), surgical or percutaneous intervention for coronary, cerebrovascular, or peripheral artery disease (aortic or lower extremity), surgical or percutaneous valvular replacement or repair, sustained ventricular tachycardia, hospitalization for HF, or stroke within 12 weeks before screening | |
| • History of active malignancy within 2 years before or during screening, except for treated basal cell carcinoma of skin, curatively resected squamous cell carcinoma of skin, or cervical carcinoma in situ | |
| • History of DVT or PE within 12 weeks before randomization | |
| • History of hemosiderosis or hemochromatosis | |
| • History of prior organ transplantation or scheduled organ transplant (patients on the kidney transplant waiting list or with a history of failed kidney transplant are not excluded), or prior hematopoietic stem cell or bone marrow transplant (corneal transplants and stem cell therapy for knee arthritis are not excluded) | |
| • Hypersensitivity to vadadustat, darbepoetin alfa, or any of their excipients | |
| • Use of an investigational medication or participation in an investigational trial within 30 days or 5 half-lives of the investigational medication (whichever is longer) before screening | |
| • Previous participation in this trial or previous participation in a trial with an HIF prolyl hydroxylase inhibitor other than vadadustat | |
| • Females who are pregnant or breastfeeding or are of childbearing potential who are unable or unwilling to use an acceptable method of contraception | |
| • Nonvasectomized male patients who are unable or unwilling to use an acceptable method of contraception | |
| • Any other reason that in the opinion of the investigator would make the patient not suitable for participation in the trial | |

| | |
|---|---|
| ALT, alanine aminotransferase; AST, aspartate aminotransferase; BP, blood pressure; CKD, chronic kidney disease; DVT, deep vein thrombosis; eGFR, estimated glomerular filtration rate; ESA, erythropoiesis-stimulating agent; Hb, hemoglobin; HF, heart failure; HIF, hypoxia-inducible factor; MI, myocardial infarction; PE, pulmonary embolism; RBC, red blood cell; SGOT, serum glutamic oxaloacetic transaminase, SGPT, serum glutamic pyruvic transaminase; ULN, upper limit of normal. | |

In total, 5457 patients were screened for the ESA-untreated NDD-CKD trial, and 1761 were randomized. The majority (N=1070) were in the US, and the remainder were in Europe or non-US/non-Europe. A total of 3422 patients were screened for the ESA-treated NDD-CKD trial, 1750 of whom were randomized. The majority (N=1063) were from Europe or non-USs/non-Europe countries, and the remainder were from the US). The most common reason for screen failure in both trials was an out-of-range hemoglobin concentration.

**Table 12** shows baseline demographic and clinical characteristics for patients randomized into the ESA-untreated and ESA-treated NDD-CKD trials. The mean age of patients was 65.0 years in the ESA-untreated NDD-CKD trial and 66.8 years in the ESA-treated NDD-CKD trial. The majority of patients in both trials were white (63.8% and 71.5%, respectively) and female (56.1% and 55.4% respectively). Both trial populations were ethnically diverse, recruiting Black/African American (20.6% and 13.2%, respectively), Asian (4.8% and 6.7%, respectively), Native Hawaiian or Other Pacific Islander (0.7% and 0.2%, respectively), American Indian or Alaska Native (2.6% and 3.3%, respectively), and other races (6.0% and 3.3%, respectively).

**Table 12. Demographic and Baseline Characteristics**

| | **ESA-untreated NDD-CKD** | | | **ESA-treated NDD-CKD** | | |
|---|---|---|---|---|---|---|
| **Characteristics** | **US (N=1070)** | **Non-US (N=691)** | **Total (N=1761)** | **US (N=685)** | **Non-US (N=1063)** | **Total (N=1750)** |
| Age (y), mean (SD) | 67.3 (12.9) | 61.4 (14.9) | 65 (14.0) | 69.0 (12.5) | 65.4 (13.7) | 66.8 (13.4) |
| Male, n (%) | 478 (44.7) | 295 (42.7) | 773 (43.9) | 309 (45.1) | 471 (44.3) | 780 (44.6) |
| BMI (kg/m²), mean (SD) | 31.1 (7.9) | 27.8 (5.7) | 29.8 (7.3) | 31.1 (8.1) | 28.3 (6.4) | 29.4 (7.3) |

| Race, n (%) | | | | | | |
|---|---|---|---|---|---|---|
| White | 682 (63.7) | 442 (64.0) | 1124 (63.8) | 451 (65.8) | 798 (75.1) | 1251 (71.5) |
| Black or African American | 309 (28.9) | 54 (7.8) | 363 (20.6) | 201 (29.3) | 30 (2.8) | 231 (13.2) |
| Asian | 48 (4.5) | 37 (5.4) | 85 (4.8) | 16 (2.3) | 101 (9.5) | 117 (6.7) |
| American Indian or Alaska Native | 3 (0.3) | 42 (6.1) | 45 (2.6) | 1 (0.1) | 57 (5.4) | 58 (3.3) |
| Native Hawaiian or other Pacific Islander | 10 (0.9) | 2 (0.3) | 12 (0.7) | 2 (0.3) | 1 (0.1) | 3 (0.2) |
| Other | 3 (0.3) | 103 (14.9) | 106 (6.0) | 4 (0.6) | 53 (5.0) | 57 (3.3) |
| Multiple | 6 (0.6) | 9 (1.3) | 15 (0.9) | 2 (0.3) | 2 (0.2) | 4 (0.2) |
| Not reported | 8 (0.7) | 2 (0.3) | 10 (0.6) | 8 (1.2) | 19 (1.8) | 27 (1.5) |

| Smoking, n (%) | | | | | | |
|---|---|---|---|---|---|---|
| Never smoked | 640 (59.8) | 486 (70.3) | 1126 (63.9) | 376 (54.9) | 772 (72.6) | 1150 (65.7) |
| Former smoker | 383 (35.8) | 157 (22.7) | 540 (30.7) | 270 (39.4) | 236 (22.2) | 506 (28.9) |
| Current smoker | 45 (4.2) | 48 (6.9) | 93 (5.3) | 39 (5.7) | 53 (5.0) | 92 (5.3) |

| Geographic region, n (%) | | | | | | |
|---|---|---|---|---|---|---|
| United States | 1066 (99.6) | 5 (0.7) | 1071 (60.8) | 685 (100.0) | 5 (0.5) | 690 (39.4) |
| Europe | 0 | 139 (20.1) | 139 (7.9) | 0 | 444 (41.8) | 446 (25.5) |
| Non-US/non-Europe | 4 (0.4) | 547 (79.2) | 551 (31.3) | 0 | 614 (57.8) | 614 (35.1) |

| CKD stage n, (%) | | | | | | |
|---|---|---|---|---|---|---|
| Stage 3 | 243 (22.7) | 133 (19.2) | 376 (21.4) | 168 (24.5) | 246 (23.1) | 415 (23.7) |
| Stage 4 | 523 (48.9) | 243 (35.2) | 766 (43.5) | 340 (49.6) | 482 (45.3) | 823 (47.0) |
| Stage 5 | 284 (26.5) | 310 (44.9) | 594 (33.7) | 163 (23.8) | 326 (30.7) | 489 (27.9) |

| | ESA-untreated NDD-CKD | | | ESA-treated NDD-CKD | | |
|---|---|---|---|---|---|---|
| Characteristics | US (N=1070) | Non-US (N=691) | Total (N=1761) | US (N=685) | Non-US (N=1063) | Total (N=1750) |
| Comorbidities/etiology of CKD, n (%)* | | | | | | |
| Autoimmune/glomerulonephritis/vasculitis | 48 (4.5) | 66 (9.6) | 114 (6.5) | 39 (5.7) | 100 (9.4) | 139 (7.9) |
| Cystic/hereditary/congenital disease | 14 (1.3) | 21 (3.0) | 35 (2.0) | 15 (2.2) | 39 (3.7) | 54 (3.1) |
| Diabetes mellitus | 693 (64.8) | 334 (48.3) | 1027 (58.3) | 407 (59.4) | 486 (45.7) | 894 (51.1) |
| Hypertension | 684 (63.9) | 323 (46.7) | 1007 (57.2) | 453 (66.1) | 498 (46.8) | 951 (54.3) |
| Interstitial nephritis/pyelonephritis | 11 (1.0) | 69 (10.0) | 80 (4.5) | 16 (2.3) | 126 (11.9) | 142 (8.1) |
| Neoplasms/tumors | 2 (0.2) | 4 (0.6) | 6 (0.3) | 4 (0.6) | 6 (0.6) | 10 (0.6) |
| Other | 72 (6.7) | 85 (12.3) | 157 (8.9) | 53 (7.7) | 140 (13.2) | 194 (11.1) |

| Laboratory values and blood pressure, mean (SD) | | | | | | |
|---|---|---|---|---|---|---|
| | | | | | | |
| Participants with pre-baseline ESA use (n) | NA | NA | NA | 640 | 991 | 1633 |
| Pre-baseline ESA (U/kg/week) | NA | NA | NA | 135.7 (177.4) | 87.6 (239.8) | 106.5 (218.6) |
| Baseline Hb (g/dL) | 9.1 (0.7) | 9.1 (0.9) | 9.1 (0.8) | 9.8 (0.8) | 10.8 (0.8) | 10.4 (0.9) |
| Ferritin (ng/mL) | 373.4 (301.4) | 346.9 (270.4) | 363.0 (289.9) | 396.7 (297.0) | 357.9 (304.1) | 373.1 (301.7) |
| TSAT (%) | 31.0 (9.7) | 30.7 (10.0) | 30.9 (9.8) | 32.0 (10.3) | 33.2 (10.0) | 32.7 (10.1) |
| TIBC (µg/dL) | 238.2 (44.6) | 245.8 (49.4) | 241.2 (46.6) | 239.1 (45.7) | 246.3 (43.1) | 243.5 (44.2) |
| Hepcidin (ng/mL) | 102.1 (95.8) | 107.7 (105.3) | 104.3 (99.6) | 114.9 (105.9) | 94.6 (86.3) | 102.4 (94.9) |
| Serum creatinine (mg/dL) | 3.1 (1.6) | 4.0 (2.5) | 3.4 (2.0) | 2.9 (1.4) | 3.2 (1.7) | 3.1 (1.6) |
| eGFR (mL/min/1.73 m²) | 23.2 (12.7) | 19.5 (12.4) | 21.7 (12.7) | 24.4 (14.6) | 22.3 (12.2) | 23.1 (13.2) |
| UACR (mg/g) | 1496.8 (1960.8) | 1676.7 (2259.2) | 1570.7 (2089.7) | 1219.7 (1862.4) | 1240.7 (2548.2) | 1231.6 (2308.2) |
| CRP (mg/dL) | 0.7 (1.2) | 0.8 (2.0) | 0.8 (1.6) | 0.8 (1.7) | 0.7 (1.7) | 0.8 (1.7) |
| Total cholesterol (mg/dL) | 159.9 (48.5) | 177.0 (58.5) | 166.6 (53.3) | 165.3 (48.8) | 178.8 (51.3) | 173.5 (50.7) |
| LDL (mg/dL) | 80.1 (38.2) | 97.3 (45.1) | 86.8 (41.9) | 82.9 (37.3) | 97.4 (42.1) | 91.7 (40.9) |
| HDL (mg/dL) | 49.0 (18.6) | 45.4 (15.8) | 47.6 (17.6) | 49.2 (19.2) | 47.3 (15.0) | 48.1 (16.8) |
| Triglycerides (mg/dL) | 155.0 (108.8) | 169.6 (111.1) | 160.7 (109.9) | 163.6 (116.4) | 172.7 (108.7) | 169.1 (111.8) |
| Systolic blood pressure (mm Hg) | 139.8 (18.1) | 138.2 (18.1) | 139.1 (18.1) | 137.6 (18.2) | 136.2 (17.2) | 136.8 (17.6) |
| Diastolic blood pressure (mm Hg) | 71.2 (12.2) | 77.4 (11.6) | 73.6 (12.3) | 70.9 (11.0) | 75.9 (10.8) | 73.9 (11.2) |
| Prior medications, n (%) | | | | | | |
| Diuretic agents | 719 (67.2) | 412 (59.6) | 1131 (64.2) | 483 (70.5) | 612 (57.6) | 1097 (62.7) |
| β-blocking agents | 663 (62.0) | 292 (42.3) | 955 (54.2) | 438 (63.9) | 494 (46.5) | 933 (53.3) |
| ACE inhibitors | 250 (23.4) | 154 (22.3) | 404 (22.9) | 158 (23.1) | 285 (26.8) | 444 (25.4) |
| Angiotensin II receptor blockers | 363 (33.9) | 289 (41.8) | 652 (37.0) | 206 (30.1) | 469 (44.1) | 676 (38.6) |
| Calcium channel blockers | 583 (54.5) | 389 (56.3) | 972 (55.2) | 367 (53.6) | 480 (45.2) | 847 (48.4) |
| Insulin | 416 (38.9) | 213 (30.8) | 629 (35.7) | 243 (35.5) | 306 (28.8) | 550 (31.4) |
| Blood glucose-lowering drugs, excluding insulins | 334 (31.2) | 155 (22.4) | 489 (27.8) | 200 (29.2) | 237 (22.3) | 437 (25.0) |
| Aldosterone antagonist | 55 (5.1) | 40 (5.8) | 95 (5.4) | 41 (6.0) | 39 (3.7) | 80 (4.6) |
| HMG-CoA reductase inhibitors | 694 (64.9) | 263 (38.1) | 957 (54.3) | 445 (65.0) | 446 (42.0) | 892 (51.0) |
| Lipid-modifying agents | 751 (70.2) | 286 (41.4) | 1037 (58.9) | 484 (70.7) | 478 (45.0) | 963 (55.0) |
| Aspirin | 442 (41.3) | 172 (24.9) | 614 (34.9) | 302 (44.1) | 264 (24.8) | 566 (32.3) |
| Vitamin K antagonists | 30 (2.8) | 14 (2.0) | 44 (2.5) | 31 (4.5) | 44 (4.1) | 77 (4.4) |
| Oral iron | 347 (32.4) | 157 (22.7) | 504 (28.6) | 218 (31.8) | 206 (19.4) | 424 (24.2) |
| IV iron | 13 (1.2) | 8 (1.2) | 21 (1.2) | 9 (1.3) | 8 (0.8) | 17 (1.0) |

| | | | | | | |
|---|---|---|---|---|---|---|
| ACE, angiotensin-converting enzyme; BMI, body mass index; CKD, chronic kidney disease; CRP, C-reactive protein; eGFR, estimated glomerular filtration rate; ESA, erythropoiesis-stimulating agent; Hb, hemoglobin, HDL, high-density lipoprotein; HMG-CoA, 3-hydroxy-3-methylglutaryl coenzyme A; IV, intravenous; LDL, low-density lipoprotein; NA, not applicable; SD, standard deviation; TIBC, total iron binding capacity; TSAT, transferrin saturation; UACR, urinary albumin-to-creatinine ratio. | | | | | | |

More than 40% of patients in the ESA-untreated NDD-CKD trial and the ESA-treated NDD-CKD trial had a history of cardiovascular disease (CVD) at baseline (46% and 43%, respectively). **Table 13** shows baseline characteristics by presence or absence of CVD. As expected, patients with a history of CVD were older, more frequently male, more frequently white, less frequently never smoked and more frequently had diabetes mellitus. Other baseline characteristics, including laboratory values, were similar for patients in both trials, irrespective of the history of CVD. With respect to concomitant medications, patients in both trials with a history of CVD were more frequently prescribed diuretic agents, β-blocking agents, aldosterone antagonists, HMG-CoA reductase inhibitors and other lipid-modifying agents, aspirin, vitamin K antagonists and insulin.

**Table 13. Demographic and Baseline Characteristics by Cardiovascular Disease Status**

| | **ESA-untreated NDD-CKD** | | **ESA-treated NDD-CKD** | |
|---|---|---|---|---|
| | **History of Cardiovascular Disease at Baseline** | | | |
| **Characteristics** | **Yes (N=806)** | **No (N=955)** | **Yes (N=760)** | **No (N=990)** |
| Age (y), mean (SD) | 68.9 (11.6) | 61.8 (15.0) | 69.4 (12.0) | 64.8 (14.0) |
| Male, n (%) | 377 (46.8) | 396 (41.5) | 366 (48.2) | 414 (41.8) |
| BMI (kg/m²), mean (SD) | 30.6 (7.7) | 29.1 (6.9) | 30.0 (7.6) | 28.9 (7) |

| Race, n (%) | | | | |
|---|---|---|---|---|
| White | 569 (70.6) | 555 (58.1) | 589 (77.5) | 662 (66.9) |
| Black or African American | 160 (19.9) | 203 (21.3) | 102 (13.4) | 129 (13.0) |
| Asian | 25 (3.1) | 60 (6.3) | 29 (3.8) | 88 (8.9) |
| American Indian or Alaska Native | 5 (0.6) | 40 (4.2) | 10 (1.3) | 48 (4.8) |
| Native Hawaiian or other Pacific Islander | 7 (0.9) | 5 (0.5) | 2 (0.3) | 1 (0.1) |
| Other | 32 (4.0) | 74 (7.7) | 17 (2.2) | 40 (4.0) |
| Multiple | 5 (0.6) | 10 (1.0) | 2 (0.3) | 2 (0.2) |
| Not reported | 3 (0.4) | 7 (0.7) | 9 (1.2) | 18 (1.8) |

| Smoking, n (%) | | | | |
|---|---|---|---|---|
| Never smoked | 472 (58.6) | 654 (68.5) | 474 (62.4) | 676 (68.3) |
| Former smoker | 292 (36.2) | 248 (26.0) | 246 (32.4) | 260 (26.3) |
| Current smoker | 41 (5.1) | 52 (5.4) | 40 (5.3) | 52 (5.3) |

| Geographic region, n (%) | | | | |
|---|---|---|---|---|
| United States | 521 (64.6) | 550 (57.6) | 329 (43.3) | 361 (36.5) |
| Europe | 75 (9.3) | 64 (6.7) | 219 (28.8) | 227 (22.9) |
| Non-US/non-Europe | 210 (26.1) | 341 (35.7) | 212 (27.9) | 402 (40.6) |

| CKD stage, n (%) | | | | |
|---|---|---|---|---|
| Stage 3 | 175 (21.7) | 201 (21.0) | 195 (25.7) | 220 (22.2) |

| | **ESA-untreated NDD-CKD** | | **ESA-treated NDD-CKD** | |
|---|---|---|---|---|
| | **History of Cardiovascular Disease at Baseline** | | | |
| **Characteristics** | **Yes (N=806)** | **No (N=955)** | **Yes (N=760)** | **No (N=990)** |
| Stage 4 | 375 (46.5) | 391 (40.9) | 364 (47.9) | 459 (46.4) |
| Stage 5 | 247 (30.6) | 347 (36.3) | 194 (25.5) | 295 (29.8) |

| Comorbidities/etiology of CKD, n (%)* | | | | |
|---|---|---|---|---|
| Autoimmune/glomerulonephritis/vasculitis | 44 (5.5) | 70 (7.3) | 53 (7.0) | 86 (8.7) |
| Cystic/hereditary/congenital disease | 7 (0.9) | 28 (2.9) | 19 (2.5) | 35 (3.5) |
| Diabetes mellitus | 498 (61.8) | 529 (55.4) | 425 (55.9) | 469 (47.4) |
| Hypertension | 467 (57.9) | 540 (56.5) | 399 (52.5) | 552 (55.8) |
| Interstitial nephritis/pyelonephritis | 46 (5.7) | 34 (3.6) | 69 (9.1) | 73 (7.4) |
| Neoplasms/tumors | 3 (0.4) | 3 (0.3) | 5 (0.7) | 5 (0.5) |
| Other | 68 (8.4) | 89 (9.3) | 71 (9.3) | 123 (12.4) |

| Laboratory values, mean (SD) | | | | |
|---|---|---|---|---|
| Participants with pre-baseline ESA use (n) | NA | NA | 715 | 918 |
| Pre-baseline ESA (U/kg/week) | NA | NA | 100.4 (130.6) | 111.2 (267.7) |
| Baseline Hb (g/dL) | 9.1 (0.8) | 9.1 (0.8) | 10.3 (0.9) | 10.4 (0.9) |
| Ferritin (ng/mL) | 366.4 (312.0) | 360.2 (269.9) | 376.7 (291.5) | 370.3 (309.4) |
| TSAT (%) | 30.3 (9.3) | 31.4 (10.3) | 32.1 (10.3) | 33.2 (10.0) |
| TIBC (µg/dL) | 240.9 (47.3) | 241.4 (46.0) | 243.0 (44.7) | 243.9 (43.9) |
| Hepcidin (ng/mL) | 105.7 (103.4) | 103.1 (96.4) | 98.7 (89.1) | 105.2 (99.1) |
| Serum creatinine (mg/dL) | 3.2 (1.8) | 3.6 (2.2) | 2.9 (1.4) | 3.2 (1.7) |
| eGFR (mL/min/1.73 m²) | 22.1 (12.1) | 21.4 (13.2) | 23.3 (11.8) | 23.0 (14.2) |
| UACR (mg/g) | 1461.1 (1894.4) | 1663.5 (2238.6) | 1204.3 (1929.0) | 1252.4 (2559.4) |
| CRP (mg/dL) | 0.8 (1.8) | 0.7 (1.3) | 0.9 (1.9) | 0.7 (1.5) |
| Total cholesterol (mg/dL) | 163.9 (55.4) | 168.9 (51.4) | 170.5 (52.8) | 175.7 (49.0) |
| LDL (mg/dL) | 84.8 (42.1) | 88.5 (41.6) | 90.0 (42.7) | 93.0 (39.4) |
| HDL (mg/dL) | 46.4 (16.3) | 48.7 (18.6) | 46.4 (15.6) | 49.3 (17.6) |
| Triglycerides (mg/dL) | 160.2 (103.7) | 161.2 (114.9) | 168.3 (109.8) | 169.6 (113.4) |

| | **ESA-untreated NDD-CKD** | | **ESA-treated NDD-CKD** | |
|---|---|---|---|---|
| | **History of Cardiovascular Disease at Baseline** | | | |
| **Characteristics** | **Yes (N=806)** | **No (N=955)** | **Yes (N=760)** | **No (N=990)** |
| Systolic blood pressure (mm Hg) | 139.8 (18.5) | 138.6 (17.8) | 136.2 (17.4) | 137.2 (17.8) |
| Diastolic blood pressure (mm Hg) | 72.4 (13.1) | 74.7 (11.5) | 73.0 (11.2) | 74.6 (11.1) |

| Prior medications, n (%) | | | | |
|---|---|---|---|---|
| Diuretic agents | 579 (71.8) | 552 (57.8) | 547 (72.0) | 550 (55.6) |
| β-blocking agents | 556 (69.0) | 399 (41.8) | 518 (68.2) | 415 (41.9) |
| ACE inhibitors | 187 (23.2) | 217 (22.7) | 198 (26.1) | 246 (24.8) |
| Angiotensin II receptor blockers | 304 (37.7) | 348 (36.4) | 277 (36.4) | 399 (40.3) |
| Calcium channel blockers | 449 (55.7) | 523 (54.8) | 372 (48.9) | 475 (48.0) |
| Insulin | 316 (39.2) | 313 (32.8) | 284 (37.4) | 266 (26.9) |
| Blood glucose-lowering drugs, excluding insulins | 227 (28.2) | 262 (27.4) | 206 (27.1) | 231 (23.3) |
| Aldosterone antagonist | 65 (8.1) | 30 (3.1) | 49 (6.4) | 31 (3.1) |
| HMG-CoA reductase inhibitors | 510 (63.3) | 447 (46.8) | 438 (57.6) | 454 (45.9) |
| Lipid-modifying agents | 547 (67.9) | 490 (51.3) | 464 (61.1) | 499 (50.4) |
| Aspirin | 378 (46.9) | 236 (24.7) | 329 (43.3) | 237 (23.9) |
| Vitamin K antagonists | 29 (3.6) | 15 (1.6) | 59 (7.8) | 18 (1.8) |
| Oral iron | 221 (27.4) | 283 (29.6) | 180 (23.7) | 244 (24.6) |
| IV iron | 8 (1.0) | 13 (1.4) | 11 (1.4) | 6 (0.6) |

| | | | | |
|---|---|---|---|---|
| ACE, angiotensin-converting enzyme; BMI, body mass index; CKD, chronic kidney disease; CRP, C-reactive protein; eGFR, estimated glomerular filtration rate; ESA, erythropoiesis-stimulating agent; Hb, hemoglobin, HDL, high-density lipoprotein; HMG-CoA, 3-hydroxy-3-methylglutaryl coenzyme A; IV, intravenous; LDL, low-density lipoprotein; NA, not applicable; SD, standard deviation; TIBC, total iron binding capacity; TSAT, transferrin saturation; UACR, urinary albumin-to-creatinine ratio. | | | | |

*Study Design and Ethics.* The trials are being conducted in accordance with the Declaration of Helsinki on Ethical Principles for Medical Research Involving Human Subjects, the International Conference on Harmonisation guideline on Good Clinical Practice, and applicable local regulatory requirements and laws.

*Treatment Protocol.* There was an 8-week screening period, and patients meeting all inclusion criteria were randomized at the baseline visit 1:1 to vadadustat (Compound 1) or darbepoetin alfa, stratified by geographic region (US vs Europe vs rest of world), New York Heart Association congestive heart failure class (0/I vs II/III), and hemoglobin level at entry (ESA-untreated NDD-CKD: <9.5 vs ≥9.5 g/dL; ESA-treated NDD-CKD: <10 vs ≥10 g/dL).

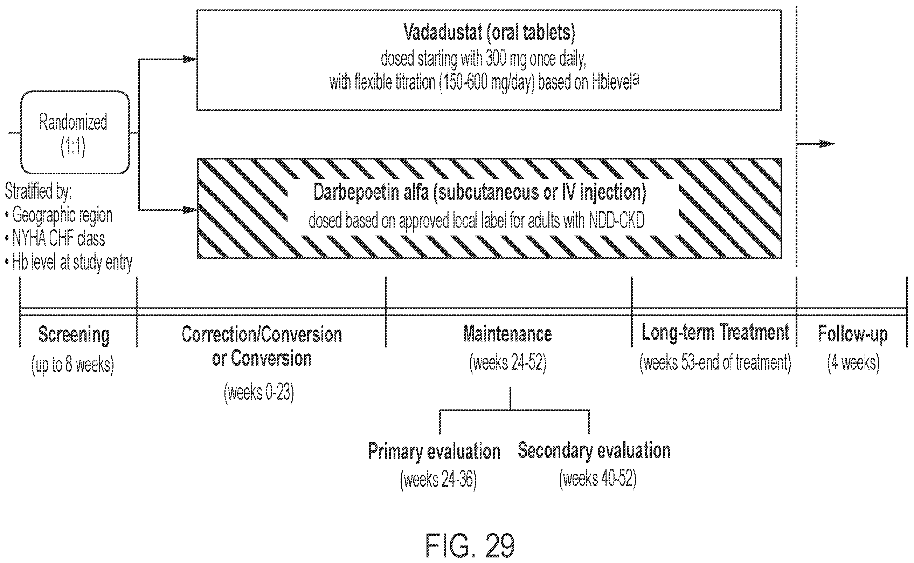

In both trials, patients entered four sequential periods for treatment and evaluation of safety and efficacy **(****FIG. 29****).** These include a correction or conversion period (weeks 0-23), a maintenance period (weeks 23-52) that encompassed the primary and a secondary efficacy evaluation periods (weeks 24-36 and weeks 40-52, respectively) and a long-term safety period (week 53 to end of treatment). After the end of treatment, there will be a post-treatment visit (in-person or remote by video or audio) at four weeks, to further assess safety.

Patients continued taking study medication until the trials were completed based on the accrual of approximately 631 MACE pooled across both trials, and all enrolled patients have completed visit 13.

*Study Drug.* The vadadustat (Compound 1) starting dose at the baseline visit was 300 mg once daily (two 150-mg tablets), and dose levels available in the trial include 150 mg, 300 mg, 450 mg, and 600 mg per day. Subcutaneous dosing of darbepoetin alfa was based on the current package insert in the US or the summary of product characteristics for all non-US investigational sites. The dosing strategy aimed to increase and maintain hemoglobin levels of 10 to 11 g/dL in the US and 10 to 12 g/dL outside the US throughout the trial. Dose adjustments for vadadustat were guided by hemoglobin concentrations and dose adjustment algorithms. Dose adjustments for both agents incorporated hemoglobin concentrations obtained via a HemoCue (point-of-care device), but for safety and efficacy analysis, central laboratory hemoglobin values were used after protocol-specified blood draws for complete blood counts. Throughout the trial, patients were prescribed iron supplementation to maintain serum ferritin ≥100 ng/mL or transferrin saturation ≥20% and may continue an ongoing oral iron supplementation regimen. Red blood cell transfusions were allowed at the discretion of the investigator. Starting at week 6, ESA therapy was permitted for patients with hemoglobin <9.0 g/dL who experienced worsening of the symptoms of anemia (e.g., fatigue, weakness, shortness of breath, chest pain, confusion, or dizziness) compared with baseline. While receiving ESA therapy, study drugs were temporarily discontinued. ESA therapy was stopped when the hemoglobin concentration increased to 9.5 g/dL or more at which time study drugs were restarted. Restarting vadadustat followed a protocol-specified interval based on the timing of the administration of the last dose of the specific ESA used for restoring hemoglobin concentrations (epoetin alfa: 2 days; darbepoetin alfa: 7 days; methoxy polyethylene glycol-epoetin beta: 14 days).

### Trial Endpoints.

Primary endpoints for safety and efficacy and key secondary safety and efficacy endpoints are listed in **Table 14.**

**Table 14: Selected safety and efficacy endpoints**

| | | |
|---|---|---|
| **Primary Safety Endpoint** | • Time to first MACE | |
| **Key Secondary Safety Endpoints** | • Time to first expanded MACE: hospitalization for HF or thromboembolic event, excluding vascular access thrombosis | |
| | • Time to Cardiovascular mortality, non-fatal MI, or non-fatal stroke | |
| | • Time to Cardiovascular mortality | |
| | • Time to All-cause mortality | |
| **Other Safety Endpoints** | • Time to first individual components of MACE | |
| | | o Non-fatal MI |
| | | o Non-fatal stroke |
| | • Time to non-cardiovascular mortality | |
| | • Time to first individual component of expanded MACE | |
| | | o MACE plus thromboembolic event |
| | | o MACE plus thromboembolic event, excluding vascular failure access |
| | | o MACE plus hospitalization for HF |
| | | o MACE plus hospitalization for HF or thromboembolic event |
| | • Treatment-emergent AEs and treatment-emergent SAEs | |
| | • Systolic and diastolic blood pressure | |
| | • Any value of Hb >12.0 g/dL; >13.0 g/dL, >14.0 g/dL, <9.0 g/dl or <8.0 g/dl | |
| | • An Hb increase >1.0 g/dL within any 2-week interval or >2.0 g/dL within any 4-week interval | |
| **Primary Efficacy Endpoint** | • Change in average Hb between baseline and the primary evaluation period (weeks 24-36) | |
| **Key Secondary Efficacy Endpoint** | • Change in average Hb value between baseline and the secondary evaluation period (weeks 40 to 52) | |
| **Other Efficacy Endpoints** | • Proportion of patients having average Hb value in the geography-specific target range in weeks 24 to 36 | |
| | • Proportion of patients having average Hb value in the geography-specific target range in weeks 40 to 52 | |
| | • Proportion of patients receiving red blood cell transfusions | |
| | • Progression of CKD | |
| **Endpoints of Special Interest** | • Mean and percentage change in hepcidin between baseline and the primary (weeks 24-36) and secondary (weeks 40-52) evaluation periods | |
| | • Mean and percentage change in ferritin between baseline and the primary (weeks 24-36) and secondary (weeks 40-52) evaluation periods | |
| | • Mean and percentage change in TSAT between baseline and the primary (weeks 24-36) and secondary (weeks 40-52) evaluation periods | |

*Data Collection.* Study visits occurred at baseline and every two weeks through week 12, and thereafter, every four weeks through week 52. Vital signs were measured and complete blood counts were performed at every study visit, and iron indices, serum chemistry measurements, liver enzymes, and estimated glomerular filtration rate were assessed at every other study visit. Lipid panels were performed at baseline and weeks 28 and 52, and determination of biomarkers were performed at baseline and weeks 12, 28, and 52. Patients continued to have study visits every 12 weeks during year 2 and later, and data were collected at protocol-specified visits. Information on safety parameters (MACE endpoint questionnaire, AEs, transfusions, and ESA rescue assessments), concomitant medication use, and drug-dispensing records were collected at every study visit. Patients may withdraw from the trials on account of death, withdrawal of consent, or being lost to follow-up, however schedule of activities and safety assessments were continued through week 52 and patients were followed for safety-only assessments after week 52.

*Power and Sample Size Calculation.* The primary safety analysis, time to first adjudicated MACE, was based on all events accruing across both trials. For the MACE endpoint, Applicants determined the planned sample size based on the number of events needed to demonstrate noninferiority of the 2-sided 95% CI for the hazard ratio (vadadustat vs darbepoetin alfa) and anticipated event rates. Applicants calculated that 631 events will be required to yield 80% power to establish noninferiority with a margin of 1.25 and >90% power to establish noninferiority with a margin of 1.3, assuming no difference between treatment groups. The power is >90% to establish a prespecified, regulatory agency-agreed-upon noninferiority margin of 1.25 if the hazard ratio is 0.95 in favor of vadadustat.

The primary efficacy analysis assumed that the mean change in hemoglobin for vadadustat was the same as for darbepoetin alfa, and the common standard deviation (SD) for the mean change from baseline was assumed to be 1.5 g/dL. Noninferiority was established based on a 2-sided 95% CI for the difference between the vadadustat and darbepoetin alfa groups using a noninferiority margin of -0.75 g/dL. Using this approach, each trial needed approximately 925 subjects per treatment group to give the noninferiority assessment of efficacy >90% power. If the lower limit of the 2-sided 95% CI for the difference between the mean in the vadadustat group and the mean in the darbepoetin alfa group is above zero, superiority would have been established for vadadustat.

*Statistical Analysis.* Both trials have four analysis populations: a randomized population; a full analysis set comprised of patients who received at least one dose of study medication and who had at least one post-dose hemoglobin measure; a safety population comprised of all randomized patients who received at least one dose of study drug; and a per-protocol population comprised of all patients who received study drug and had at least one hemoglobin measurement during the primary efficacy period and who had no major protocol deviations.

The primary analysis of the primary and secondary efficacy endpoints would use an analysis of covariance with multiple imputations stratified by the randomization strata using baseline hemoglobin as the covariate. A 2-sided 95% CI would be calculated for the difference between treatment groups (vadadustat minus darbepoetin alfa). Noninferiority of vadadustat would be established if the lower limit of this CI is -0.75 g/dL or higher.

### Efficacy Endpoints Results

Vadadustat achieved each of the studies' primary efficacy endpoints of mean change in Hb between baseline and the primary evaluation period (mean Hb from weeks 24 to 36) compared to darbepoetin alfa, in adult patients on dialysis, demonstrating non-inferiority to darbepoetin alfa based on using a non-inferiority margin of -0.75 g/dL. In the Correction study, the least square mean difference in Hb was 0.05 g/dL (95% CI: -0.04, 0.15), achieving the pre-specified non-inferiority criterion of -0.75 g/dL. The mean (SD) Hb level at week 24 to week 36 was 10.39 (0.99) g/dL for vadadustat-treated patients compared to 10.35 (1.03) g/dL for darbepoetin alfa-treated patients. In addition, vadadustat sustained the target Hb efficacy response at weeks 40 to 52 achieving non-inferiority compared to darbepoetin alfa. The least square mean difference in Hb was 0.04 g/dL (95% CI: -0.06, 0.14). The mean (SD) Hb level at week 40 to week 52 was 10.48 (1.05) g/dL for vadadustat-treated patients compared to 10.45 (1.01) g/dL for darbepoetin alfa-treated patients. For the Conversion study, the least square mean difference in Hb was -0.01 g/dL (95% CI: -0.09, 0.07), achieving the pre-specified non-inferiority criterion of -0.75 g/dL. The mean (SD) Hb level at week 24 to week 36 was 10.77 (0.98) g/dL for vadadustat-treated patients compared to 10.77 (0.99) g/dL for darbepoetin alfa-treated patients. Vadadustat also sustained efficacy in the Conversion study demonstrating non-inferiority to darbepoetin with a least square mean difference in Hb of 0.00 g/dL (95% CI: -0.10, 0.09). The mean (SD) Hb level at week 40 to week 52 was 10.80 (1.04) g/dL in the vadadustat-treated patients compared to 10.79 (1.05) g/dL for darbepoetin alpha-treated patients.

## Claims

1. A compound for use in a method of treating anemia in a patient having anemia associated with or secondary to chronic kidney disease, wherein the compound is {[5-(3-chlorophenyl)-3-hydroxypyridine-2-carbonyl]amino}acetic acid having the structure of Compound 1, 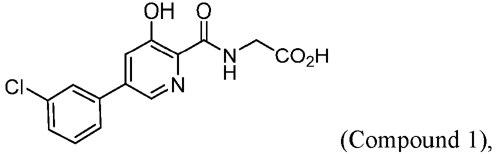 or a pharmaceutically acceptable salt thereof, wherein the patient has been previously treated with an erythropoiesis stimulating agent (ESA) within six weeks or eight weeks prior to or during a screening period before the administration of the Compound 1, and
wherein the method comprises orally administering the compound to the patient for 53-260 weeks.

2. A compound for use in a method of increasing hemoglobin levels to 10.0 - 13.0 g/dL in a patient having anemia associated with or secondary to chronic kidney disease, wherein the compound is {[5-(3-chlorophenyl)-3-hydroxypyridine-2-carbonyl]amino}acetic acid having the structure of Compound 1, 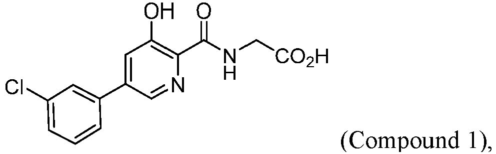 or a pharmaceutically acceptable salt thereof, wherein the patient has been previously treated with an erythropoiesis stimulating agent (ESA) within six weeks or eight weeks prior to or during a screening period before the administration of the Compound 1, and
wherein the method comprises orally administering the compound to the patient for 53-260 weeks.

3. A compound for use in a method of maintaining or controlling hemoglobin levels at 10.0 - 13.0 g/dL in a patient having anemia associated with or secondary to chronic kidney disease, wherein the compound is {[5-(3-chlorophenyl)-3-hydroxypyridine-2-carbonyl]amino}acetic acid having the structure of Compound 1, 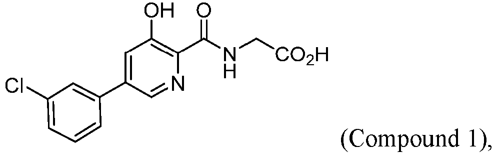 or a pharmaceutically acceptable salt thereof, wherein the patient has been previously treated with an erythropoiesis stimulating agent (ESA) within six weeks or eight weeks prior to or during a screening period before the administration of the Compound 1, and
wherein the method comprises orally administering the compound to the patient for 53-260 weeks.

4. The compound for use of any one of claims 1-3, wherein the patient has non-dialysis dependent chronic kidney disease (NDD-CKD).

5. The compound for use of any one of claims 1-3, wherein the patient has dialysis-dependent chronic kidney disease (DD-CKD).

6. The compound for use of any one of claims 1-5, wherein the erythropoiesis stimulating agent is epoetin, darbepoetin alfa (DA), or methoxy polyethylene glycol-epoetin beta (epoetin beta pegol).

7. The compound for use of any one of claims 1-6, wherein the method comprises orally administering once daily to the patient 150, 300, 450, or 600 mg of Compound 1.

8. A pharmaceutical composition for use in a method of treating anemia in a patient having anemia associated with or secondary to chronic kidney disease, the composition comprising {[5-(3-chlorophenyl)-3-hydroxypyridine-2-carbonyl]amino}acetic acid having the structure of Compound 1, 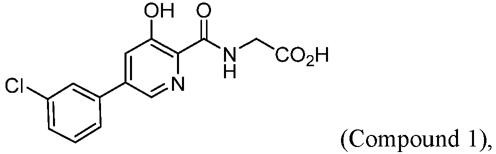 or a pharmaceutically acceptable salt thereof, and one or more excipients,
wherein the patient has been previously treated with an erythropoiesis stimulating agent (ESA) within six weeks or eight weeks prior to or during a screening period before the administration of Compound 1, and
wherein the method comprises orally administering the pharmaceutical composition to the patient for 53-260 weeks.

9. The composition for use of claim 8, wherein the patient has non-dialysis dependent chronic kidney disease (NDD-CKD).

10. The composition for use of claim 8, wherein the patient has dialysis-dependent chronic kidney disease (DD-CKD).

11. The composition for use of any one of claims 8 to 10, wherein the erythropoiesis stimulating agent is epoetin, darbepoetin alfa (DA), or methoxy polyethylene glycol-epoetin beta (epoetin beta pegol).

12. The composition for use of any one of claim 8 to 11, wherein the method comprises orally administering once daily to the patient 150, 300, 450, or 600 mg of Compound 1.

## Patentansprüche

1. Verbindung zur Verwendung in einem Verfahren zur Behandlung von Anämie bei einem Patienten mit Anämie, die mit einer chronischen Nierenerkrankung assoziiert oder sekundär dazu ist, wobei die Verbindung {[5-(3-Chlorphenyl)-3-hydroxypyridin-2-carbonyl]amino}essigsäure mit der Struktur der Verbindung 1, 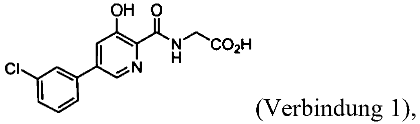 oder ein pharmazeutisch verträgliches Salz davon ist, wobei der Patient zuvor innerhalb von sechs Wochen oder acht Wochen vor oder während einer Screening-Phase vor der Verabreichung einer Dosis der Verbindung 1 mit einem Erythropoese-stimulierenden Mittel (ESA) behandelt worden ist, und
wobei das Verfahren die orale Verabreichung der Verbindung an den Patienten über einen Zeitraum von 53 bis 260 Wochen umfasst.

2. Verbindung zur Verwendung in einem Verfahren zur Erhöhung des Hämoglobinspiegels auf 10,0 bis 13,0 g/dl bei einem Patienten mit Anämie, die mit einer chronischen Nierenerkrankung assoziiert oder sekundär dazu ist, wobei die Verbindung {[5-(3-Chlorphenyl)-3-hydroxypyridin-2-carbonyl]amino}essigsäure mit der Struktur der Verbindung 1, 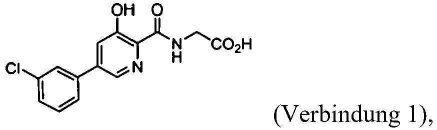 oder ein pharmazeutisch verträgliches Salz davon ist, wobei der Patient zuvor innerhalb von sechs Wochen oder acht Wochen vor oder während einer Screening-Phase vor der Verabreichung einer Dosis der Verbindung 1 mit einem Erythropoese-stimulierenden Mittel (ESA) behandelt worden ist, und
wobei das Verfahren die orale Verabreichung der Verbindung an den Patienten über einen Zeitraum von 53 bis 260 Wochen umfasst.

3. Verbindung zur Verwendung in einem Verfahren zur Aufrechterhaltung oder Kontrolle des Hämoglobinspiegels bei 10,0 bis 13,0 g/dl bei einem Patienten mit Anämie, die mit einer chronischen Nierenerkrankung assoziiert oder sekundär dazu ist, wobei die Verbindung {[5-(3-Chlorphenyl)-3-hydroxypyridin-2-carbonyl]amino}essigsäure mit der Struktur der Verbindung 1, 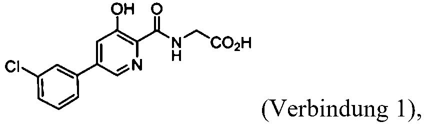 oder ein pharmazeutisch verträgliches Salz davon ist, wobei der Patient zuvor innerhalb von sechs Wochen oder acht Wochen vor oder während einer Screening-Phase vor der Verabreichung einer Dosis der Verbindung 1 mit einem Erythropoese-stimulierenden Mittel (ESA) behandelt worden ist, und
wobei das Verfahren die orale Verabreichung der Verbindung an den Patienten über einen Zeitraum von 53 bis 260 Wochen umfasst.

4. Verbindung zur Verwendung nach einem der Ansprüche 1 bis 3, wobei der Patient an einer nicht dialyseabhängigen chronischen Nierenerkrankung (NDD-CKD) leidet.

5. Verbindung zur Verwendung nach einem der Ansprüche 1 bis 3, wobei der Patient an einer dialyseabhängigen chronischen Nierenerkrankung (DD-CKD) leidet.

6. Verbindung zur Verwendung nach einem der Ansprüche 1 bis 5, wobei das Erythropoese-stimulierende Mittel Epoetin, Darbepoetin alfa (DA) oder Methoxypolyethylenglykol-Epoetin beta (Epoetin beta pegol) ist.

7. Verbindung zur Verwendung nach einem der Ansprüche 1 bis 6, wobei das Verfahren die einmal tägliche orale Verabreichung von 150, 300, 450 oder 600 mg der Verbindung 1 an den Patienten umfasst.

8. Pharmazeutische Zusammensetzung zur Verwendung in einem Verfahren zur Behandlung von Anämie bei einem Patienten mit Anämie, die mit einer chronischen Nierenerkrankung assoziiert oder sekundär dazu ist, wobei die Zusammensetzung {[5-(3-Chlorphenyl)-3-hydroxypyridin-2-carbonyl]amino}essigsäure mit der Struktur der Verbindung 1, 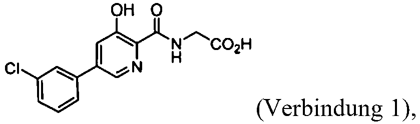 oder ein pharmazeutisch verträgliches Salz davon, und ein oder zwei Hilfsstoffe umfasst,
wobei der Patient zuvor innerhalb von sechs Wochen oder acht Wochen vor oder während einer Screening-Phase vor der Verabreichung einer Dosis der Verbindung 1 mit einem Erythropoese-stimulierenden Mittel (ESA) behandelt worden ist, und
wobei das Verfahren die orale Verabreichung der pharmazeutischen Zusammensetzung an den Patienten über einen Zeitraum von 53 bis 260 Wochen umfasst.

9. Zusammensetzung zur Verwendung nach Anspruch 8, wobei der Patient an einer nicht dialyseabhängigen chronischen Nierenerkrankung (NDD-CKD) leidet.

10. Zusammensetzung zur Verwendung nach Anspruch 8, wobei der Patient an einer dialyseabhängigen chronischen Nierenerkrankung (DD-CKD) leidet.

11. Zusammensetzung zur Verwendung nach einem der Ansprüche 8 bis 10, wobei das Erythropoese-stimulierende Mittel Epoetin, Darbepoetin alfa (DA) oder Methoxypolyethylenglykol-Epoetin beta (Epoetin beta pegol) ist.

12. Zusammensetzung zur Verwendung nach einem der Ansprüche 8 bis 11, wobei das Verfahren die einmal tägliche orale Verabreichung von 150, 300, 450 oder 600 mg der Verbindung 1 an den Patienten umfasst.

## Revendications

1. Composé pour une utilisation dans un procédé de traitement de l'anémie chez un patient présentant une anémie associée ou secondaire à une maladie rénale chronique, dans lequel le composé est de l'acide {[5-(3-chlorophényl)-3-hydroxypyridine-2-carbonyl]amino}acétique présentant la structure du Composé 1, 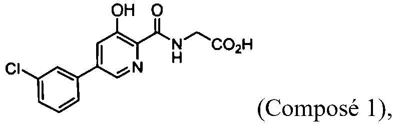 ou un sel pharmaceutiquement acceptable de celui-ci, dans lequel le patient a préalablement été traité par un agent stimulant l'érythropoïèse (ESA) au cours des six semaines ou huit semaines précédant ou pendant une période de sélection avant l'administration d'une dose du Composé 1, et
dans lequel le procédé comprend l'administration orale du composé au patient pendant 53 à 260 semaines.

2. Composé pour une utilisation dans un procédé d'augmentation des niveaux d'hémoglobine à 10,0 - 13,0 g/dl chez un patient présentant une anémie associée ou secondaire à une maladie rénale chronique, dans lequel le composé est de l'acide {[5-(3-chlorophényl)-3-hydroxypyridine-2-carbonyl]amino}acétique présentant la structure du Composé 1, 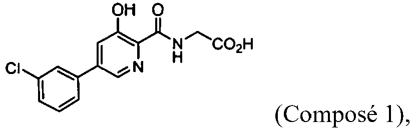
ou un sel pharmaceutiquement acceptable de celui-ci, dans lequel le patient a préalablement été traité par un agent stimulant l'érythropoïèse (ESA) au cours des six semaines ou huit semaines précédant ou pendant une période de sélection avant l'administration d'une dose du Composé 1,
et dans lequel le procédé comprend l'administration orale du composé au patient pendant 53 à 260 semaines.

3. Composé pour une utilisation dans un procédé de maintien ou de contrôle des niveaux d'hémoglobine à 10,0 - 13,0 g/dl chez un patient présentant une anémie associée ou secondaire à une maladie rénale chronique, dans lequel le composé est de l'acide {[5-(3-chlorophényl)-3-hydroxypyridine-2-carbonyl]amino}acétique présentant la structure du Composé 1, 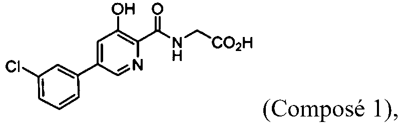
ou un sel pharmaceutiquement acceptable de celui-ci, dans lequel le patient a préalablement été traité par un agent stimulant l'érythropoïèse (ESA) au cours des six semaines ou huit semaines précédant ou pendant une période de sélection avant l'administration d'une dose du Composé 1,
et dans lequel le procédé comprend l'administration orale du composé au patient pendant 53 à 260 semaines.

4. Composé pour une utilisation selon l'une quelconque des revendications 1 à 3, dans lequel le patient présente une maladie rénale chronique non dialysée (NDD-CKD).

5. Composé pour une utilisation selon l'une quelconque des revendications 1 à 3, dans lequel le patient présente une maladie rénale chronique dialysée (DD-CKD).

6. Composé pour une utilisation selon l'une quelconque des revendications 1 à 5, dans lequel l'agent stimulant l'érythropoïèse est l'époétine, la darbépoétine alfa (DA) ou la méthoxy polyéthylène glycol-époétine bêta (époétine bêta pégol).

7. Composé pour une utilisation selon l'une quelconque des revendications 1 à 6, dans lequel le procédé comprend l'administration orale une fois par jour au patient de 150, 300, 450 ou 600 mg de Composé 1.

8. Composition pharmaceutique pour une utilisation dans un procédé de traitement de l'anémie chez un patient présentant une anémie associée ou secondaire à une maladie rénale chronique, dans lequel le composition comprenant de l'acide {[5-(3-chlorophényl)-3-hydroxypyridine-2-carbonyl]amino}acétique présentant la structure du Composé 1, 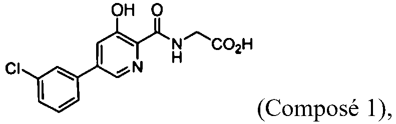 ou un sel pharmaceutiquement acceptable de celui-ci, et un ou plusieurs excipients, dans lequel le patient a préalablement été traité par un agent stimulant l'érythropoïèse (ESA) au cours des six semaines ou huit semaines précédant ou pendant une période de sélection avant l'administration d'une dose du Composé 1, et
dans lequel le procédé comprend l'administration orale du composition pharmaceutique au patient pendant 53 à 260 semaines.

9. Composition pour une utilisation selon la revendication 8, dans lequel le patient présente une maladie rénale chronique non dialysée (NDD-CKD).

10. Composition pour une utilisation selon la revendication 8, dans lequel le patient présente une maladie rénale chronique dialysée (DD-CKD).

11. Composition pour une utilisation selon l'une quelconque des revendications 8 à 10, dans lequel l'agent stimulant l'érythropoïèse est l'époétine, la darbépoétine alfa (DA) ou la méthoxy polyéthylène glycol-époétine bêta (époétine bêta pégol).

12. Composition pour une utilisation selon l'une quelconque des revendications 8 à 11, dans lequel le procédé comprend l'administration orale une fois par jour au patient de 150, 300, 450 ou 600 mg de Composé 1.
